**Europäisches Patentamt**

**European Patent Office**

**Office européen des brevets**

(19)

(11) Publication number: **0 123 430**
**A1**

## EUROPEAN PATENT APPLICATION

(12)

(21) Application number: **84301951.4**

(22) Date of filing: **22.03.84**

(51) Int. Cl.³: **H 04 Q 9/00**

(30) Priority: **24.03.83 GB 8308202**

(43) Date of publication of application:
**31.10.84 Bulletin 84/44**

(84) Designated Contracting States:
**DE FR IT**

(71) Applicant: **Possum Controls Limited**
**63 Mandeville Road**
**Aylesbury Buckinghamshire HP21 8AE(GB)**

(72) Inventor: **Gemmell, David**
**96 Sandy Lane**
**Cheam Surrey(GB)**

(72) Inventor: **Irvine, Duncan**
**28 Kingsland Road**
**Aylesbury Bucks(GB)**

(72) Inventor: **Longley, Anthony**
**4 Everest Road**
**Crowthorne Berkshire(GB)**

(74) Representative: **Jackson, David Spence et al,**
**REDDIE & GROSE 16, Theobalds Road**
**London, WC1X 8PL(GB)**

(54) Control system for apparatuses in the environment of a disabled person.

(57) An environmental control system for use by severely physically disabled persons has a suck or blow pneumatic input switch device (12) connected through a junction box (23) to a microprocessor-based control unit (11) which controls, in response to operation of the input switch device (12), an indicator (13) with a white lamp (24) and a red lamp (25) in each of fourteen squares, each except the first indicating the operating or non-operating state of a controlled device, the controlled devices being a door catch release (14), an alarm bell (15), external and internal intercom units (16) and (17), a mains appliance switching unit (18), a loudspeaking telephone (19) and a master speaker (22). The intercom units (16) and (17) and the speaker (22) form a controlled two-way intercom system. Controlled devices can be selected for turning on or off by stopping successive illumination of the white lamps (24) in a sequential scan at the chosen square whereupon the red lamp (25) is turned on or off to indicate activation or de-activation. Some of the squares serve also for entering telephone subscriber number digits into a RAM store for automatic display with or without dialling out from the telephone (19). The control signals to the indicator (13) are transmitted by a one-way single-line UART channel from the control unit (11) through a junction box (23). Relay controlled mains switches in the unit (18) are controlled directly from the circuit of the indicator (13).

FIG. 1

EP 0 123 430 A1

0123430

ENVIROMENTAL CONTROL SYSTEM

This invention relates to an environmental control system for use by a disabled person.

Examples of known environmental control systems for severely physically disabled persons are described in British patent specifications no's. 956302 and 1021531. Typically in such known systems a display provides the user with an indication of which one of a plurality of pieces of equipment, for example a radio, a television, a telephone, a page turner and an alarm is in operation under the control of the control system. In particular, where such a system is intended to be used by a severely disabled person, the input means is typically in the form of one or two on/off switches, possibly adapted to be controlled by air pressure by sucking and/or blowing by the user, the operations of the switch or switches being automatically responded to by the systemto provide the user with a method of initiating, controlling and terminating a scanning of the display positions of the display.

According to the present invention there is provided an environmental control system having a control unit and an indicator unit coupled by a channel for indicator control signals to pass along to the indicator unit from the control unit, the control unit including a central processor and the indicator unit including decoding means, the system including an input device adapted to be operable as a switch coupled to the control unit, an intercom circuit controlled by the control unit and including a master acoustic transducer for use adjacent the input device, and at least one remote acoustic transducer, the intercom circuit being capable of providing a speech signal channel from the master transducer to a remote transducer and vice versa, an audible alarm device coupled to the control unit, a door releasing device coupled to the control unit, at least one electrically controllable on/off switching device arranged to be controlled by the control unit and having controlled terminals to which an electrically powered appliance can be manually connected by plug and socket connection, and a telephone control circuit controlled by the control unit and such as to enable a telephone unit incorporating a telephone interface circuit to be so controlled by the control unit as to make and receive telephone calls, the control unit including a substantially permanent memory containing a control program for the central processor unit, a temporary memory, and a plurality of ports coupled to the central processor and arranged to control or sense operation of the input device, the indicator unit, the intercom circuit, the audible alarm device, the door releasing device, and the telephone control circuit, the indicator unit having a plurality of visually distinguishable indications of elements of the system controllable by the control unit and being operable by the control unit in such a manner that a user can select activation of indication of a particular element chosen by the user through the operation of the input device, and the control unit being such that activation of indication of a chosen element of the system is accompanied by the bringing into operation of that element.

The substantially permanent memory is preferably an erasable programmable read-only memory.

Further features of preferred embodiments of the invention are defined in the dependent claims hereinafter.

The invention will now be described by way of example with reference to the accompanying drawings in which:-

Fig. 1 is a diagrammatic illustration of a patient operated enviromental control system embodying the present invention;

Fig. 2 is a front view of a visual indicator display panel of the system of Fig. 1;

Fig. 3 is a block diagram of part of a microprocessor-based control unit of the system of Fig. 1;

Fig. 4 is a block diagram of electronic circuitry associated with the display panel of Fig. 2;

Fig. 5 is a circuit diagram including the microprocessor of the control unit of Fig. 3 and showing control signal logic circuitry therefor;

Fig. 6 is a circuit diagram of clock pulse and interrupt signal generating circuitry of the control unit of Fig. 3;

Fig. 7 is a circuit diagram of a read-only memory of the control unit of Fig. 3;

Fig. 8 is a circuit diagram of a temporary memory of the control unit of Fig. 3;

Fig. 9 is a circuit diagram of a stand-by power source for the temporary memory of Fig. 8;

Figs. 10 and 11 are circuit diagrams of a power supply section of the control unit of Fig. 3;

Fig. 12 is a circuit diagram of an input/output decoder unit of Fig. 3;

Fig. 13 is a circuit diagram of an input port for user input signals, display scan parameter signals, and a UART busy signal;

Fig. 14 is a circuit diagram of display scan parameter setting circuitry;

Fig. 15 is a circuit diagram of an output UART circuit of Fig. 3;

Figs. 16 to 20 are circuit diagrams of parts of an indicator unit of the system of Fig. 1;

Fig. 21 is a circuitry diagram of a junction box of the system of Fig. 1;

Fig. 22 is a circuit diagram of a system and intercom sensor port and an intercom circuit control of the control unit of Fig. 3;

Fig. 23 is a circuit diagram of an alarm and door control circuit of the control unit of Fig. 3;

Fig. 24 is a circuit diagram of an intercom circuit of the system of Fig. 1;

Fig. 25 is a circuit diagram of telephone control circuits of the unit of Fig. 3; and

Figs. 26 to 42 are flow charts illustrating operation of the control system of Figs. 1 to 25.

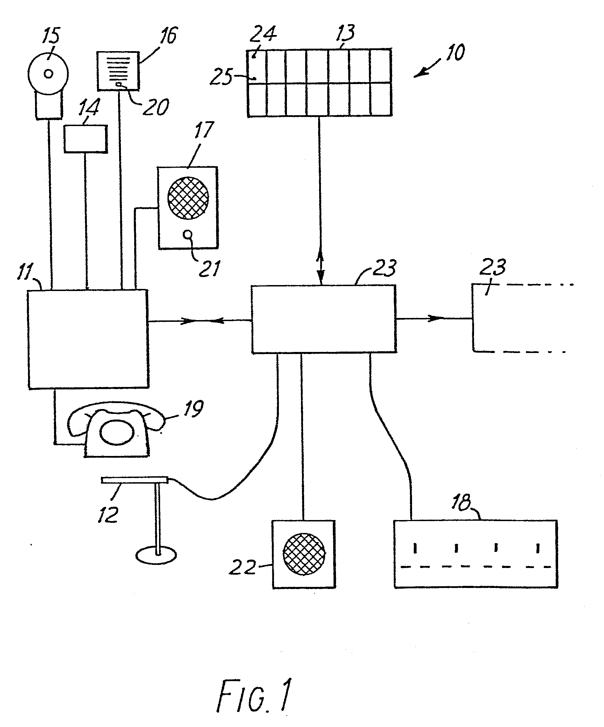

Fig. 1 illustrates diagrammatically a patient operated environmental control system 10 in which a microprocessor-based control unit 11, in response to operation of a switch in a patient operated input switch unit 12, controls a number of electrically actuated devices and an indicator unit 13 which visually informs the user that the various electrically actuated devices are available for actuation or have been brought into operation by the system 10. The input switch unit 12 illustrated is a pneumatic unit constructed to enable a user to close a switch by sucking, or by puffing, at a tubular mouthpiece. Such input switch units are well known. Other patient operated input units can be used instead of the illustrated pneumatic unit 12, as will be apparent to those skilled in the art.

The electrically actuated devices which are illustrated as controllable in the system of Fig. 1 are a door lock release 14, an alarm bell 15, an external intercom unit 16, an internal intercom unit 17, a four way mains switching unit 18 and a loudspeaking telephone 19 of the British Telecom type LST 7A.

The external and internal intercom units 16 and 17 have "press-to-speak" buttons 20 and 21 and can be connected to an audio amplifier circuit in the control unit 11 with a master speaker 22 serving as either a loudspeaker or a microphone for the user of the system, the intercom units 16 and 17 and the master speaker 22 each having a moving coil loudspeaker which can operate as a microphone.

The mains switching unit 18 has a connection (not shown) to a standard three-pin domestic mains outlet socket and contains four sets of relays (not shown), each relay set being arranged to control the connection of a respective three-pin mains outlet socket of the unit 18 to the standard mains outlet socket (not shown). Such mains switching units are well known. The system 10 allows the user to select any one or more of the four outlet sockets of the unit 18 for connection and thus enables the user to control the supplying of power to four pieces of mains operated electrical equipment. For example, the unit 18 may have plugged into it a television receiver, a tape replay system, a standard lamp and an electric heater.

The external intercom unit 16 will usually be mounted adjacent the front door to the user's dwelling and be accessible to a caller wishing to gain entry. The caller can then request entry by speaking to the user through the external intercom unit 16, if the user is "at home", and the user can cause the system 10 to operate the door lock release 14 to allow entry by the caller. The door lock release 14 is so mounted on the door frame (not shown) that the latch of a Yale type lock locates inside a pivoting catch plate (not shown). When the release 14 is energised, a solenoid-operated locking device releases the catch plate which can then rotate if the door is pushed. Such door lock release devices are well known.

To allow the user to operate the system 10 from more than one room of a dwelling, one or more junction boxes 23 may be provided which enable connections to be made from the input switch unit 12, indicator unit 13, mains switching unit 18 and the master speaker 22 to the control unit 11, as shown in Fig. 1.

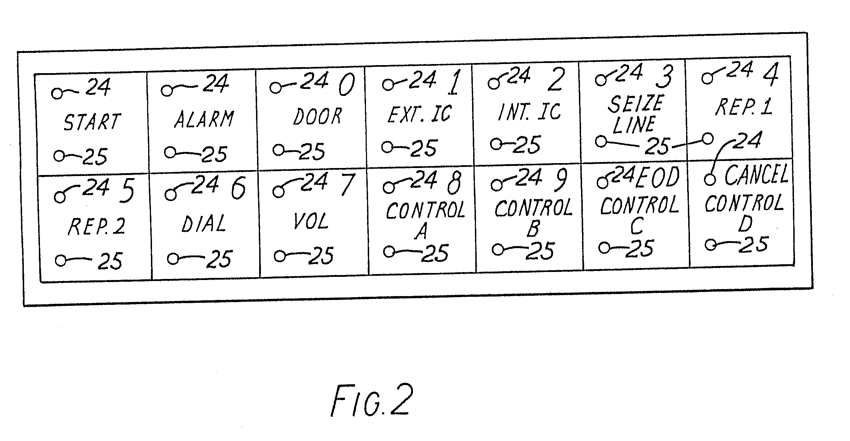

Fig. 2 shows the display panel of the indicator unit 13. The display panel has two rows of seven squares. Each square except a first, START, square is inscribed with an indication of a facility which can be selected by the system 10, and includes a white scan light 24, and a red selection light 25. In use, if any of the facilities is being used, the red light 25 in the corrsponding square of the display panel is illuminated. For example, if the facility indicated by the square inscribed CONTROL A is being used, the red light 25 in that square is illuminated. The four squares inscribed CONTROL A, CONTROL B, CONTROL C and CONTROL D indicate the operation of four mains operated devices plugged into the four-way mains switching unit 18. Thus illumination of the CONTROL A red light 25 may indicate operation of a television receiver. Whenever the user wishes to initiate or terminate operation of a facility, the input switch device 12 is used to signal to the control unit 11 to start a scan of the indicator panel. Every scan starts with the white light 24 of the START square being the only white light illuminated. The other white lights 24 are then illuminated one at a time in turn from left to right in the top row of display squares and then from left to right in the bottom row of display squares. If during a scan the user operates the input switch unit 12 so as to signal selection of the facility associated with the display square whose white scan light 24 is currently illuminated, then the red light 25 of that square is illuminated and the scan ceases, the white light 24 of the START square being illuminated again. The red light 25 of the START square is illuminated only if the mains, which is supplied to the control unit 11 by a cable (not shown) from another domestic three-pin mains supply outlet socket (not shown), fails. To draw attention to this condition the red light 25 of the START square is flashed on and off at regular intervals.

The system described herein is arranged to operate in response to either one of two possible modes of operation of the input switch unit 12, the prevailing mode being determined by the state of a manually operable switch in the control unit 11 which is accessible to a helper. In one of these modes, a scan is initiated by momentary closure of a switch in the input switch device 12 and is halted at a chosen square on the indicator 13 by another such momentary closure. Thus a first momentary closure initiates a scan, and the subsequent momentary closure selects a facility and terminates the scan. In the other scanning mode, a scan is initiated by closure of the switch of the input switch device 12 and continues while the switch is held closed, selection and termination being effected by release of the switch. The first mode of operation is referred to hereinafter as the momentary mode and the second as the held mode.

When the system is switched on by manual closure of a main switch on the control unit 11, provided the indicator 13 is properly connected to the control unit 11, the white lamp 24 of the START square, and no other lamp, will be illuminated. If then a scan is begun and the ALARM square is selected, the red lamp 25 of the ALARM square, the red lamp 25 of the DOOR square, the alarm bell 15 and the door catch release device 14 are all energised. The white lamp 24 of the DOOR square, which is automatically energised after selection of the ALARM square, is then extinguished and the white lamp 24 of the START square is energised again and remains so until another scan is begun. The alarm bell 15 and the door catch release device 14, and the red lamps 25 of the corresponding indicator squares, can be de-energised by subsequent selection of the ALARM square. Alternatively, the door catch release device 14 and the red lamp 25 of the DOOR square can be de-energised by selection of the DOOR square, leaving the alarm bell 15 and its red lamp 25 energised.

The indicator 13 produces a low tone for 0.1 seconds each time a new scan begins and the white lamp 24 of the ALARM square is energised, and whenever the white lamp 24 of the START square is energised during a continuing scan. Whenever any other white lamp 24 is energised during a scan, a high tone is produced for 0.1 seconds by the indicator 13. The 0.1 second high tone is produced for energisation of the ALARM square white lamp 24 after the first such energisation in a continuing scan.

The effect of selection of any of the DOOR, EXT.IC, INT.IC, SEIZE LINE, REP1, REP2, DIAL, VOL, CONTROL A, CONTROL B, CONTROL C and CONTROL D squares depends on whether the system is in a dialling mode or not. If the system is not in the dialling mode, the door catch release device 14 can be controlled by selection of the DOOR square, communication with the external intercom 16 and the internal intercom 17 can be controlled by selection of the EXT. IC and INT. IC squares respectively, seizure of a telephone line by the SEIZE LINE square, various telephone facilities by selection of the REP1, REP2 and DIAL squares, the output volume at the telephone 19 by the VOL square, and four mains powered pieces of equipment connected to the mains switching unit 18 by the four CONTROL squares. When the system is in the dialling mode, selection of any of the DOOR to CONTROL B squares enters an associated digit into a temporary memory in the control unit 11. Selection of the CONTROL C square arranges the stored digits for use in response to subsequent selection of the REP1, REP2 or DIAL squares when not in the dialling mode, and selection of the CONTROL D square cancels the dialling mode.

The control unit 11 contains secondary batteries which are used to power the system if the a.c. mains to which the system is connected fails. Since the mains powered pieces of equipment connected through the mains switching unit 18 will not operate if the mains fails, the control unit 11 automatically opens the mains connections to such equipment at the mains switching unit 18 when mains failure occurs, and also switches off the red lamps 25 of the four CONTROL squares. The indicator 13 automatically indicates mains failure by switching on the red lamp 25 of the START square twice for 0.1 seconds with an interval of 0.1 seconds, and repeating this indication at intervals of 1 second. This flashing of the START lamp is accompanied by a high tone beep at the indicator 13 and a 150 Hz tone beep at the internal intercom 17 unless the CONTROL D square is selected, which in these circumstances acts to cancel these audible signals.

When an incoming telephone call occurs, which is indicated in the conventional way by the ringing of the telephone 19, the call can answered by the user selecting the SEIZE LINE square, the dialling mode being cancelled beforehand, if necessary.

To make an outgoing telephone call to a subscriber number not stored already in the control unit 11, the user clears all selected telephone functions, then selects the DIAL square to put the system into the dialling mode. The user then selects the digits constituting the subscriber number. If a mistake is made in this process, the CANCEL square can be selected to clear th dialling mode, and a fresh start made. When the subscriber number is successfully entered, the End of Dialling square, inscribed EOD, (i.e. CONTROL C) is selected to shift the subscriber number into a DIAL buffer memory. Then the SEIZE LINE square is selected to simulate lifting of the handset of a telephone, and finally DIAL is selected again, which results in automatic transmission of the necessary telephone dialling signals with the correct timing from the telephone 19.

The volume of received speech at the telephone 19 during a telephone call can be set to a high level by selection of the VOL square when the system is not in the dialling mode.

Selection of REP1 and then DIAL enables the user to enter a subscriber number into a first repertory store. Similarly, selection of REP2 then DIAL enables entry into a second repertory store. To call either of these repertory numbers, the indicator 13 is cleared of selection of DIAL, then SEIZE LINE followed by REP1 or REP2 is selected. Automatic dialling out of the chosen repertory number then takes place.

Whenever a subscriber number is fully entered in the dialling mode, whether for immediate use by means of DIAL only or for subsequent use by means of REP1 or REP2, the entered number is "replayed", immediately after completion of entry, at the indicator 13 by the blinking in sequence of the white lamps 24 corresponding to the digits making up the subscriber number.

The indicator 13 also indicates the subscriber number being called whenever telephone digit signals are transmitted by the telephone 19.

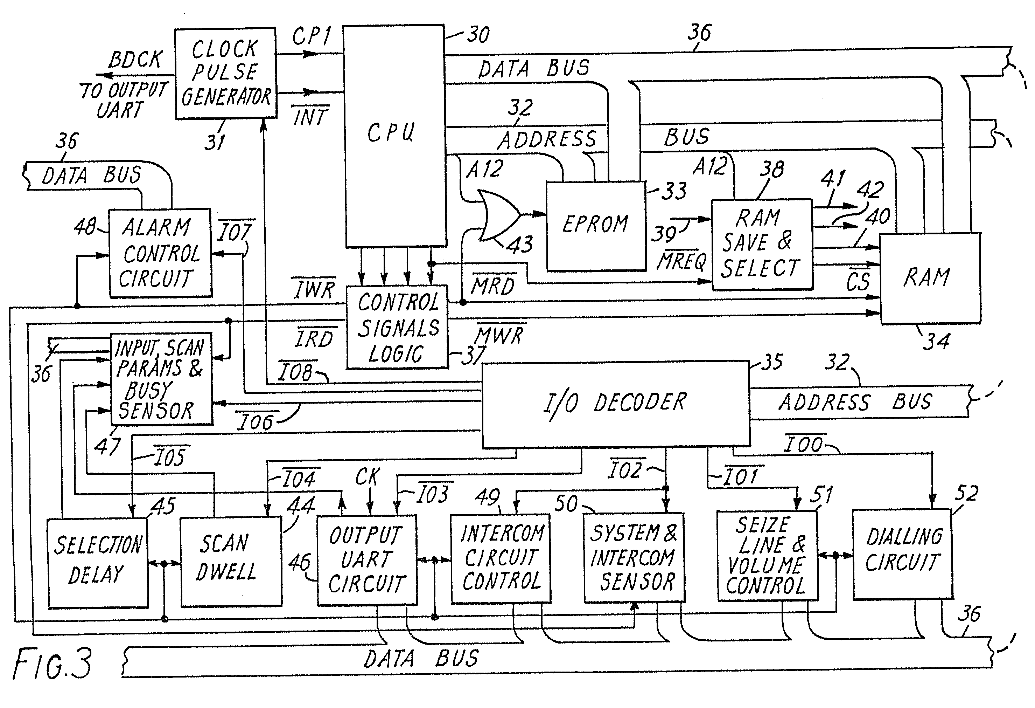

Fig. 3 shows in block form the control unit 11. The control unit 11 has a central processor unit 30 which receives clock pulses CP1 and interrupt signals $\overline{INT}$ from a clock pulse generator 31. An address bus 32 from the CPU 30 is connected to an Erasable Read Only Memory 33, a Read Only Memory 34, and an Input/Output Decoder 35. A data bus 36 also connects the CPU 30 to the EPROM 33 and the RAM 34 and to a number of input or output interface circuits. The EPROM 33 stores a program which controls the operation of the CPU 30. The RAM 34 is used as a temporary memory for data manipulated by the CPU 30. In addition to addresses and data for the buses 36 and 32, the CPU 30 produces four control signals. All four of these CPU control signals are supplied to a control signals logic 37 where they are combined to produce interface read and write signals $\overline{IRD}$ and $\overline{IWR}$ and memory read and write signals $\overline{MRD}$ and $\overline{MWR}$. One of the CPU control signals, a memory request signal $\overline{MREQ}$, is supplied directly to a RAM save and select circuit 38 which produces a chip select signal $\overline{CS}$ for the RAM 34 whenever the twelfth line A12 of the address bus 32 and the memory request signal $\overline{MREQ}$ are concurrently active during normal power supply conditions. If an abnormal power supply condition is signalled to the RAM save and select circuit 38 on an input terminal 39, or no power is being supplied to the system as a whole, the circuit 38, which contains a small secondary battery, supplies a memory saving voltage to the RAM 34 through a connection 40. The circuit 38 also produces inhibiting and resetting signals at output terminals 41 and 42 for other parts of the system. The chip select signal CS for the RAM 34 is inhibited whenever the battery of the circuit 38 comes into use.

The address bus line A12 and the memory read signal $\overline{MRD}$ are gated by an OR gate 43 to produce a chip select signal for the EPROM 33. Read and write commands are supplied to the RAM 34 by the control signals logic in the form of the memory read and memory write signals $\overline{MRD}$ and $\overline{MWR}$.

The input and output interface circuits are arranged to be controlled as nine ports, the I/O decoder 35 generating nine respective interface port enabling signals $\overline{I00}$, $\overline{I01}$, $\overline{I02}$, $\overline{I03}$, $\overline{I04}$, $\overline{I05}$, $\overline{I06}$, $\overline{I07}$ and $\overline{I08}$. Two of these signals, $\overline{I04}$ and $\overline{I05}$, serve to trigger respectively a scan dwell monostable circuit 44 which serves to determine how long each white lamp 24 of the indicator 13 remains energised during a scan, and a selection delay monostable circuit 45 which serves to determine how long it is necessary for the user to signal selection of an indicator square before the system accepts the selection as valid. The conditions of the monostable circuits 44 and 45, the state of the switch of the input switch device 12, and the condition of a UART in an output interface circuit 46, represented by a block inscribed OUTPUT UART CIRCUIT, are supplied to an input port circuit 47 which includes port 6 and is represented in Fig. 3 by a block inscribed INPUT, SCAN PARAMS and BUSY SENSOR. The input port circuit 47 receives from the output UART circuit 46 a signal indicating whether or not the UART is

0123430

busy transmitting data to the indicator 13, which includes a UART operated in the receiver mode. Those skilled in the art will know that UART stands for Universal Asynchronous Receiver Transmitter. The UARTs used in the system being described in this example are of the type which generate and decode their own stop and start and parity bits. The rate of transmission of data bits by the UART of the circuit 46 is determined by a baud rate clock pulse train BDCK generated by the clock pulse generator 31 and supplied to the clock input terminal CK of the circuit 46.

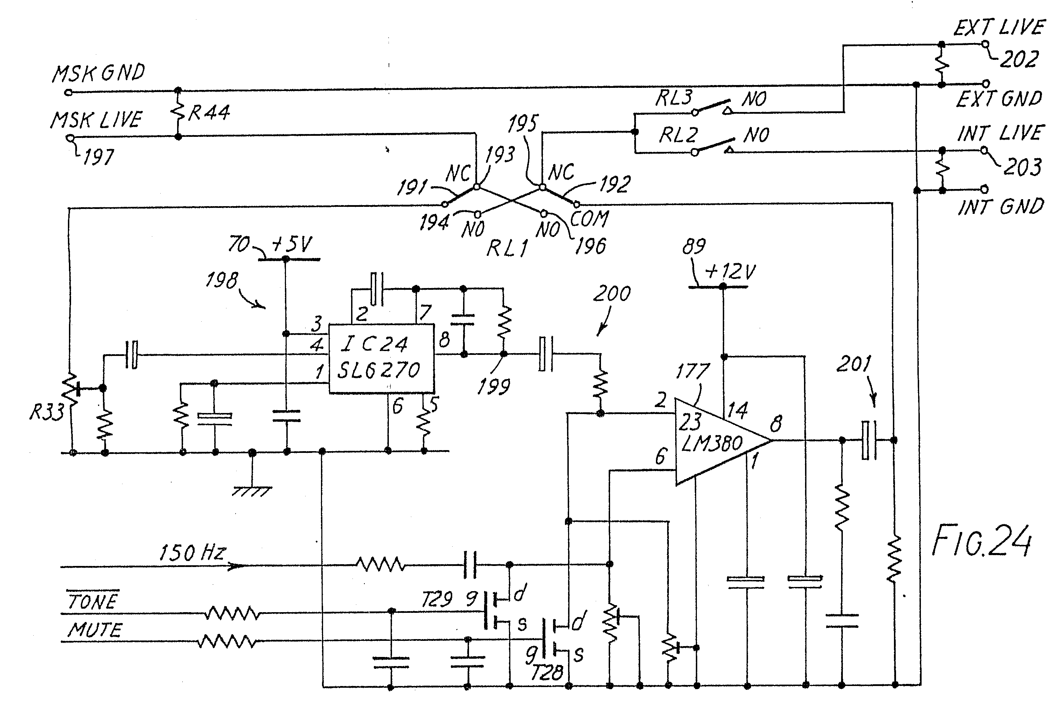

The interface port enabling signal IO7 is supplied to port 7, which controls a circuit 48 represented in Fig. 3 by a block inscribed ALARM CONTROL CIRCUIT. The port 7 circuit 48 controls energisation of the alarm bell 15 and the door catch release device 14, data written from the data bus 36 to port 7 when the interface write signal $\overline{IWR}$ is active determining that a selected one or both of the alarm bell 15 and the door catch release device 14 is or are energised or de-energised. Port 7 also controls relays which determine direction of communication in the intercom circuit, shown in Fig. 24, and connection of the internal intercom 17.

The interface port enabling signal IO6 is supplied to the input port circuit 47 which is read by the CPU 30 when the signal $\overline{IO6}$ and the interface read signal $\overline{IRD}$ are both active, port 6 being connected to the data bus 36.

When the UART of circuit 46 is not busy and data is to be transmitted to the indicator 13, the port 3 enabling signal $\overline{IO3}$ is made active and parallel data is written into the UART, which is port 3, when the interface write signal $\overline{IWR}$ is active concurrently with the enabling signal $\overline{IO3}$.

Port 2 is included partly in an intercom circuit control 49 and partly in a system and intercom sensor circuit 50. The intercom circuit control 49 determines whether the intercom circuit, shown in Fig. 24 and included within the control unit 11, operated to allow a 150 Hz tone to be generated at an intercom station (i.e. at 16, 17 or 22), or to allow speech to be transmitted, and to determine whether the external intercom 16 is connected or not. Port 2 also serves in circuit 49 to generate a 150 Hz square wave for generating the 150 Hz tone. Data is latched into the part of port 2 which is included in circuit 49 when the enabling signal $\overline{IO2}$ and the interface write signal $\overline{IWR}$ are both active. The system and intercom sensor circuit 50 receives signals indicating whether or not the indicator 13 is properly connected to the control unit 11 and whether or not the mains supply is present, and signals representative of the respective states of the push buttons 20 and 21 of the external and internal intercoms 16 and 17. These four input signals are read by port 2 when the enabling signal $\overline{IO2}$ and the interface read signal $\overline{IRD}$ are both active.

Port 1, enabled by the signal $\overline{IO1}$, is included in a telephone line seizing and volume controlling circuit 51 inscribed SEIZE LINE & VOLUME CONTROL. Data controlling these functions is written into port 1 from the data bus 36 when the signal $\overline{IO1}$ and the interface write signal $\overline{IWR}$ are both active. Similarly, signals representing the ten telephone digits are written into port 0, which is included in a dialling circuit 52, when the enabling signal $\overline{IO0}$ and the interface write signal $\overline{IWR}$ are both active.

The interface port enabling signal $\overline{IO8}$ is used to reset a bistable circuit included in the clock pulse generator 31 and arranged to supply the interrupt signal $\overline{INT}$ to the CPU 30.

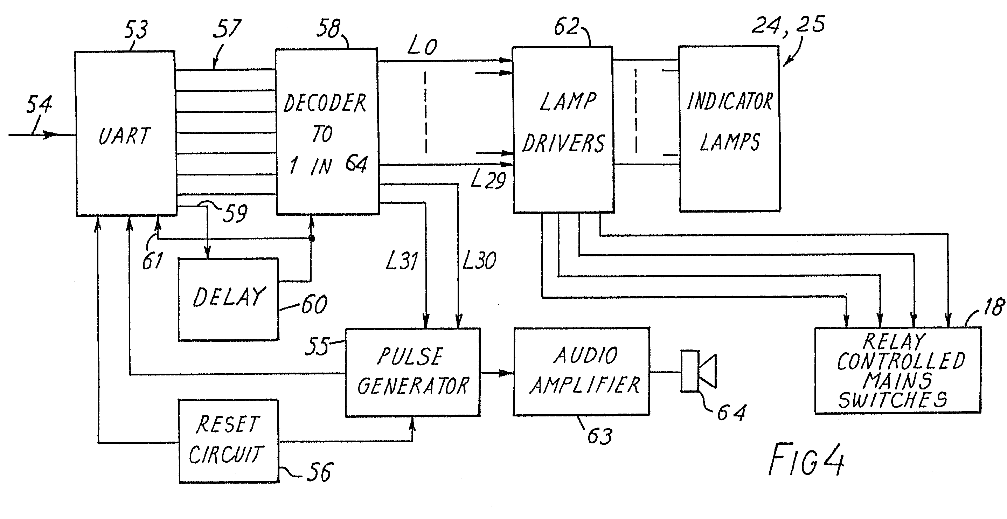

Fig. 4 shows in block form the indicator 13. Serial character codes with start, stop and parity bits are generated by the transmitting UART of the output UART circuit 46 of the control unit 11 and are supplied over a single data line to the receiver UART 53 of the indicator 13 at its serial data input terminal 54. Bit rate clock pulses are supplied to the receiver UART 53 by a pulse generator 55. The receiver UART 53 and the pulse generator 55 are reset to initial states simultaneously by resetting signals supplied by a reset circuit 56 whenever the system as a whole is switched on. Each time that the receiver UART 53 decodes a valid serial character code into the corresponding parallel data character code, the parallel data character code is applied at the parallel output terminals 57 of the UART 53 to a decoder circuit 58, inscribed DECODER TO 1 IN 64 since the decoder circuit 58 in effect decodes the parallel character output of the UART into one out of a possible 64 codes. The parallel character at the terminals 57 is latched into the decoder circuit 58 shortly after a "valid output" signal is supplied from a terminal 59 of the UART 53 to a delay circuit 60, the delayed signal being also supplied to the reset terminal 61 of the UART 53 to clear the UART 53 to receive the next serial character code at the terminal 54.

Only sixty of the possible 64 character codes are used. Of these, twenty-eight are used to turn on respective ones of the fourteen white lamps 24 and fourteen red lamps 25 of the indicator 13, through respective lamp driver circuits 62, and another twenty-eight are used to turn off the respective ones of the lamps 24 and 25. For this purpose, the decoder circuit 58 is connected to the lamp driver circuits 62 by twenty-eight lines designated L0 to L13 for the white lamps 24 and L16 to L29 for the red lamps 25. The remaining four codes are used to control the states of two electronic switches in the pulse generator 55, two of the codes determining the signal level on a line L30 to one of these electronic switches, and the other two codes determining the signal level on a line L31 to the other electronic switch. The two electronic switches control a filter circuit to determine what frequency periodic signal generated by the pulse generator 55 is supplied to an audio amplifier 63 which drives the loudspeaker 64 of the indicator 13. When the received code is such that the line L30 is at its active level, a low tone is generated by the loudspeaker 64, and when the received code is such that the line L31 is at its active level, a high tone is generated by the loudspeaker 64. These low and high tones are the audible signals provided to indicate aurally the stepping of the indicator scan from one white lamp 24 to another, as described hereinbefore.

The lamp driver circuits for the red lamps 25 of the four CONTROL squares of the indicator 13, which are controlled by the signal levels on the lines L26 to L29 are provided with corresponding respective relay drive circuits which are connected through the junction box 23 (Fig. 1; not shown in Fig. 4) to the mains switching unit 18 which contains four relay-controlled mains switches, each one of these four relay-controlled switches being controlled by a respective one of the relay driver circuits associated with the red lamps of the CONTROL squares so that whenever any of these four red lamps is energised, the corresponding relay-controlled switch is closed in the mains switching unit 18.

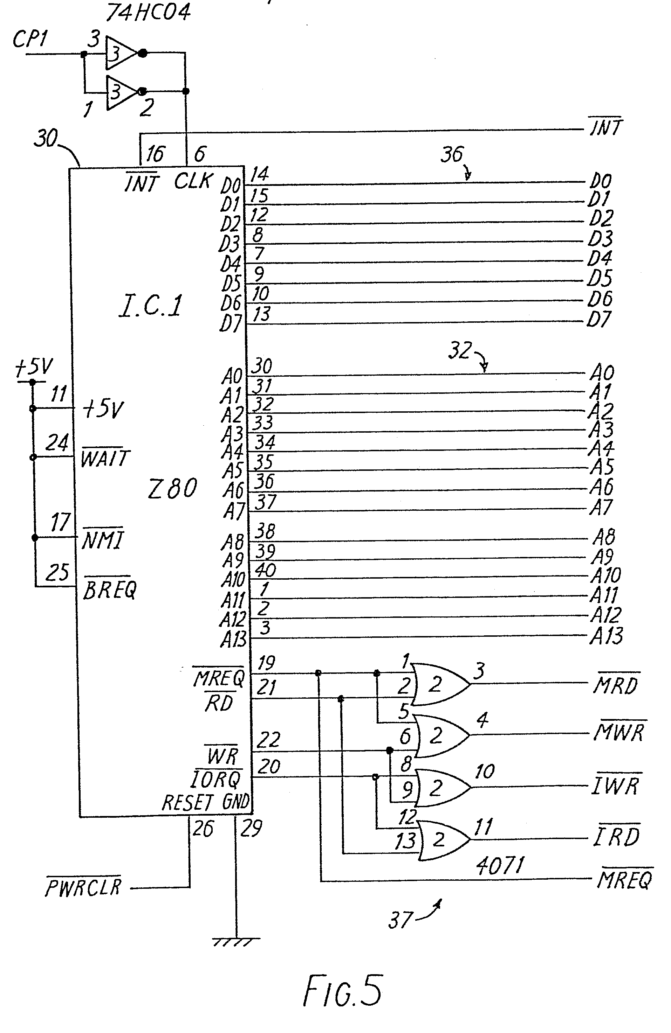

Fig. 5 shows the CPU 30, which in this example is a Z80 microprocessor available from Zilog of Los Altos, California 94022, United States of America, and the control signals logic 37 which consists of four OR gates operating on the four control signals $\overline{MREQ}$, $\overline{RD}$, $\overline{WR}$ and $\overline{IORQ}$ provided at pins 19, 21, 22 and 20 of the CPU 30. It will be appreciated by those skilled in the art that since the microprocessor generated control signals are low active, the OR gates carry out AND logic operations to produce the four low-active control signals provided by the control signals logic 37. The data bus 36 consists of eight data lines D0 to D7 connected as shown to eight data in/out pins of the CPU 30, and the address bus 3 has fourteen lines A0 to A13 conneced as shown to fourteen address pins of the CPU 30. The CPU clock input CP1 and the interrupt signals $\overline{INT}$ are supplied to pins 6 and 16 of the CPU 30, the clock pulses being applied through suitable buffer amplifiers, in this case a parallel pair of 74HC04 amplifiers. A high signal level, +5 volts for the Z80, is supplied to pins 11, 24, 17 and 25 to supply the CPU 30 and to de-active permanently the inputs for $\overline{WAIT}$, $\overline{NMI}$ and $\overline{BREQ}$ which are not used. The reset signal pin 26 receives a resetting signal $\overline{PWRCLR}$ from the RAM save and select circuit 38 whenever the system is switched on.

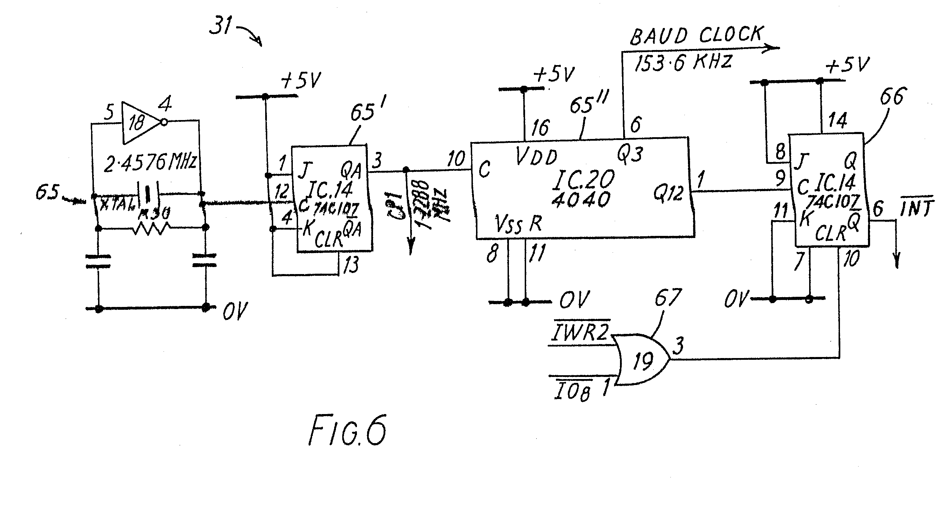

Fig. 6 shows the integrated circuits which make up the clock pulse generator 31, and their interconnections. A quartz crystal controlled oscillator 65 generates a 2.4576 megahertz oscillation which is divided by 2 by a first J-K bistable circuit 64, type 74C107, to produce the CPU clock signal CP1 at 1.2288 megahertz, which is also supplied to a counter-divider circuit 63, type 4040, which produces a baud clock for the transmitter UART at 153.6 kilohertz, and an interrupt clock signal at 300 hertz which is supplied to the clock input of a second J-K bistable circuit 66, type 74C107, the $\overline{Q}$ output of which is the interrupt signal $\overline{INT}$. The second bistable circuit 66 is reset by the gating of the enabling signal I08 by the interface write signal $\overline{IWR}$, these two signals being supplied to an OR gate 67 having its output terminal connected to the resetting (CLR) terminal of the second bistable circuit 66. As a result of the program in the EPROM 33, the output from the OR gate 67 goes low almost immediately after the $\overline{Q}$ output of the bistable circuit 66 goes low, and consequently the interrupt signal $\overline{INT}$ is a low pulse and is generated every 1/300 of a second.

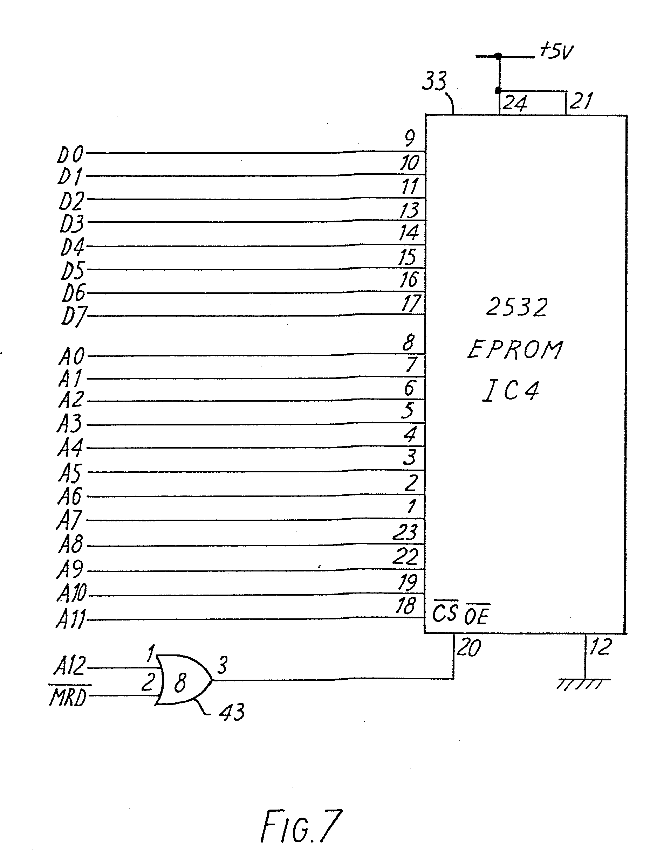

Fig. 7 shows the EPROM 33, which in this example is a type 2532, and the OR gate 43. The eight lines D0 to D7

of the data bus 36 are all connected to respective data output pins 9 to 17 of the EPROM 33, and the first eleven lines, A0 to A10, of the address bus 32 are connected to respective address input pins of the EPROM 33 as shown in Fig. 3. The output of the gate 43 is supplied to the output enable pin 20, and the address bus line A11 is connected to the chip select input pin 18.

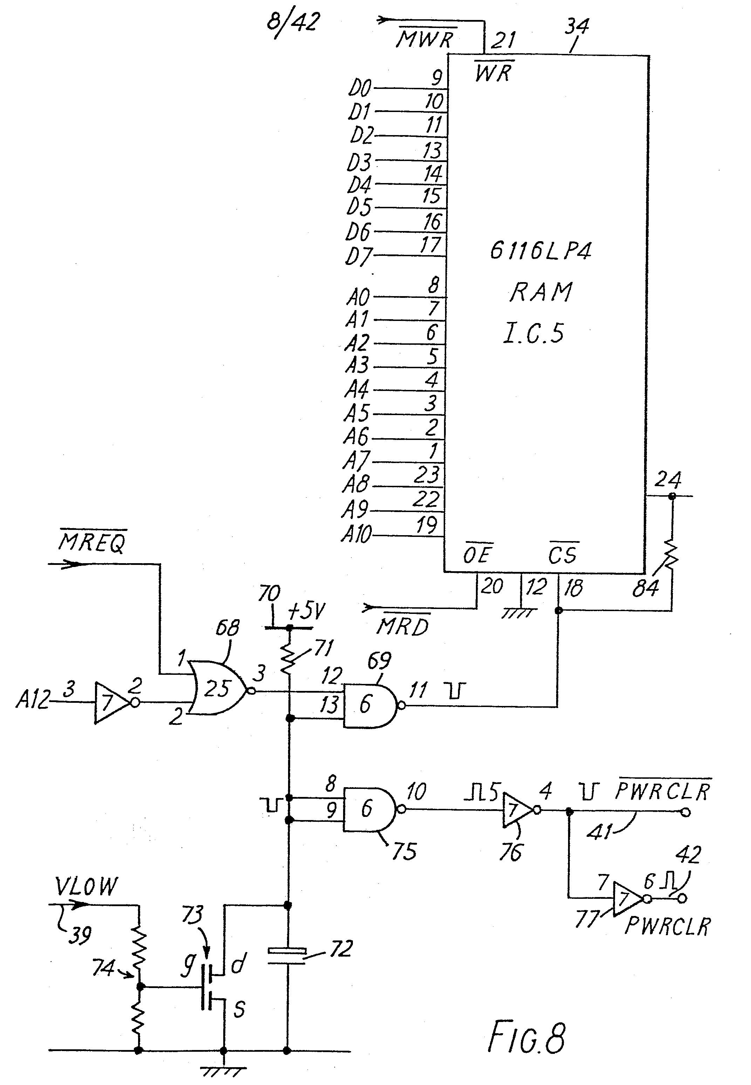

Fig. 8 shows the RAM 34 and part of the RAM save and select circuit 38. All eight lines D0 to D7 of the data bus 36 are connected to respective data input/output pins 9 to 17 of the RAM 34, which is a type 6116LP4. The first eleven lines, A0 to A10, of the address bus 32 are connected to respective address input pins of the RAM 34, and the memory write control signal $\overline{MWR}$ and the memory read control signal $\overline{MRD}$ from the control signals logic 37 are supplied to the write and output enable pins 21 and 20 of the RAM 34. The memory request signal $\overline{MREQ}$ from the CPU 30 and the thirteenth address bus line A12 are coupled to the RAM save and select circuit 38, the $\overline{MREQ}$ signal directly as one input to a NOR gate 68, and the A12 address line through an inverter 69 to provide the other input for the NOR gate 68. Consequently the output from the NOR gate 68 will be high only if the $\overline{MREQ}$ signal is active, i.e. low, and the signal on the A12 address bus line is high. Thus a high output from the NOR gate 68 can only occur when the EPROM 33 is not enabled. The output produced by the NOR gate 68 is supplied as one input to a two-input NAND gate 69, the other input to which is provided by a +5 volt supply line 70 through a resistor 71, so that normally this input is high and the NAND gate 69 acts as an inverter for the output of the NOR gate 68. The output of the NAND gate 69 is supplied to the chip select pin 18 of the RAM 34 and is low only when the output from the NOR gate 68 is high. Thus the RAM 34 is selected by the CPU 30 making the $\overline{MREQ}$ signal active when the A12 address bus line is high. To close the NAND gate 69 for a suitable time immediately following switching on of the system, the resistor 71 is coupled to ground by an electrolytic capacitor 72. The NAND gate 69 can also be closed by the closure of a switch in parallel with the capacitor 72, this switch being provided in the form of a field effect transistor 73 connected as shown and having its gate terminal, indicated at g, connected to a tapping point on a potential divider 74 which receives a voltage signal VLOW at the input terminal 39 of the RAM save and select circuit 38. The signal VLOW is high provided that a nominal +12 volts supply level in the control unit 11 does not fall below about 8 volts. With VLOW high, the gate of the FET73 is sufficiently positive to prevent conduction between the source s and drain d. When VLOW falls to a low level, corresponding to the nominal +12 volts level being slower than +8 volts, the FET73 conducts, thereby short-circuiting the capacitor 72 and applying a low signal, substantially ground potential, as the gating input to the NAND gate 69, which therefore supplies a high signal to the chip select pin 18 of the RAM 34.

The voltage across the capacitor 72 is also used to develop two resetting and inhibiting signals, $\overline{\text{PWRCLR}}$ and PWRCLR at the output terminals 41 and 42 respectively, the voltage being shaped and buffered by a series combination of NAND gate 75 and an inverter 76 to provide the low active signal $\overline{\text{PWRCLR}}$, which is inverted by a further inverter 77 to provide the high active signal PWRCLR. The NAND gates 69 and 75 are provided on one and the same integrated circuit, I.C.6 in the drawings. Other I.C. numbers are also shown. The NAND gate I.C.6 is a type 4093 in this example.

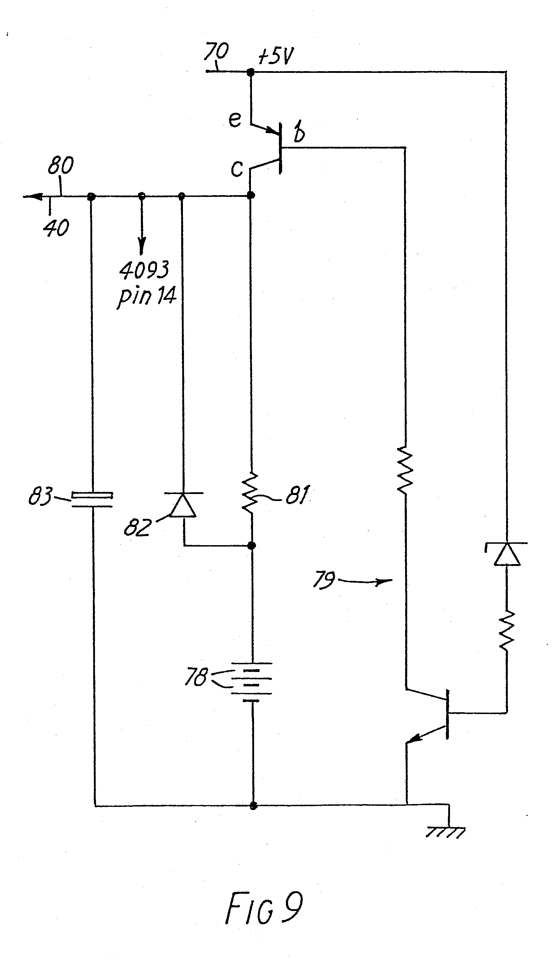

The RAM 34 has a pin 24 to which +5 volts is normally supplied to hold the data stored in the RAM 34. A minimum of substantially +3.5 volts must be supplied to this pin 24 to preserve the stored data. When the system as a whole is turned off, or the nominal +12 volts supply level falls to the point at which the FET73 short circuits the capacitor 72, the normal +5 volts falls to below +3.5 volts and a small secondary battery, providing +3.6 volts, in the RAM save and select circuit 38 comes into operation. This 3.6 volt battery is shown at 78 in Fig. 9 which also shows the normal +5 volt line 70, a junction transistor circuit 79 which isolates the +5 volt line 70 from the RAM supply line 80 whenever the voltage on the line 70 falls below about +3.5 volts. The battery 78 is trickle charged from the RAM supply line 80 through a rsistor 81 when the +5 volts line 70 is at +5 volts, a diode 82 providing a by-pass path for current from the battery 78, and an electrolytic capacitor 83 de-coupling the line 80. The line 80 includes the terminal 40 {Fig. 3) which is connected directly to the pin 24 of the RAM 34, and to pin 14 of I.C.6 so that the NAND gate 69 can provide a high output signal. A resistor 84 couples the chip select pin 18 of the RAM 34 to its pin 24 to ensure that the chip select pin is high unless the output from the NAND gate 69 is low.

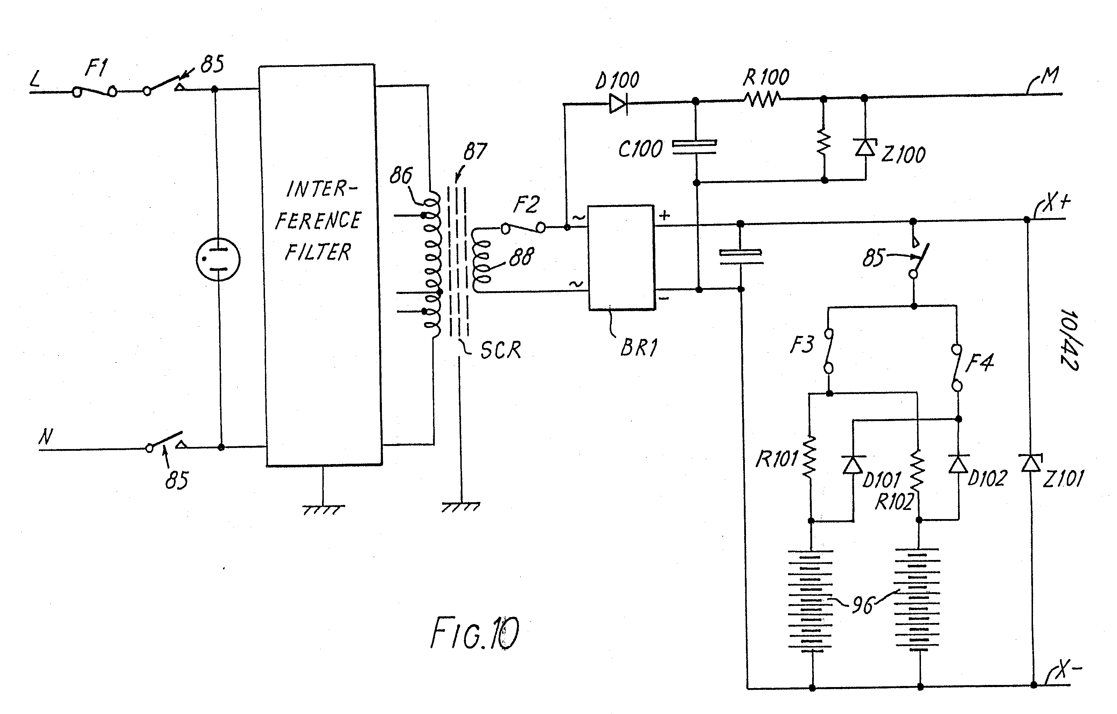

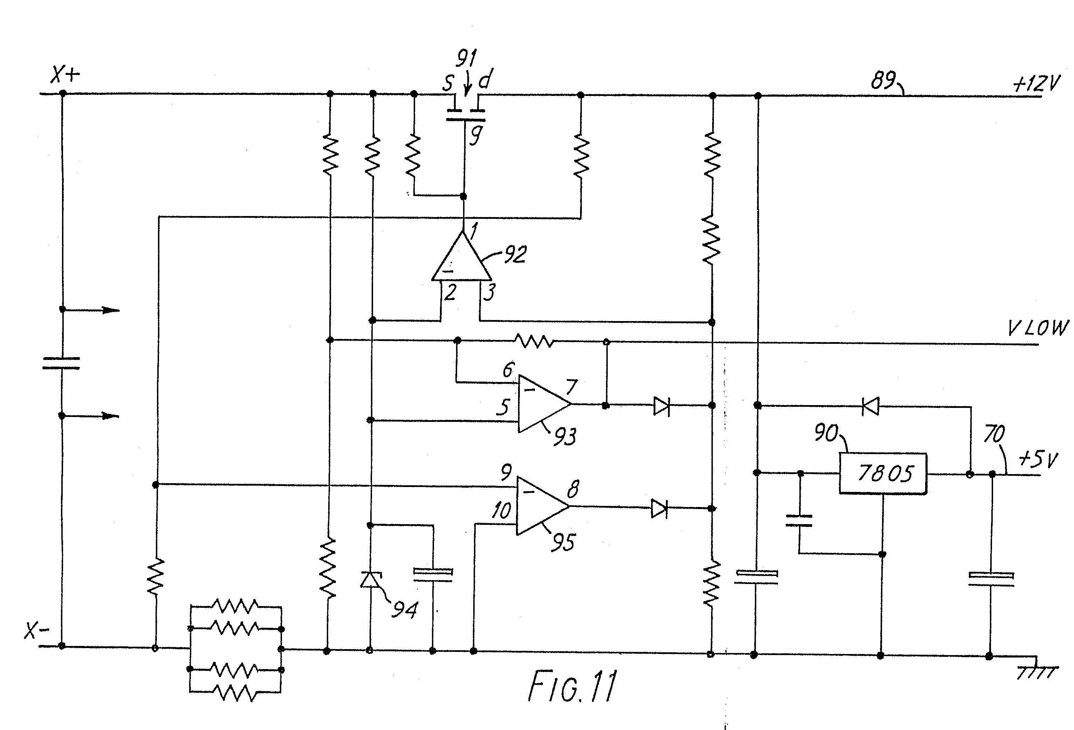

Figs. 10 and 11 show the power supply circuitry of the control unit 11. In Fig. 10, the live (L) and neutral (N) input terminals of the a.c. mains supply socket (not shown) of the control unit 11 are shown, the live terminal L being connected through a first mains fuse F1 to one pole of a three pole main on/off switch 85, and the neutral terminal N being connected directly to another pole of the switch 85. A neon indicator lamp 86 is connected between these two poles at the output side of the switch 85, as shown, in parallel with an interference filter which couples the line and neutral output poles of the switch 85 to the primary winding 86 of a step-down transformer 87 having an earthed screen SCR. The secondary winding 88 provides an a.c. supply of 13.5 volts, 4 amps, through a second fuse F2 to a bridge

rectifier BR1 which provides about +18 volts on a d.c. positive line X+ relative to a d.c. negative line X-. This d.c. supply is regulated by a regulator circuit shown in Fig. 11 to provide +12 volts on a supply line 89. A monolithic regulator circuit 90, type 7805, is connected as shown to the line 89 and ground to provide +5 volts on the line 70. The voltage on the line 89 is controlled by a field effect transistor 91 in response to differential voltage sensing by a differential amplifier 92, of the type LM324 in this example. Another such amplifier 93 provides the voltage level indicating signal VLOW by comparing a fraction of the voltage on the line X+ with a zener voltage provided by a zener diode 94. It will be understood that the zener diode 94 ceases to conduct if the voltage on X+ falls below a certain value, and that then the amplifier 93 will produce a low output since its non-inverting input will be the full voltage on X+ whereas its inverting input will be a fraction of that. The values of resistors in the regulator circuit of Fig. 11 are chosen to ensure that the FET 91 saturates if the voltage on X+ falls below +12 volts. Supplies for the two differential amplifiers 92, 93 and a third, 95, on the same integrated circuit are provided directly from the lines X+ and X-. The amplifier 95 and the resistors coupled thereto serve to adjust the voltage at the non-inverting input terminal of the first amplifier 92 in response to variation in the voltage on the live X-relative to the line 89.

The power supply circuit includes a pair of 12 volt secondary batteries 96 which, as shown in Fig. 10, are connected between the lines X+ and X- when the main switch 85 is closed. If the bridge rectifier BR1 is supplying more than substantially +12 volts on X+, two diodes D101 and D102 are non-conducting and the batteries 96 are trickle charged through resistors R101 and R102 and fuses F3 and F4 respectively from the line X+. If the bridge rectifier BR1 supplies slightly less than +12 volts on X+, the diodes D101 and D102 conduct and the batteries 96 provide substantially +12 volts on X+. A zener diode Z101 connected across X+ and X- limits the voltage between these lines to 27 volts.

A signal indicating whether or not the a.c. mains supply is present is provided on a line M by a zener diode Z100 connected between the X- line and a half-wave rectifier circuit consisting of a diode D100 in series with a parallel combination of an electrolytic capacitor C100 and a voltage divider resistor chain, the zener diode Z100 being in series with the diode D100 and one of the resistors, R100, as shown in Fig. 10. The anode of the diode D100 is connected to one end of the secondary winding 88 through the fuse F2, so that if there is no mains a.c. present, the voltage on line M falls from a high level to that on line X-. The line M is connected to the system and intercom sensor circuit 50.

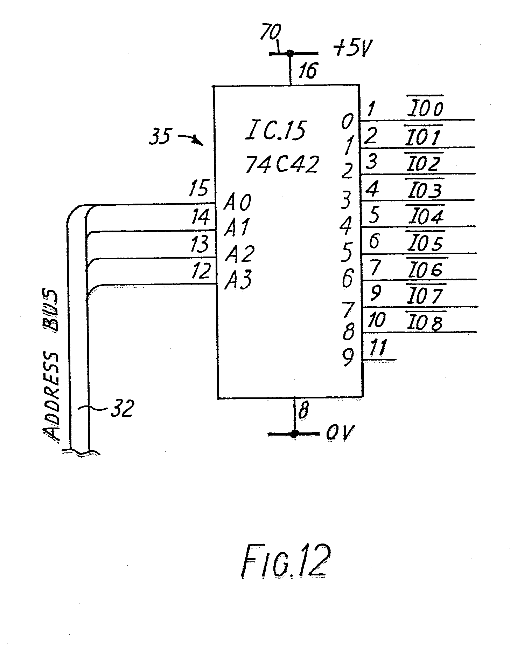

Fig. 12 shows the integrated circuit, I.C.15, which forms the I/O decoder 35 of Fig. 3, and its connections to the address bus 32, ground, and +5 volts supply line 70. The nine interface port enabling signals $\overline{I00}$ to $\overline{I08}$ are provided at output pins 1 to 7, 9 and 10 of the decoder 35, and four address bus lines A0, A1, A2 and A3 are connected to respective input pins 15, 14, 13 and 12. The integrated circuit is a type 74C42 four line to ten line decoder, the output $\overline{I09}$ on the output pin 8 not being used.

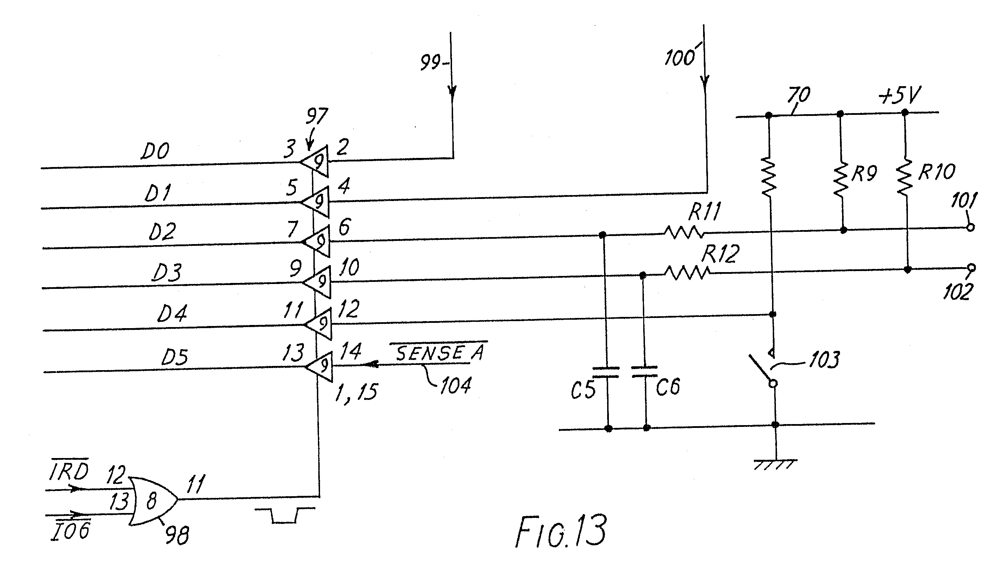

Fig. 13 shows the input, scan params and busy sensor circuit 47 of Fig. 1. Six data lines D0 to D5 of the data bus 36 are connected to the six output terminals of port 6 in the circuit 47, port 6 consisting of six tri-state buffers 97 provided by an integrated circuit I.C.9. The buffers 97 are enabled when a low signal is applied to pins 1 and 15 of I.C.9 by an OR gate 98 having two input terminals which are pins 12 and 13 of an integrated circuit I.C.8. The signals applied to pins 12 and 13 of I.C.8 are the interface read signal $\overline{IRD}$ and the port 6 enabling signal $\overline{IO6}$, both of which are low active signals.

The two buffers 97 connected to the data lines D0 and D1 are supplied with the compliment output signals produced by respective monostable circuits in the scan dwell and selection delay circuits 44 and 46, these signals being supplied by lines 99 and 100 respectively. The input to the D0 buffer of port 6 is low during the time that the scan dwell monostable circuit is in its set state. Similarly, the input to the D1 buffer of port 6 is low during the time that the selection delay monostable circuit is in its set state. Port 6 can also receive input signals from two, user-operated input switching devices such as the device 12 of Fig. 1, each comprising a single normally open switch (not shown) connected between the system ground and either an input terminal 101 or an input terminal 102. The terminals 101 and 102 are coupled through respective resistors R11 and R12 to the input terminals of the D2 and D3 buffers of port 6, these being pins 6 and 10 of I.C.9. The input switch terminals 101 and 102 are also coupled by respective resistors R9 and R10 to the +5 volts line 70, so that the D2 and D3 buffer 97 supply the high signal level to the D2 and D3 data lines when port 6 is enabled and read, unless one or other of the input switches grounds the corresponding terminal 101 or 102. In use, only one input switch is used, and a further, test switch (not shown) which is manually operable by a helper may be connected to the input terminal 101 to allow testing of the system. A further switch 103 connected in series with a resistor to the +5 volts line 70 and directly to the input terminal pin 12 of the D4 buffer of port 6 is provided for setting the scanning mode. This scan mode switch 103 is manually operable and when it is open, as shown, the system operates in the momentary mode, and when closed, the system operates in the held mode, referred to hereinbefore. When read, the D4 buffer provides a high signal for the momentary mode and a low signal for the held mode. The D5 buffer receives a low active signal $\overline{\text{SENSE A}}$ which is produced by the transmitting UART of the output UART circuit 46 on a line 104 to indicate that the UART is ready to receive a fresh input of parallel data.

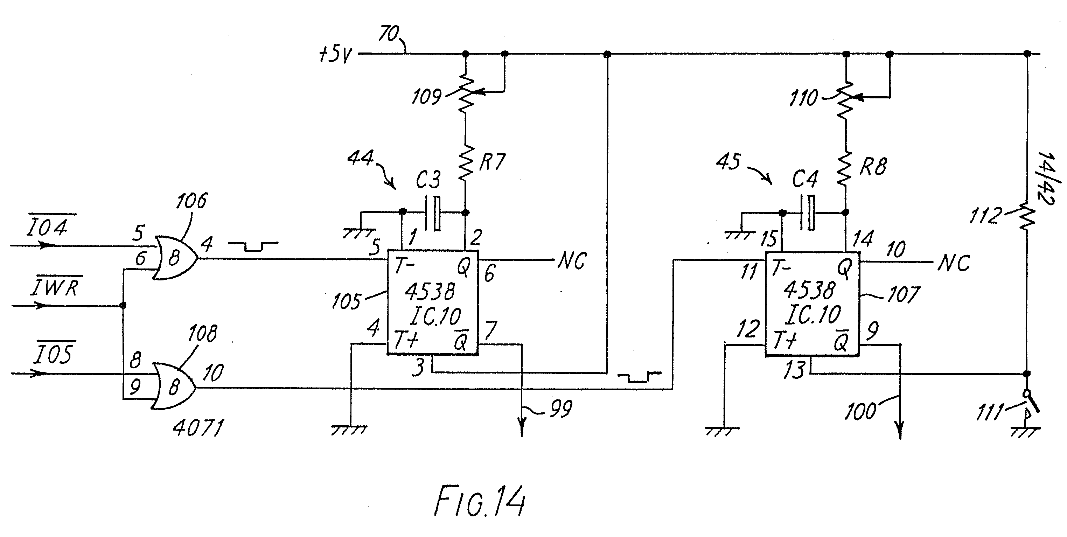

Fig. 14 shows the scan dwell and selection delay circuits 44 and 45. Each of the circuits 44 and 45 includes a type 4538 monostable circuit which is part of the integrated circuit I.C.10. The scan dwell monostable circuit 105 is set by the output of an OR gate 105 whenever both $\overline{\text{IO4}}$ and $\overline{\text{IWR}}$ go low. The selection delay monostable circuit 107 is set by the output of an OR gate 108 whenever both $\overline{\text{IO5}}$ and $\overline{\text{IWR}}$ go low. The complement outputs $\overline{\text{Q}}$ of the monostable circuits 105 and 107 are supplied to the lines 99 and 100 respectively. The scan dwell set time can be adjusted by manual adjustment of a variable resistor 109 connected to the timing components R7 and C3 of the scan dwell circuit 44. Similarly the selection delay set time can be adjusted by manual adjustment of a variable resistor 110 connected to the timing components R8 and C4 of the selection delay circuit 45. The selection delay can be eliminated by manually closing a switch 111 which grounds the resetting input pin 13 of the selection delay monostable circuit 107 which is normally held at the high level by a resistor 112 coupling the pin 13 to the +5 volts line 70.

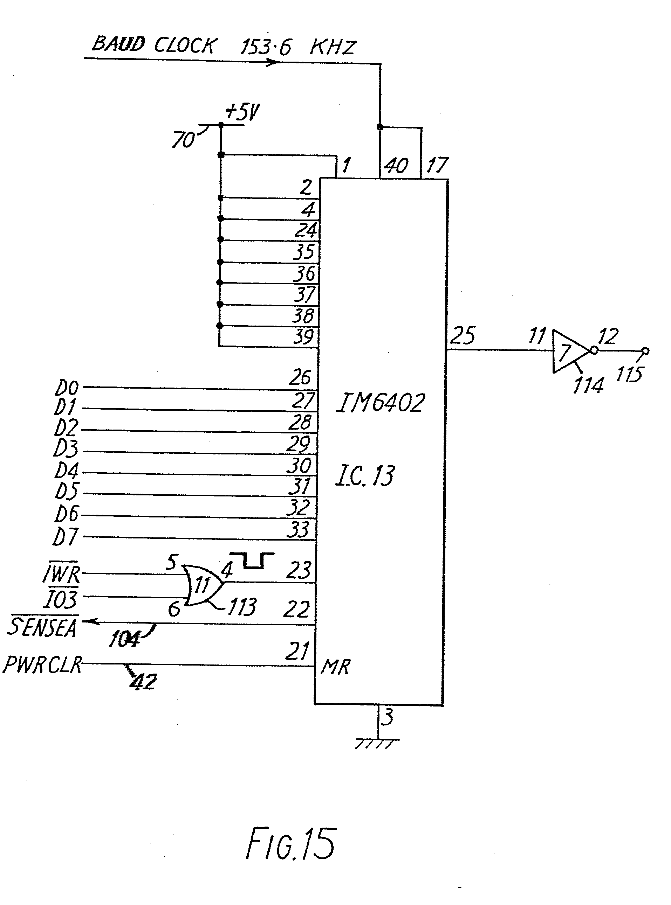

Fig. 15 shows the transmitting UART which is an integrated circuit I.C.13 of the type IM6402 and receives baud clock pulses from the clock pulse generator circuit 31 at 153.6 kilohertz. All eight data bus lined DO to D7 of the data bus 36 are connected to respective parallel data input pins of the UART I.C.13. A resetting signal, PWRCLR at 42, is supplied by the RAM save and select circuit 38 to a master reset (MR) pin 21 of I.C.13. The UART status signal SENSE A is output at a pin 22 to line 104. An enabling signal is supplied to pin 23 of I.C.13 whenever the two signals IWR and I03 are both low, this condition causing the output from an OR gate 113 supplying pin 23 to be low. The UART I.C.13 is adapted to operate only to transmit serial data from its pin 25 through an inverter 114 to an output terminal 115, various pins concerned with conversion of serial data to parallel data for receiving being connected to the high signal level line 70 as shown in Fig. 15. The output terminal 115 is connected through the junction box 23 of Fig. 1 to the indicator input terminal 54 of Fig. 4.

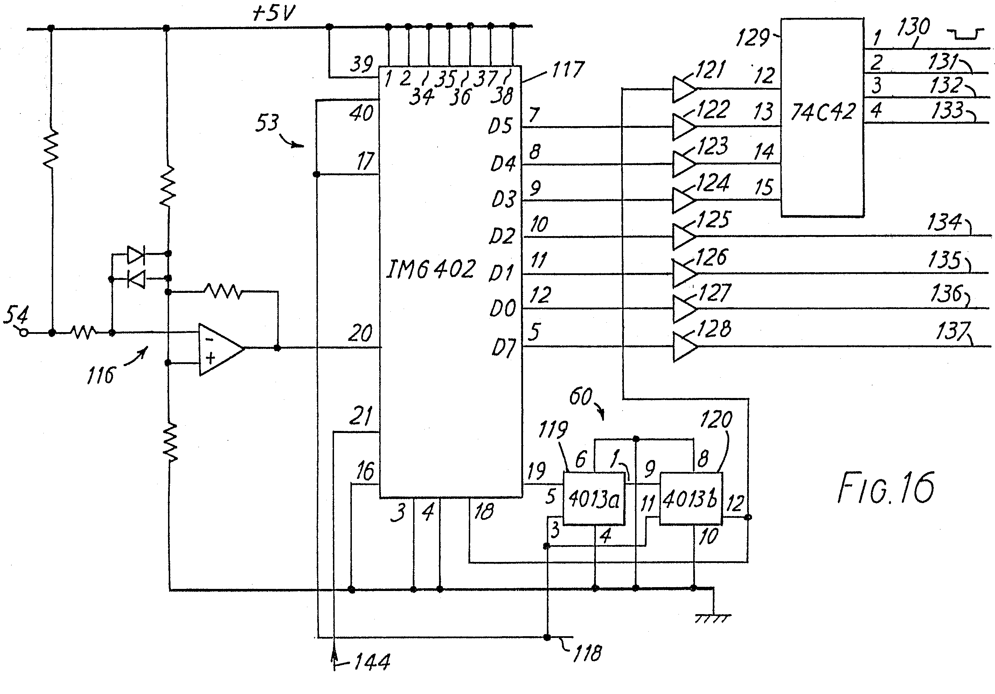

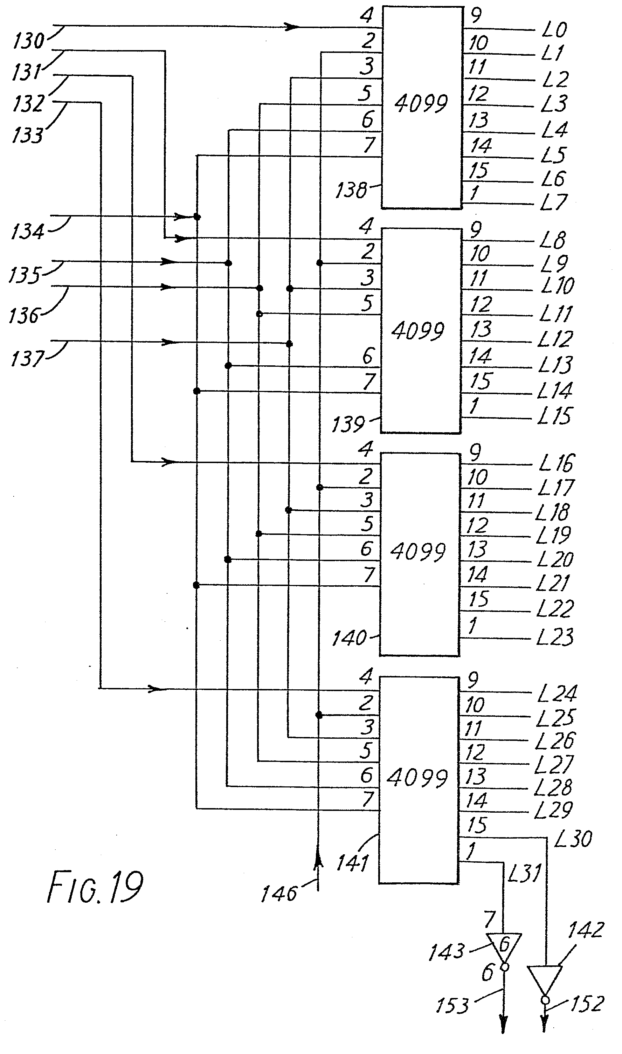

Fig. 16 shows part of the indicator circuit of Fig. 4 in more detail, including the receiving UART 53, the delay circuit 60 and part of the decoder 58. The UART 53 includes input amplifier and shaping circuitry 116 connected to shape and amplify the serial data signals supplied to the terminal 54, an a UART integrated circuit 117, again of the type IM6402, which is adapted to operate only in the serial to parallel data receiving mode. Baud rate pulses at 153.6 kilohertz are supplied to pins 17 and 40 of the UART i.c. 117 from a line 118 which also supplies these pulses as clocking pulses to two bistable circuits 119 and 120 constituting the delay circuit 60. The two bistable circuits 119 and 120 are halves of an integrated circuit type 4013 and are D-type bistables, the valid output signal pin 19 of the UART i.c. 117 being connected to the D-input of the first bistable circuit 119, which drives the second bistable circuit 120 at its D-input as shown, and the complementary output of the second bistable circuit 120 being supplied to the output reset pin 18 of the UART i.c. 117, and to a first buffer 121. Parallel data output pins 8, 9, 10, 11, 12, and 5 of the UART i.c. 117 are connected respectively to seven further buffers 122 to 128. The first four buffers 121 to 124 supply their outputs to the input pins 12, 13, 14, and 15 of a 4-bit to 10 way decoder 129, a type 74C42 integrated circuit, of which only the first four outputs, at its pins 1, 2, 3 and 4, are used, these outputs being applied to lines 130, 131, 132 and 133. The outputs of the other four buffers 125 to 128 are supplied respectively to four lines 134, 135, 136 and 137. Fig. 19 shows how the lines 130 to 137 are connected to four latch-decoder integrated circuits 138 to 141, each of the type 4099, which form the remainder of the decoder 58, and shows the lines L30 and L31 connected to respective inverters 142 and 143.

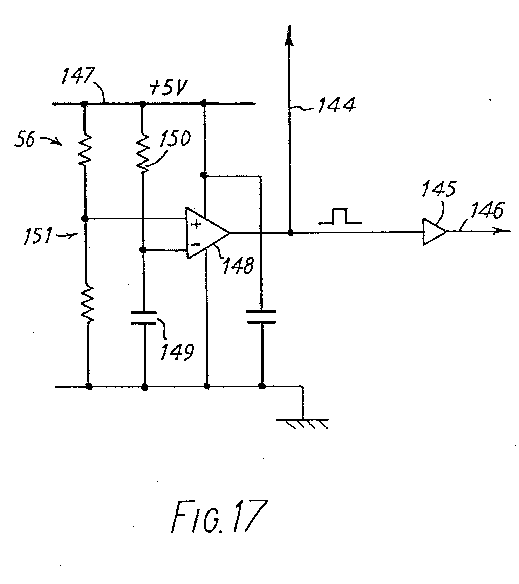

Fig. 17 shows the reset circuit 56 of Fig. 4 in more detail. This circuit 56 supplies positive-going resetting pulses over a line 144 to the master reset pin 21 of the UART i.c. 117 of Fig. 16 whenever the system is switched on. These pulses are also coupled through a buffer 145 in the circuit 56 and are applied by a line 146 to the four latch-decoder i.c.s 138 to 141 of Fig. 19 as resetting pulses. The resetting pulses are generated in the circuit 56 when an indicator supply line 147 rises from ground to +5 volts at switching on of the system, the inverting input terminal of a differential amplifier 148 being initially held low by a capacitor 149 and rising to +5 volts at a rate determined by a series resistor 150 to the capacitor 149 from the line 147 whereas the non-inverting input terminal of the amplifier 148 rises substantially immediately to +2-5 volts through a voltage divider 151 coupling the ground and +5 volt lines.

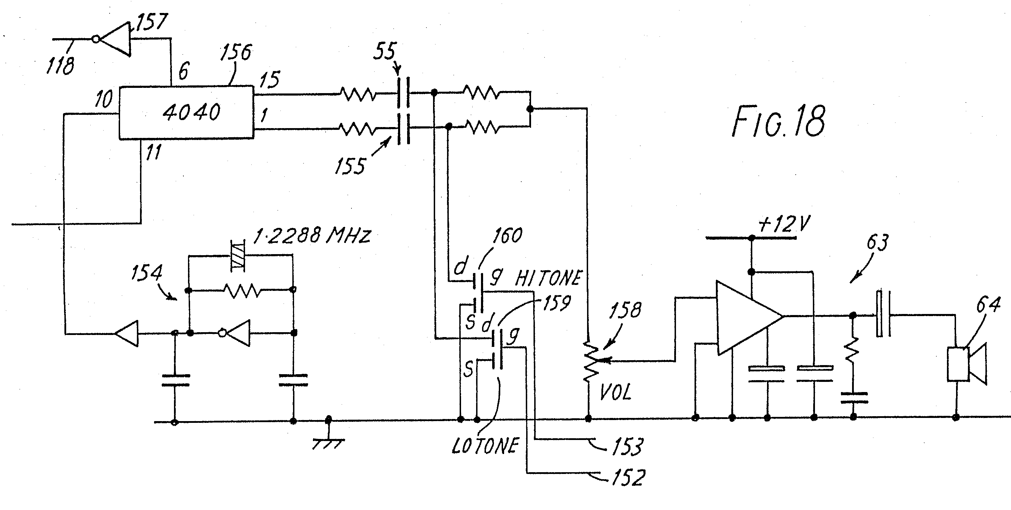

Fig. 18 shows in detail the circuits of the pulse generator 55 and the audio amplifier 63. The pulse generator 55 induces a 1.2288 megahertz crystal-controlled oscillator 154 and a switchable output filter 155 controlled by the inverted outputs from the lines L30 and L31 of Fig. 19 supplied over lines 152 and 153 respectively. The oscillator 154 drives a counter-scaler 156, which is an integrated circuit of the type 4040 and supplies the 153.6 kilohertz baud rate clock pulses through an inverter 157 to the line 118, and two audio frequency square waves from output pins 1 and 15 to the output filter 155 which consists of two series combinations each of a capacitor with two resistors and coupling the pins 1 and 15 respectively to a volume control potential divider 158 at the input end of the amplifier 63. The lines 152 and 153 are connected to the gate terminals of respetive switching field effect transistors 159 and 160. When the transistor 159 conducts, the output from pin 15 of the counter-scaler 156 fails to reach the divider 158, and when the transistor 160 conducts, the output from pin 1 fails to reach the divider 158. The audio frequency at pin 1 is appreciably lower than that at pin 15. Consequently if switching transistor 159 is on and switching transistor 160 is off, the amplifier 63 drives the loudspeaker 64 at a low tone, and if switching transistor 159 is off and switching transistor 160 is on, the loudspeaker 64 is driven at a high tone. Hence it will be seen that an active signal at line L30 in Fig. 19 results in the low tone, and an active at line L31 in Fig. 19 results in a high tone. The circuitry of the amplifier 63 is conventional, an integrated circuit type LM380 being used as the amplifying element.

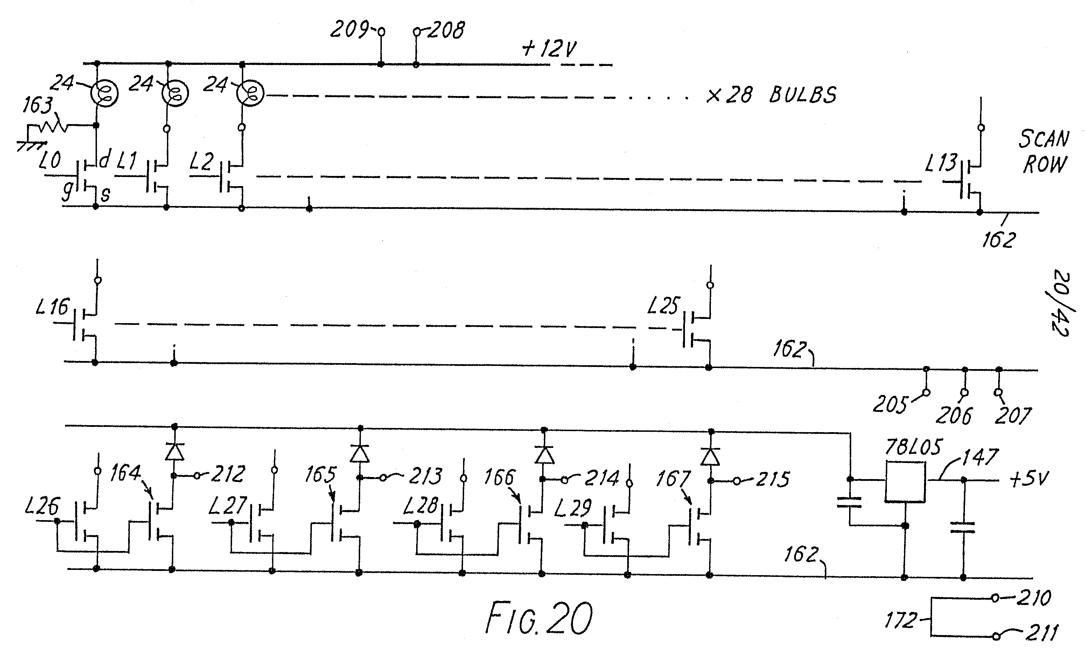

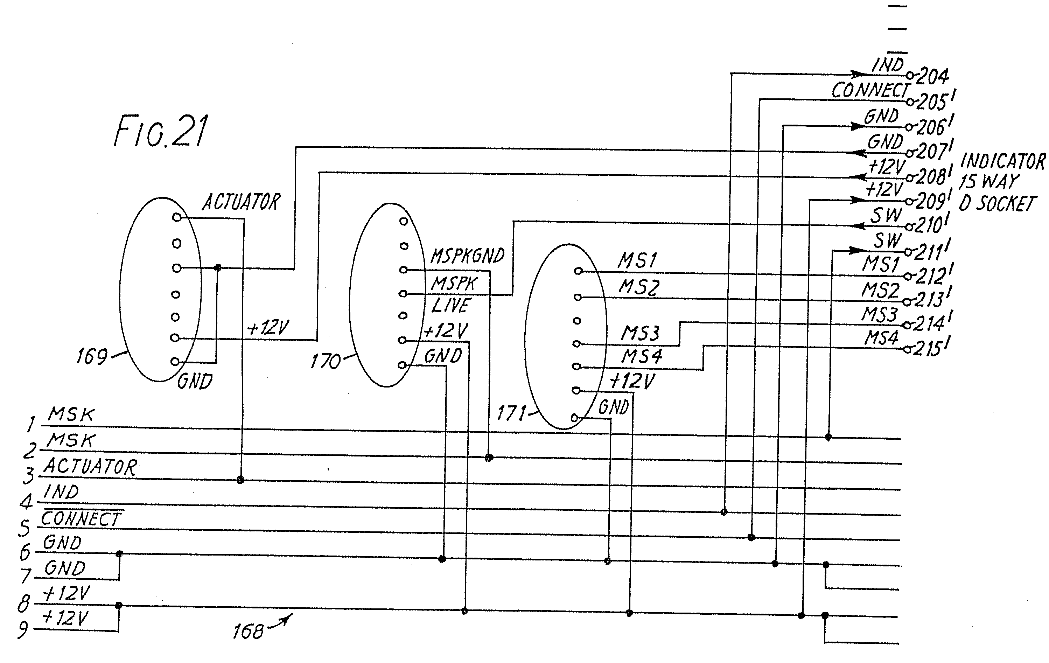

Fig. 20 shows the lamp drivers circuit 62 which includes a d.c. regulator circuit 161 which provides +5 volts on the line 147 when +12 volts is supplied to terminal 209 of a fifteen way D socket of the indicator 13. Each lamp driver circuit has a field effect transistor having its source-drain channel connected in series with a lamp filament between the terminal 209 and a ground line 162. Only three of the twenty-eight lamps are shown. The gate terminal of each lamp driver transistor is connected to a respective one of the twenty eight output lines L0 to L13 and L16 to L29 of the decoder 58, the lines L14 and L15 shown in Fig. 19 not being used. Thus the signal levels on the lines L0 to L13 determine the states of the white lamps 24, and the signal levels on the lines L16 to L29 determine the states of the red lamps 25. Since the white lamp 24 of the start square, which is controlled by line L0, is subjected to the most switching while the system is operating, a resistor 163 is connected in parallel with its switching FET so that a small current continues to flow through the lamp filament when the lamp is off, thereby reducing the temperature fluctuations undergone by the lamp filament during operation. The current through the resistor 163 is of course too low to make the lamp appear to be on which it should be off. The lines L26, L27, L28 and L29 are also connected to the gate terminals of respective switching field effect transistors in four relay driver circuits 164, 165, 166 and 167 with respective output terminals connected to terminals 212,213, 214 and 215 of the indicator D socket. Relay driver 164 controls a first mains switching relay in the mains switching unit 18, and the relay drives 163, 164 and 167 control second, third, and fourth mains switching relays in the unit 18, the respective connections to these relays through the junction box 23 being indicated by MS1, MS2, MS3 and MS4 in Fig. 21.

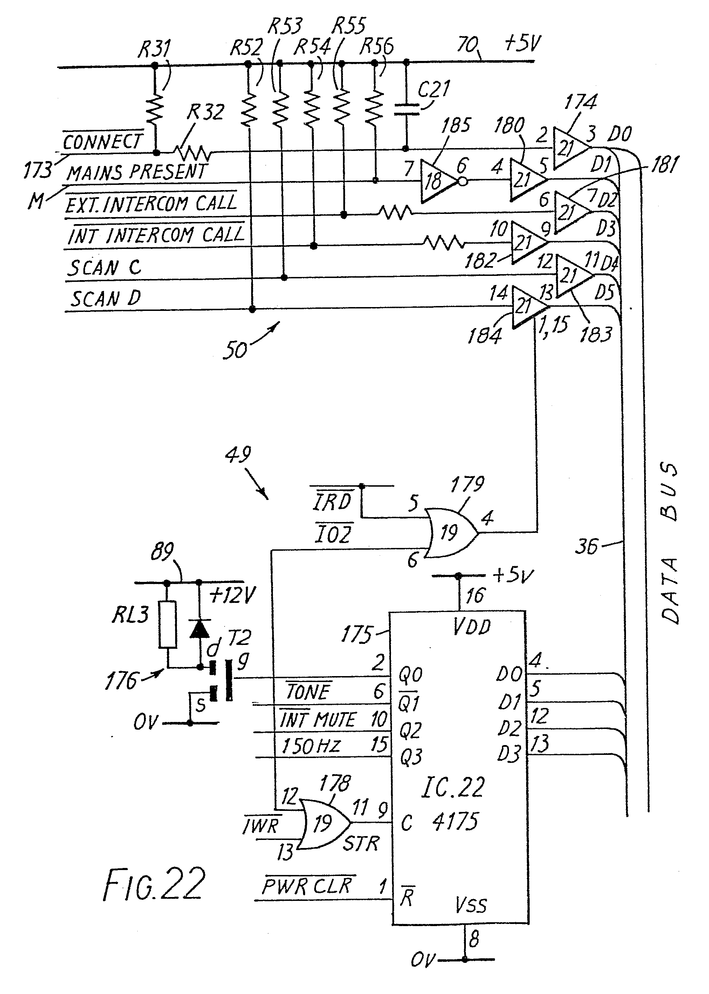

Fig. 21 shows the wiring of the junction box 23 which has a 15-way D socket corrsponding to the 15-way D socket (not shown) at the indicator, the terminals of this socket of the junction box being connected to the corresponding terminals of the socket at the indicator 13 by respective lines of a cable. A seven line junction bus 168 connects nine input and nine output terminals of the junction box 23 as shown, the nine input terminals being connected by respective lines in a nine line cable to the control unit 11 as indicated in Fig. 1. The junction box 23 includes an actuator socket 169 to which the input device 12 is connected, a master speaker socket 170 to which the master speaker 22 is connected, and a mains switching unit socket 171 to which the mains switching unit 18 is conneted. Ground and +12 volt connections are provided directly to the sockets 170 and 171 from the bus 168, and via the lamp drivers circuit 62 of Fig. 20 to the actuator socket 169, so that the input device 12 is disabled if the indicator 13 is not connected to the junction box 23. The input signal return line from the input device 12 is connected directly through the socket 169 to a respective line of the bus 168. One of the audio signal lines of the bus 168 is connected through the lamp drivers circuit 62 of Fig. 20, by a link 172, the other audio signal line being connected directly to the socket 170. Each of the four mains switching unit lines from the socket 171 is connected through the junction box indicator socket to the circuit of Fig. 20 by connections MS1, MS2, MS3 and MS4 to terminals 212', 213', 214' and 215' of the junction box 15-way D socket which are connected respectively to terminals 212, 213, 214 and 215 of the corresponding socket at the indicator, these terminals being represented in Fig. 20. The pairs of corresponding terminals of the two 15-way sockets, one at the indicator 13 and the other at the junction box 23, are connected together by insulated lines in the cable between the two sockets. Thus the +12 volt supply from the control unit 11 is applied through the junction box 23 via terminal 209' to the indicator terminal 209 and thence via the terminals 208 and 208' to the actuator socket 169. The serial data output from the transmitting UART output terminal 115 (Fig. 15) is applied through the junction box 23 to its terminal 204 and thence to the serial data input terminal 54 of the receiving UART 53 (Figs. 4 and 16). The audio link 172 of the circuit of Fig. 20 connects the terminals 210 and 211 at the indicator 13 which are respectively connected to the terminals 210' and 211' at the junction box 23. The system ground line is connected through the junction box 23 to its terminal 206' and thence via terminals 206, 207 and 205 of the indicator (Fig. 20) to its terminal 205' and 207', the terminal 207' being connected to the ground terminals of the actuator socket 169. The terminal 205' is connected to a return line, designated $\overline{CONNECT}$, of the bus 168 of the junction box 23. Consequently if the control unit 11 is properly connected to the junction box 23 or to the indicator 13, it senses a ground, i.e. low, signal level on the $\overline{CONNECT}$ line at a terminal 173 shown in Fig. 22. This terminal 173 is also coupled through a resistor R31 to the +5 volts line 70 of the control unit 11 and consequently if the junction box 23 is not connected to the control unit 11, or if the indicator 13 is not connected to the junction box 23, the control unit 11 senses a high signal level at the terminal 173. The terminal 173 can be coupled to the D0 data line of the data bus 36 through a D0 tri-state buffer 174 of port 2 which is included in the system and intercom sensor circuit 50 of Figs. 3 and 22.

From Figs. 2, 18, 19 and 20 it will be seen that the data transmitted to the indicator 13 controls thirty separate items, these being the fourteen white-lamp driver circuits, ten red-lamps driver circuits, four parallel combinations of a red-lamp driver circuit and a relay-driver circuit, and two audio signal filter switches (transistors 159 and 160 in Fig. 18). A six bit binary code is therefore used to identify the separate items, these being, at the control unit 11, on data lines D0 to D5. A seventh bit on the data line D7 is used to determine whether the item is to be on or off, i.e. active or not. The receiver UART i.c. 117 (Fig. 16) of the indicator 13 decodes the incoming serial characters to provide the same six item identifying (address) bits on its pins 7 to 12 in such a way that the data bits of lines D0 to D5 are arranged on pins 12, 11, 10, 9, 8 and 7 as indicated by D0 to D7 in Fig. 16. The "on/off" bit of data line D7 is supplied at pin 5, as indicated by D7 in Fig. 16. The delayed "valid data" output from the delay bistable 120 of the delay circuit 60 is used as an enable signal for the 4-bit to 10-way decoder 129, and the three most significant bits D3, D4 and D5 of the indicator item address are decoded to appropriate ones of the lines 130, 131, 132 and 133. Thus the address bits D3, D4 and D5 determine which one of the decoders 138 to 141 of Fig. 19 is selected. The three least significant bits D0, D1 and D2 of the address are supplied to each of the four decoders 138 to 141 and are decoded by the selected one of the four into one of eight sub-addresses corresponding to one of the eight lines L0 to L7 or L8 to L15 or L16 to L23, or L24 to L31, although the sub-addresses for L14 and L15 never occur. The "on/off" bit D7 is applied by the line 137 to all four decoders 138 to 141 at their respective input pins 3 so that the signal level at the addressed one of the lines L0 to L31 is the same as the signal level for the D7 bit. Thus the complete decoder circuit 58 of Figs. 16 and 19 provides sixty different outputs, there being two for each separate indicator item, i.e "on" and "off" levels.

Since no data is transmitted back by the indicator 13 to the control unit 11, other than the $\overline{\text{CONNECT}}$ signal, areas of the RAM memory of the control unit 11 are allocated as images of the states of the twenty-eight lamps and two audio switches. In these RAM images, the state of each item is stored by storing the most significant bit of a byte corresponding to the item, i.e. the D7 data bit is stored to indicate "on" or "off". Other bits of the stored byte corresponding to any particular item may be used as flags for various purposes.

Fig. 22 shows the circuits of the intercom control circuit 49 and the system and intercom sensor circuit 50 in detail, these two circuits 49 and 50 being output and input sections of port 2. The intercom control circuit 49 includes an integrated circuit latch, IC22, which is a type 4175 and is connected to the D0 to D3 lines of the data bus 36. Of the data supplied, D0, D2 and D3 are latched to provide Q0, Q2 and Q3 outputs, and D1 is latched to provide a $\overline{Q1}$ output. The Q0 output controls a switching FET T2 in a relay-driver circuit 176 which controls the winding of a relay RL3. The contacts of the relay RL3 are a single normally open (NO) set in the intercom circuit, which is shown in Fig. 24, and control the connection of the internal intercom 17 to the circuit of Fig. 24, which is included within the control unit 11. The $\overline{Q1}$ output serves as a $\overline{TONE}$ signal which in the circuit of Fig. 24 controls a switching FET T29. The Q2 output serves as an $\overline{INT\ MUTE}$ signal in Fig. 24 where it controls a switching FET T28. The D3 data line of the data bus 36 is switched at every interrupt and consequently its signal level changes 300 times a second. It is arranged that the Q3 output of the latch circuit 175 follows this changing signal level so that a 150 Hz square wave is generated at the pin 15 of the latch 175. In the intercom circuit of Fig. 24, the 150 Hz square wave is resistance-capacitance coupled to an integrated circuit amplifier 177, type LM380, as shown provided that the FET 29 is not conducting, since this FET 29, when conducting, grounds the input pin 6 of the amplifier 177 to which the 150 Hz signal is coupled. Thus the $\overline{TONE}$ signal controls the coupling of the 150 Hz signal to the amplifier 177. The $\overline{INT\ MUTE}$ signal similarly controls the coupling of speech signals to the amplifier 177, the FET 28 grounding the input pin 2 of the amplifier 177 when conducting. The circuit 49 includes an OR gate 178 to which the $\overline{IWR}$ and $\overline{IO2}$ signals are supplied to produce a strobe signal STR to be supplied to the clock (C) input pin, pin 9, of the integrated circuit latch 175. The $\overline{PWRCLR}$ signal from the RAM save and select circuit 38 (Fig. 8) is applied as a resetting signal to the reset (R) input pin, pin 1, of the latch 175.

The system and intercom sensor circuit 50 includes an OR gate 179, which is part of an integrated circuit IC19 which includes the OR gate 178, and the signals $\overline{IO2}$ and $\overline{IRD}$ are supplied to the OR gate 179 to produce an enabling signal for six tri-state buffers 174 and 180 to 184 which form the input section of port 2. The signal indicating whether or not the a.c. mains supply is present, which is produced on the line M in the power supply circuitry of Fig. 10 is coupled by an inverter 185 to the buffer 180 and can thus be read on data line D1. The "press-to-speak" push buttons 20 and 21 control respective grounding switches (not shown) connected respectively in series with resistors to the buffers 181 and 182 so that $\overline{\text{EXT. INTERCOM CALL}}$ and $\overline{\text{INT. INTERCOM CALL}}$ signals can be read at data lines D2 and D3. The remaining two buffers 183 and 184 are not used in the present example but could, with a suitable program in the EPROM 33, be used to sense respective signals, generated by closure of respective grounding switches, requesting two further different modes of input control for the scanning and selection of the indicator squares by the user, these signals being designated SCAN C and SCAN D, and being read by data lines D4 and D5. The input pins of the buffers 174 and 180 to 184 are also respectively coupled, directly or indirectly, through resistors R31, R56, R55, R54, R53 and R52 to the +5 volts line 70. The line 173, on which the signal level indicates whether the control unit 11 is connected to the indicator 13, is connected to a circuit node common to the two resistors R31 and R32 which are in parallel with a smoothing capacitor C21 which smooths the voltage changes applied to the input pin 2 of the buffer 174. If the control unit 11 is not connected to the indicator 11, the capacitor C21 discharges and +5 volts is applied to the buffer 174. The high signal level of +5 volts is also applied to the buffers 181 and 182 when the respective push-button controlled switches at the intercoms 16 and 17 are open. However, since the line M of the power supply circuit of Fig. 10 is connected to the inverter 185, if there is mains failure the voltage at the input pin, pin 7, of the inverter 185 falls to about +2.5 volts, whereas when the mains supply is present, the voltage on line M is about +4.7 volts, this being the zener voltage of the zener diode Z100. The inverter 185 produces a low signal level in response to the +4.7 volts input, and a high signal level in response to the +2.5 volts input.

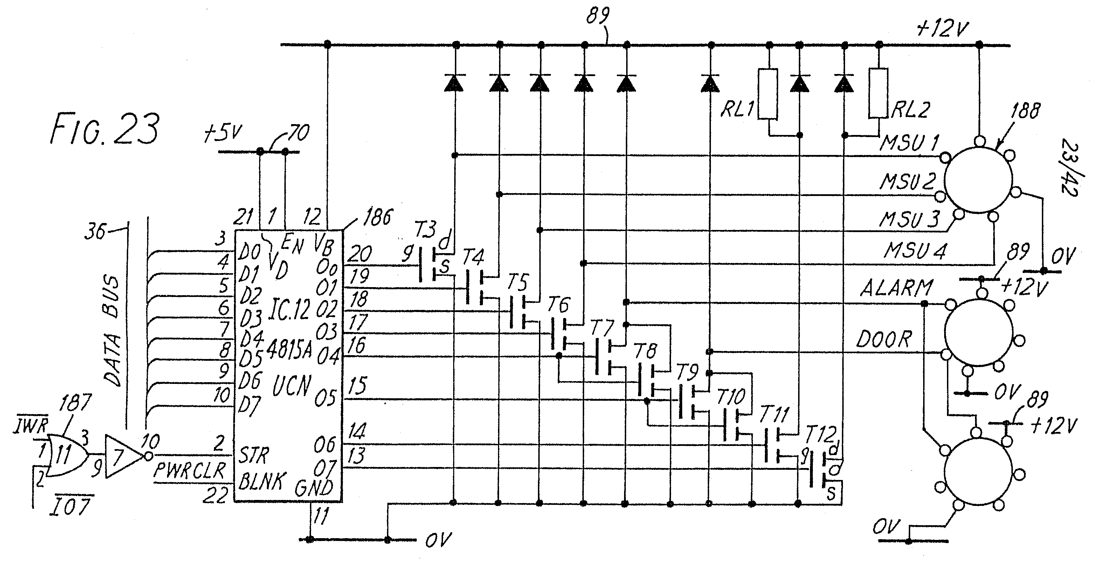

Fig. 23 shows the alarm control circuit 48 in detail. This circuit 48 includes port 7 which is an output port that controls ten switching FETs T3 to T12. All eight lines D0 to D7 of the data bus 36 are connected to port 7 which is provided in the form of a latching circuit 186 which in this example is a type 4815A integrated circuit I.C.12. The port 7 enabling signal $\overline{IO7}$ and the interface write signal $\overline{IWR}$ are supplied to an OR gate 187 from which the output is inverted by an inverter 188 and controls, through coupling by the inverter 188, strobe input pin STR, pin 2, of the latching circuit 186. The PWRCLR signal from the RAM save and select circuit 38 is supplied to a blanking pin BLNK, pin 22, of circuit 186. The outputs from the circuit 186 corresponding to D0 to D7 are designated 00 to 07 and are supplied to the respective gate terminals of the FETs T3 to T12. The source-drain channels of the FETs are protected from relay back e.m.f. by respective diodes coupling the drain terminals to the +12 volt supply line 89. The FETs T3 to T6 respectively provide, when conducting, ground connections for the relay windings of the relays of the four relay-controlled mains switches (not shown) of the mains switching unit 18 if the unit 18 is not connected to the junction box 23 but is connected to a mains switching unit control socket 188 provided on the control unit 11. The FETs T7 and T8 when conducting provide ground connections for the relay windings of respetive control relays (not shown) in the alarm bell 15 and the door catch release device 14. Two sockets 189 and 190 are provided for connecting the FETs T7 and T8 to the alarm bell 15 and the door catch release device 14 respectively so that these connections can be made through a simple multi-core cable or two such cables. The +12 volt line 89 and the system ground line are also connected to respective terminals of the three sockets 188, 189, 190 so that +12 volts can be supplied to the control relay windings (not shown) and a common ground provided for all equipment in the system. The FETs T11 and T12 when conducting provide ground connections for the windings of two relays RL1 and RL2 which control contacts in the intercom circuit shown in Fig. 24. The contacts controlled by the relay RL1 are a pair of commutating sets with respective movable contacts 191 and 192 and respective pairs of fixed contacts 193, 194 and 195,196. The contacts of 191 and 193 and the contacts 192 and 195 are normally closed (NC). In the normal, i.e. unenergised, condition of the relay RL1, an input terminal 197 for the master speaker live line is connected to a signal level setting input resistor R33 of an audio automatic gain control stage 198 having an output terminal 199 coupled by a series combination 200 of a capacitor and a resistor to the input pin 2 of the amplifier 177. The automatic gain control stage 198 and the amplifier 177 with an output coupling capacitor-resistor combination 201 provide audio frequency amplification between the movable contacts 191 and 192 of relay RL1. The relay RL2, controlled by the FET T12, has a single set of normally open contacts connected between the contact 195 of relay RL1 and a terminal 202 for the internal intercom live line. The contacts of the relay RL3 (Fig. 22) control connection of the contact 195 to a terminal 203 for the external intercom live line. Respective ground terminals for the ground lines to the three intercom speakers are connected together to the system ground line. Thus when the relay RL1 is not energised, the master speaker 22 operates as a microphone for the audio amplification circuit 198, 200, 177 and 201, and either of the two remote intercom units 16 and 17 can act as a loudspeaker. When the relay RL1 is energised, the movable contacts 191 and 192 change over to the fixed contacts 194 and 196 so that either the internal intercom 17 or the external intercom 16 can act as a microphone while the master speaker 22 acts as a loudspeaker. The transmission of speech through the intercom circuit is possible only if the transistor T28 is not conducting. Thus the signal $\overline{\text{INT}}$ MUTE produced by the output section of port 2 in circuit 49 of Fig. 22 controls the passage of speech through the audio amplification circuit 198, 200, 177 and 201. The 150 Hz signal is supplied to whichever of the three units - master speaker 22, internal intercom 17, or external intercom 16 - is serving as a loudspeaker when the transistor T29 is not conducting. The transistors T28 and T29 are operated in a complementary manner such that at least one of them is conducting at any time. The 150 Hz tone is used as an audible signal to announce at the master speaker 22 that a "press-to-speak" push button 20 or 21 has been operated and to call or respond at the remote units 16 and 17.

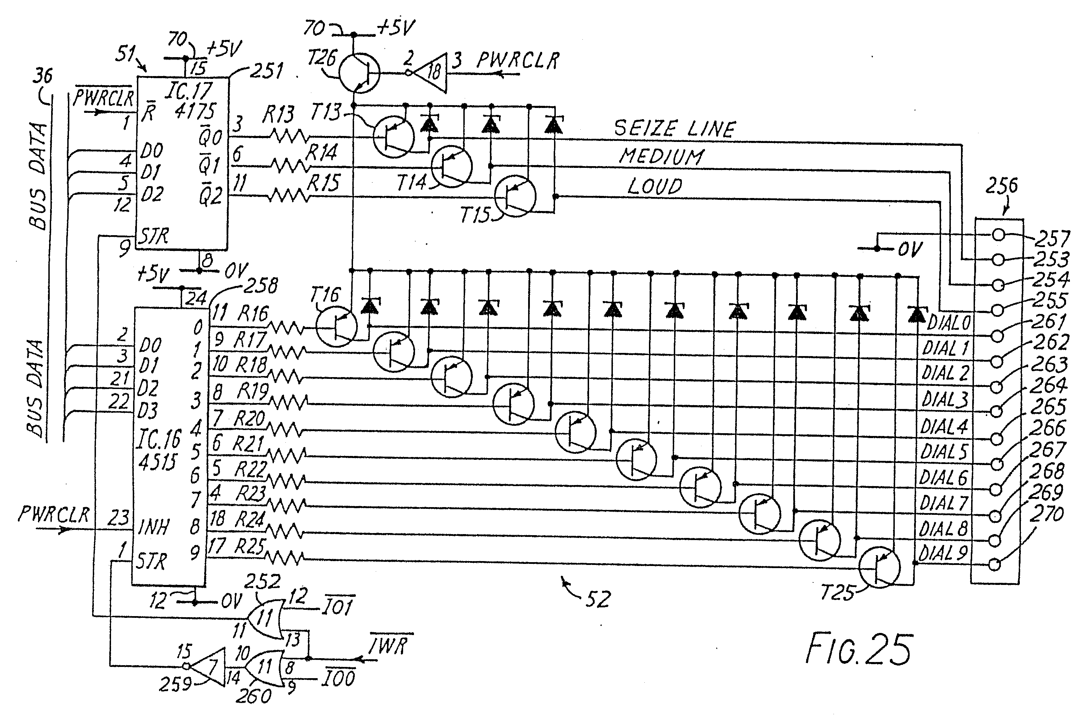

Fig. 25 shows the line seizing and volume control circuit 51 and the dialling circuit 52 of Fig. 3 in more detail. The line seizing and volume control circuit 51 includes port 1 in the form of an integrated circuit latching circuit 251 which in this example is a type 4175 integrated circuit (I.C.17). The $\overline{\text{PWRCLR}}$ signal is supplied as a resetting signal to pin 1 of I.C.17, and a strobe signal is applied to its pin 9 by an OR gate 252 in response to the signals $\overline{\text{IO1}}$ and $\overline{\text{IWR}}$ both being active. The data lines D0, D1 and D2 of the data bus 36 are connected respectively to input pins 4, 5 and 12 of I.C.17 and data on these lines is used to latch the output levels

of the $\overline{Q0}$, $\overline{Q1}$ and $\overline{Q2}$ of I.C.17 which are applied through resistors R13, R14 and R15 to the base terminals of switching transistors T13, T14 and T15. The transistors T13, T14 and T15 are p-n-p transistors and have their emitters coupled by an n-p-n transistor T26 to the +5 volt line 70. The transistor T26 is momentarily held non-conducting, at switching on of the system, by the signal PWRCLR. The transistor T26 is a type 2N4401 and the transistors T13, T14 and T15 are type BC477 in this example. Respective 68 volt zener diodes protect the transistors T13, T14 and T15 which control the signal levels at a seize line command terminal 253, a medium command terminal 254, and a loud command terminal 255 of a telephone command output socket 256 of the control unit 11 which is connected by thirteen lines (not shown) to the LST 7A telephone 19. The system ground line is connected to a first socket terminal 257. The transistors T13, T14 and T15, and thus the data on lines D0, D1 and D2 latched into port 1, control the seizing of a telephone line, production of incoming speech at medium volume by the telephone 19, and production of incoming speech at a loud volume by the telephone 19.

The dialling circuit 52 includes port 0 in the form of an integrated circuit 4-bit to 10-way decoder 258 which in this example is a type 4515 integrated circuit (I.C.16). The PWRCLR signal is supplied as an inhibiting signal to pin 23 of I.C.16, and a strobe signal is applied to its pin 1 by an inverter 259 driven by the output of an OR gate 260 in response to the signals $\overline{IO0}$ and $\overline{IWR}$ both being active. The four data lines D0 to D3 are connected respectively to input pins 2,3,21 and 22 of I.C.16, and data on these four lines strobed in by the OR gate 260 is decoded into one of ten outputs which appears as an active signal on the appropriate one of the ten output pins 4 to 11, 17 and 18 which are coupled through respective resistors R16 to R25 to bases of ten p-n-p transistors T16 to T25.  These ten transistors are supplied and protected
in the same manner as the three transistors T13, T14 and
T15, and each has its collector terminal connected to a
respective one of ten dialling digit terminals 261 to 270
of the socket 256.  Active signals at these terminals 261
to 270 respectively serve as commands to transmit the
telephone signals corresponding to dialling 0 to dialling
9 from the telephone 19.  Thus the parallel data latched
into port 0 by OR gate 260 from the four data lines D0 to
D3 acts to simulate dialling at the telephone 19.

From the preceding description and Fig. 3 it will be
seen that the CPU 30 can receive data from only ports 2
and 6, which are in the system and intercom sensor circuit
50 and the input, scan params and busy sensor circuit 47.
The input sections of port 2 can be read to provide data
representative of the connection or otherwise of the
indicator 13 to the control unit 11, the presence or
absence of a.c. mains power, and the respective states of
the intercom "press-to-speak" push buttons 20 and 21.
Thus the status of bits 0 to 3 of the input section of
port 2 provides information on the general operation of
the system and the use of the intercom system by those
other than the principal user.  The status of bits 0 to 5
read from port 6 provides information on the respective
conditions of the scan dwell monostable 44 and the
selection delay monostable 45, the state of the user input
switch device 12, the state of the scan mode switch 103,
and the condition of the transmitting UART of the output
UART circuit 46.  The status of the input section of port
2 and the status of port 6 are read as required for the
operation of the system and are stored in allocated
regions of the RAM 34.  The output ports, which are port
0, port 1, the output section of port 2, and ports 3,4,5,7
and 8 receive commands from the CPU 30 over the data lens
36.  To keep an account of the current, and in some cases
the previous, state of certain of these output ports, regions of the RAM are allocated as stores for status information relating to such ports. No status information is stored for ports 0, 3, 4, 5 and 8. Activation of port 0 results in the dialling out of telephone digits, and these digits are stored in other allocated regions of the RAM. Only the actual states of the scan dwell monostable 44 and the selection delay monostable 45 (ports 4 and 5) are required and this information is obtained by the CPU 30 through port 6 in the input, scan params and busy sensor circuit 47. The state of the UART (port 3) of the output UART circuit 46 is also read through port 6. One region of the RAM, labelled P1STAT in the EPROM program, is allocated for storing the current output status of port 1 in the seize line and volume control circuit 51, at which bits 0 and 2, i.e. D0 and D2, respectively determine whether or not a telephone line is seized and whether or not incoming speech is reproduced at high volume. Two regions of the RAM, labelled STAT2 and P2STAT, are allocated for storing respectively the intended return states of the external intercom connection relay RL3, the 150 Hz tone switch T29, and the intercom speech switch T28, these being controlled by bits 0, 1 and 2 (i.e. D0, D1 and D2) at the output section of port 2, and the current output status of port 2 output section. Similarly two regions, STAT7 and P7STAT store respectively the intended return states and current output states of bits 0 to 7 (D0 to D7) for port 7. Bits 0 to 3 of port 7 control the mains switching unit 18 if it is connected directly to the control unit 11 instead of to the indicator 13 through the junction box 23. Bits 4, 5, 6 and 7 of port 7 control the alarm bell 15, the door catch release device 14, relay RL1, and relay RL2. The regions STAT2 and STAT7 are needed to allow the states of the output section of port 2, and port 7 to be returned to their intended states after execution of a routine for effecting audible indication of a mains failure through the intercom system.

The RAM images of the indicator 13 consist of fourteen 8-bit bytes containing the indicator addresses and on/off conditions of the white lamps 24, with bits 0 to 5 of each byte giving the indicator address and bit 7 giving the on/off condition, a further fourteen 8-bit bytes containing the indicator addresses and on/off conditions of the red lamps 25 in the same way and separated from the first fourteen bytes by two bytes corresponding to the unused output lines L14 and L15 of the indication decoder 58, a further two bytes containing the addresses and on/off conditions of the low and high tone switches 159 and 160 (Fig. 18) of the indicator 13, and a further thirty bytes corresponding respectively to the first thirty bytes in containing data relating to the twenty-eight indicator lamps 24 to 25 and the two tone switches 159 and 160. Each of the further thirty bytes can be used, if required, to store an intended return state, on or off, of the lamp or switch concerned, and possibly other information which must be preserved while the actual condition of the respective lamp or switch is temporarily altered. The RAM addresses of the further thirty bytes are 20H (where H means hexadecimal number system) from the RAM addresses of the thirty bytes containing the indicator addresses.

Allocated regions of the RAM 34 are also used to implement fourteen timers, labelled TIMER 1 to 9, TIMER A, TIMER B, TIMER C, TIM1 and DELAY1 in the EPROM program.

TIMER1 is loaded with 0.1 seconds and determines the duration of high and low tones at the indicator 13.

TIMER2 is loaded with 0.2 seconds and provides an extra length of time in the dwell of the scan on the ALARM square of the indicator 13 at each energisation of the white lamp 24 of the ALARM square.

TIMER3 is loaded either with 3 seconds or 0.25 seconds and determines the duration of a 150 Hz call or acknowledgement tone at the internal intercom 17 when the user selects the INT.IC square of the indicator 13 either to call a helper or to acknowledge a helper at the internal intercom 17.

TIMER4 is loaded with 10 seconds and determines the length of time that the door catch release device 14 is energised when the DOOR square, but not the ALARM square, of the indicator 13 is selected.

TIMER5 is loaded with 0.1 seconds and determines the duration of a 150 Hz cuing tone produced at the master speaker 22 as a cue to the user to speak immediatley after another person has released the push button 20 or 21 of the external intercom 16 or the internal intercom 17.

TIMER6 is loaded with 0.25 seconds and determines the duration of a 150 Hz tone provided at the master speaker 22 as an audible signal to indicate that the push button 20 or 21 of the external intercom 16 or the internal intercom 17 has begun to be depressed, and as an audible signal at the external or internal intercon 16 or 17 to indicate that the system has responded to depression of the respective push button 20 or 21.

TIMER7 is loaded with 10 seconds and is used to determine whether the 3 second or the 0.25 second 150 Hz tone should be produced at the internal intercom 17 when the user selects the INT.IC square, a count down of TIMER7 being triggered by depression of the push button 21 of the internal intercom 17 and the 3 second tone being produced only if TIMER7 has expired or is not running when the user selects the INT.IC square.

TIMER8 is loaded with 0.02 seconds and determines the length of a delay between the end of a cuing tone at the master speaker 22 and change over of the relay RL1 to allow speech to be transmitted from the master speaker 22 to a remote intercom unit 16 or 17.

TIMER9 is loaded with 0.05 seconds and determines the on and off periods for a white lamp 24 when a blinking routine is executed.

TIMER A is loaded with a value suitable for the timing of telephone dialling signals transmitted by the telephone 19 under the control of port 0.

TIMER B is loaded with a value determining a long interval between successive generations of indicator signals in a mains failure routine.

TIMER C is loaded with a value determining a series of short intervals in which the red lamp 25 of the START square is on or off during the indicator signals in the mains failure routine.

TIM1 is loaded two successive different values which determine the respective pitches of two different tones produced at the internal intercom 17 in a routine indicating that the indicator 13 is not connected to the control unit 11.

DELAY 1 is loaded with a value which determines the minimum period over which the state of the input device switch must remain in one state after a first change of state for a further change of state to be processed in the controlling of the indicator 13.

When any particular one of the timers TIMER1 to TIMER8 is running, it is decremented automatically at every occurence of an interrupt. The status of each of these eight timers is stored in two RAM bytes labelled DESAV1, bits 1 to 8 of the most significant byte showing whether or not each of the TIMERs 1 to 8 is running, so that any timer of the eight which is running can be decremented in response to a test of the corresponding bit of DESAV1.

Input data from the input section of port 2, i.e. the state of the D0, D1, D2 and D3, D4 and D5 lines at circuit 50, is stored in a RAM byte labelled FLAG1.

To obtain data from the input port 6 relating to the transmitter UART (i.e. the signal $\overline{SENSE\ A}$) on data line D5, the scan dwell monostable 44 on data line D0, and the selection delay monostable 45 on data line D1, the port is

read directly. Data on data lines D2 and D3 is read and processed on the assumption that only one of these data lines is receiving signals from a user. In the reading routine, the data bit 3 is tested, and if high, the data bit 2 is left unchanged. If the data bit 3 is found to be low, indicating closure of an input device switch coupled to data line D3, data bit 2 is reset to the low value. Since the D2 level is continually high if no input device switch coupled to data line D2 is being used, bit 2 of the processed input data always gives the currently read state of whichever input device switch is being used, whether it be coupled to the data line D2 or to the data line D3. There is also a debouncing routine, and the debounced input device switch data is stored finally as bit 2 of the most significant byte of two RAM bytes labelled BCSAV2.

Another pair of RAM bytes labelled BCSAV1 is used as a counter for counting transitions from on to off and from off to on of any white lamp 24 being blinked.

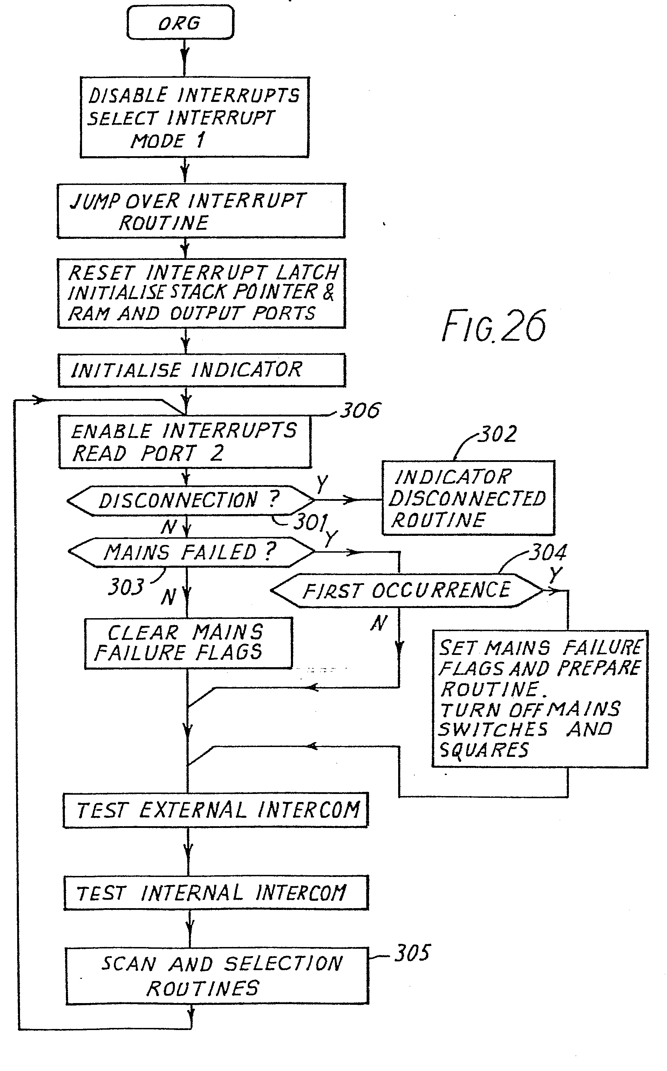

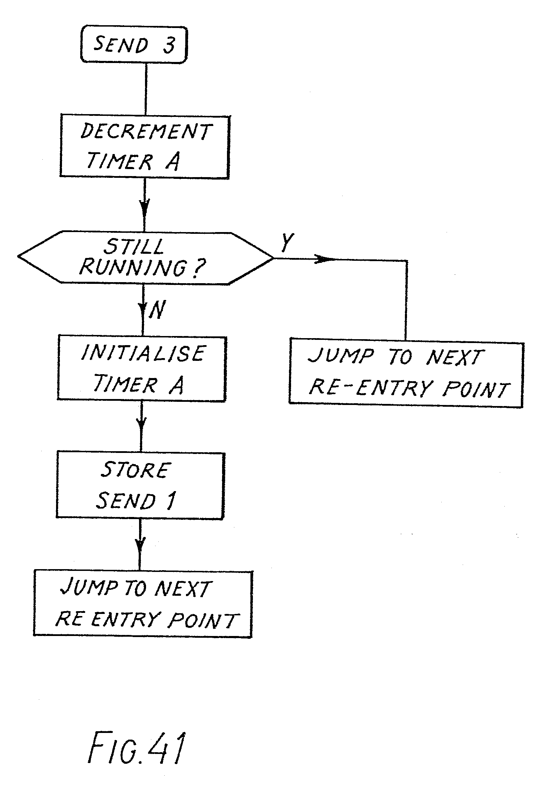

The operation of the system will now be described in more detail with reference to Figs. 26 to 42 which are flow charts illustrating routines determined by the program held in the EPROM 33.

Fig. 26 shows the main stages of the main program starting from the original ORG when the system is turned on. Initially, interrupts are disabled and, the microprocessor being a Z80, interrupt mode 1 is selected which, as explained for example in "Basic Principles and Practice of Microprocessors" by DE Heffer, GA King and DC Keith, published in 1981 by Edward Arnold (Publishers) Ltd of 41 Bedford Square, London WC1B 3DQ, at page 106, is a mode in which the Z80 responds to an interrupt signal $\overline{INT}$ by automatically branching to the memory location 0038H and, having executed the interrupt program thus initiated, returns to the main program at the step immediately following the last before the interrupt signal $\overline{INT}$ occurred. Having selected interrupt routine, resets the interrupt latch by resetting the bistable circuit 66 (Fig. 6) of the clock pulse generator 31 and initialises the stack pointer, various allocated regions of the RAM 34, and the output ports 7, 3, 1, 0 and 2. The initialisation of the regions allocated as indicator images loads the indicator addresses of the lamps 24 and 25 and the switches 159 and 160 into the appropriate RAM locations, and resets the status bits (bit 7) of all except those of the actual and intended state bytes for the START square white lamp 24, which are set in preparation for the indicator 13 to begin with the white lamp 24 of the START square illuminated and all other lamps off. The data in the actual state bytes for the indicator 13 is then transmitted by the output UART circuit 46 to the indicator 13. When the transmission to the indicator 13 is completed, using the OUTD block data transfer instruction of the Z80, interrupts are enabled and the input section of port 2 is read and the resultant input data stored in FLAGS1. Bit 0 of FLAGS1 is then tested to determine whether the active $\overline{\text{CONNECT}}$ signal is being received, i.e. whether the indicator 13 is actually connected to the control unit 11. If this bit is high, the indicator 13 is not connected, and a routine for signalling that the indicator is not connected is carried out, as shown by a branching from a decision 301 to a routine 302 in Fig. 26. If, as should be the case, the indicator 13 is connected to the control unit 11, either directly or through the junction box 23, bit 1 of FLAGS1 is tested to discover whether the a.c. mains supply is present on line M of the circuit 50 (Fig. 22). If bit 1 is high, the mains supply is not present and the program branches from the MAINS FAILED? decision 303 to a FIRST OCCURRENCE? decision 304 which determines from a test of bit 6 of the START square intended status byte (used as a flag for this purpose) whether or not this is a fresh occurrence of mains failure since the previous execution of the decision 303. It will be noted from Fig. 26 that a positive answer to the question posed at each decision is indicated by a branch with the reference letter Y, whereas a negative answer is indicated by continuation of the principal path of the program with the reference letter N. If the answer to decision 303 is negative, the flag bit 6 of the START square intended status byte is reset, and also bit 5 of this byte is reset, bit 5 serving as a flag for triggering execution of the audible indication of mains failure by another routine. If the decision 304 leads to a positive answer, bits 5 and 6 of the START square intended status byte are set, a re-entry pointer for a mains failure routine, which is part of the interrupt routine, is prepared, the CONTROL squares of the indicator 13 are turned off, and the switches of the mains switching unit 18 are opened. Whatever the results of the decisions 303 and 304, the next routines test first the external intercom 16 and then the internal intercom 17 before indicator scanning and square selection routines are executed at 305. After the scan and selection routines 305, the main program returns to the enabling of interrupts and reading of port 2 at 306 before the indicator connection decision 301.

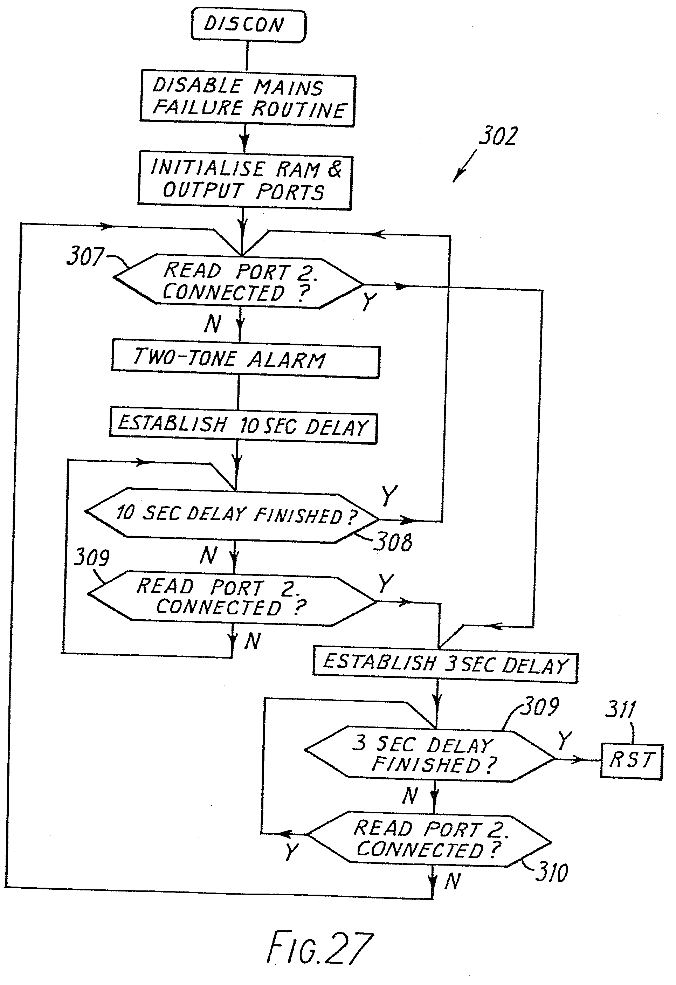

The "indicator disconnected" routine 302 which is labelled DISCON, is shown in detail in Fig. 27 and begins with the disabling of the mains failure routine by a suitable loading of the aforementioned re-entry pointer. After this, the RAM and the output ports are initialized as in the beginning part of the main program shown in Fig. 26. Port 2 is then read at data line D0 to test the $\overline{CONNECT}$ line. This test is represented by a block 307 in Fig. 27. If the indicator 13 is still disconnected, whether at the junction box 23 or at the control unit 11, a two-tone alarm is generated using TIM1 as a counter determining the frequencies of the two tones. Such routines are well known to those skilled in the art. A flow chart for the generation of a two-tone note is shown for example on page 68 of "Interfacing to microprocessors and microcomputers" by Owen Bishop published in 1982 by Butterworth & Co. (publishers) Ltd. of Borough Green, Sevenoaks, Kent TN15 8PH, England. A 10 second delay is then established using TIMER7 and a 10 second loop is executed in which port 2 is read at data line DO as indicated by the decisions at 308 and 309 in Fig. 27. Provided that the indicator 13 does not become connected to the control unit 11 during this 10 second loop, when the 10 seconds has expired, the routine returns to the reading of port 2 at 307 in Fig. 27. Thus the signalling of disconnection of the indicator 13 is maintained until the indicator 13 is connected to the control unit 11 or the system is turned off. If the indicator 13 is connected to the control unit 11 during the execution of the disconnected routine, a routine branches either from the decision 307 or from the decision 309 to the establishment of a 3 second delay, again using TIMER7 but with a 3 second count, and a 3 second loop is carried out with repeated reading of port 2 until either the indicator 13 becomes disconnected again or the 3 seconds expires in which case the whole system is re-started again as indicated by the two decisions 309 and 310 and the re-start command at 311 in Fig. 27. If the indicator 13 becomes disconnected during the 3 second loop, the routine jumps back to the decision at 307.

The testing of the external intercom indicated in the main program flow chart of Fig. 26 begins by testing bit 2 of FLAGS1 to discover whether the push button 20 of the external intercom 16 is depressed. If it is not depressed, bit 1 of a region of the RAM 34 labelled FLAGS2 is tested to discover whether the push button 20 was in the depressed state at the previous execution of this part of the program, the bit 1 of FLAGS2 having been set or re-set during the previous execution. If this bit 1 is found to be set, indicating that the push button 20 was not in the depressed state at the previous execution, then the program passes to the testing of the internal intercom. However, if bit 1 of FLAGS2 is found to be re-set, indicating that the push button 20 has just been released, the state of the relay RLY3 which controls connection of the external intercom 16 to the internal intercom is tested by testing bit 0 of P2STAT. If this test indicates that the external intercom 16 is connected, then a cuing tone of 0.1 seconds duration at 150 Hz is produced at the master speaker 22 by using TIMER5 and suitable setting of the tone switch T29, the speech switch T28, and the contacts of the change over relay RL1 of the circuit of Fig. 24. After the cuing tone has ended, the 0.02-seconds delay of TIMER8 is run before the intercom change-over relay RLY1 is switched over to allow the user to speak into the master speaker 22. If on the other hand, the test of bit 0 of P2STAT indicates that the contacts of relay RLY3 are open, then the byte in the RAM image of the lamps 24 and 25 representative of the red lamp 25 of the EXT.IC square is retrieved and the red lamp 25 of the EXT.IC square is turned off by re-setting bit 7 of this byte and transmitting the indicator address and the re-set bit 7 to the indicator 13 through the UART serial data link. This last process only has an observable effect if the red lamp 25 of the EXT.IC square was already on. If when at the beginning of the testing of the external intercom push button data bit in FLAGS1 the state of this bit is found to indicate that the push button 20 is depressed, then the state of this push button at the previous execution is tested and if found also to be in the depressed state, the program jumps to the testing of the internal intercom. If however, it is found that at the previous execution the push button 20 was not depressed, bit 1 of FLAGS2 is re-set to update the penultimate state data for the push button 20, and the present state of the change over relay RLY1 is stored in the RAM 34. The contacts of relay RLY1 are then switched to the position for the external intercom 16 to act as a microphone and the master speaker 22 to act as a loudspeaker and bit 0 of P2STAT is tested to discover whether the external intercom connection relay RLY3 has its contacts in the closed state. If these contacts are found to be closed, the program jumps straight to the testing of the internal intercom. If the contacts of relay RLY3 are found to be open, the red lamp 25 of the EXT.IC square is turned on, TIMER6 is run to determine the duration of a call tone of 0.25 seconds at the master speaker 22 and a re-entry pointer is loaded so that the program will enter the routine for testing the external intercom at the next point in the program at the next execution of the testing of the external intercom routine. When this next execution occurs, a test is carried out to determine whether TIMER6 has expired. If it has not expired, the program jumps directly to testing of the internal intercom. After a sufficient number of executions of the main loop of the main program between the routines at blocks 306 and 305 in Fig. 6 have been carried out for the TIMER6 to expire, the result of the testing for expiry of TIMER6 is switching of the contacts of the change-over relay RLY1 to allow the master speaker 22 to act as microphone, storing of the current state of port 7, dis-connection of the internal intercom 17 by de-energisation of its connection relay RLY2, energisation of relay RLY3 to ensure connection of the external intercom 16, running of the change-over delay TIMER8, production of the 0.25 second tone at the external intercom 16, and loading of the re-entry pointer to ensure that at the next execution of the main program loop will test for expiry of TIMER6 immediately on entering the testing of the external intercom routine. The main program jumps from the testing of the external intercom directly to the testing of the internal intercom at each execution of the main program loop between blocks 306 and 305 until TIMER6 has expired whereupon the result of this test is to restore the re-entry pointer data to ensure that the next execution of the main program loop the testing of the external intercom will begin again with testing of FLAGS1 for the current state of the push button 20, de-energisation of the external intercom connection relay RLY3, and restoration of the intercom circuitry to the state prevailing before the last change in the relay contacts, the main speaker 22 being switched to serve as a loudspeaker. This ensures that if the user has not selected the external intercom square on the indicator 13, the external intercom 16 is disconnected. The testing of the internal intercom indicated in the main program flow chart of Fig. 26 is substantially the same routine with changes to take into account that the push button 21 and the intercom unit 17 are involved, with the addition that when the testing of the internal intercom push button bit, which is bit 3, of FLAGS1 is carried out and found to show that the push button 21 has just been depressed since the previous execution of the main loop 306 to 305, then the TIMER7 is set running to allow 10 seconds for the user at the master speaker 22 to acknowledge the use of the internal intercom 17 by selecting the INT.IC square of the indicator 13 and thus cause a 0.25 second acknowledgement tone to be produced at the internal intercom 17 instead of the 3 seconds call tone which occurs when the user at the master speaker 22 selects the internal intercom square of the indicator 13 in the absence of the internal intercom push button 21 having been pushed within the last 10 seconds.

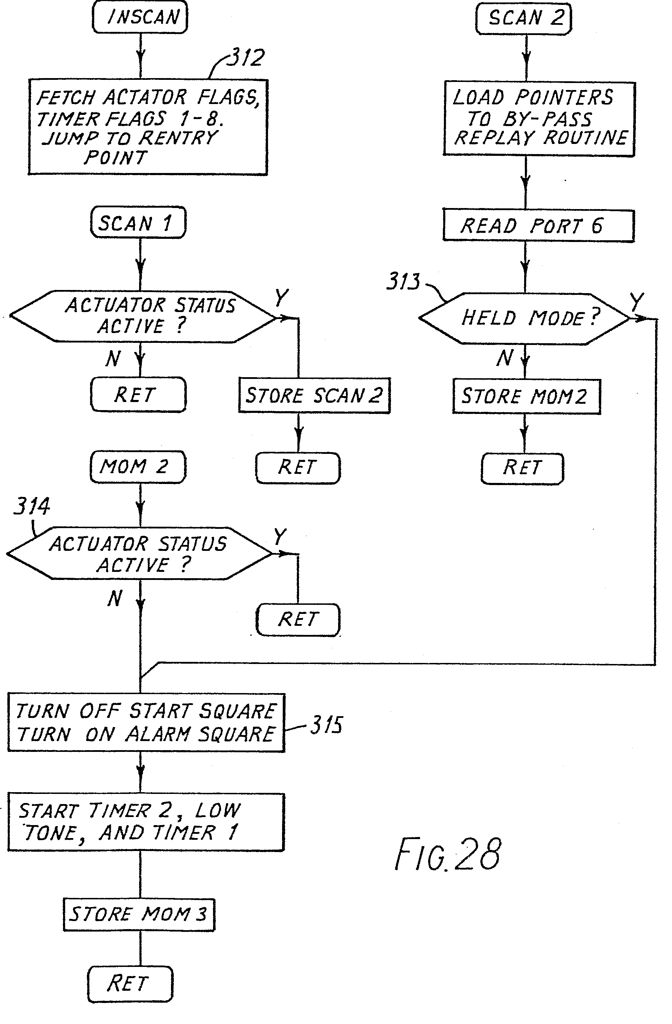

The scan and selection routines indicated by the block 305 in Fig. 26 are shown in more detail by the flow charts of Figs. 28 to 37.

The scan routine begins at a point labelled INSCAN in

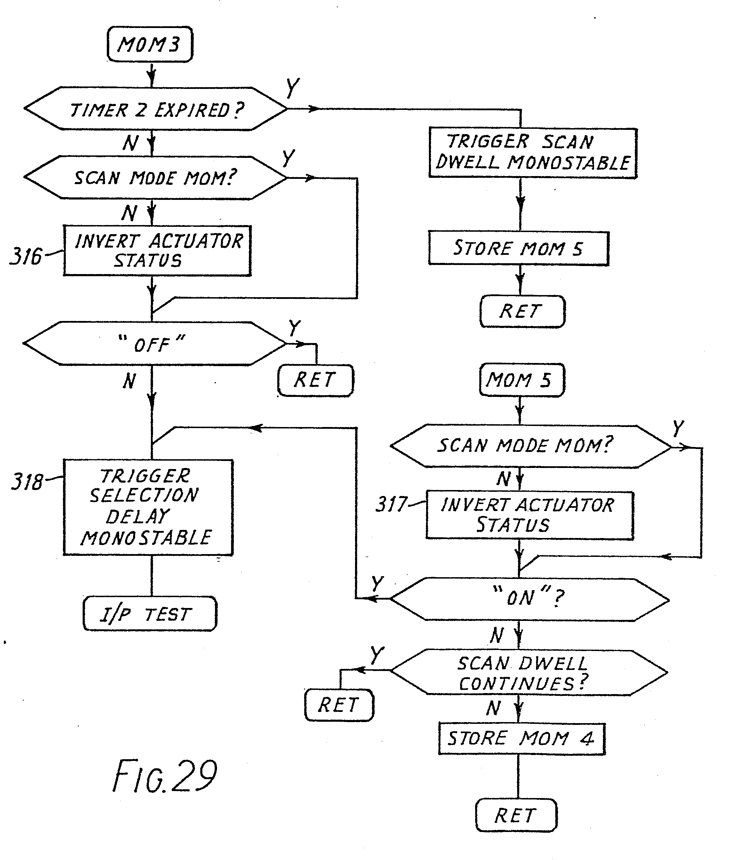

the program and the first processes in this routine are indicated in a block 312 in Fig. 28. The processes at 312 are data retrieval processes in which the de-bounced state of the input device switch, the running or not running states of the TIMERs 1 to 8 and the current re-entry point held in a re-entry pointer are retrieved from the RAM 34 by the CPU 30 and a jump is made to the current re-entry point. The re-entry points are labelled SCAN1, SCAN2, MOM2, MOM3, MOM4 and MOM5. The routines followed after these re-entry points are shown in the flow charts on Figs. 28 to 31. Normally, the first re-entry point jumped to at the first execution of the main program loop between blocks 306 and 305 of Fig. 26 is SCAN1. This tests for the input device switch, referred to in the flow charts as ACTUATOR, being active, i.e. closed. If the ACTUATOR is not active, the program returns to block 306. This return is indicated by the inscription RET in the flow charts of Figs. 28 to 31. If the ACTUATOR is found to be active, the label for the next re-entry point, SCAN2, is stored in the re-entry pointer and the program again returns to block 306. From SCAN2, the program loads re-entry pointers which determine the path through an interrupt routine and in this case so as to by-pass a replay routine which will be described hereinafter. Port 6, which is the port of the input, scan params and busy sensor circuit 47 of Fig. 3 is read and the data line D4 is tested to discover whether the switch 103 is closed or open (Fig. 13). If the switch 103 is open, the momentary mode of operation of the input device 12 is selected whereas if the switch 103 is closed, the held mode is selected. This test is represented by the decision at 313 in Fig. 28. If the momentary mode has been selected, the re-entry label MOM2 is stored in the re-entry pointer and the program returns to block 306. Re-entry at MOM2 tests the ACTUATOR status again at 314 and if the ACTUATOR is still active, i.e. closed, the program returns to block 306. When the decision 314 shows that the ACTUATOR is no longer active, the START square white lamp 24 is turned off and the white lamp 24 of the ALARM is turned on. TIMER2 is started to give the prolonged dwell on the ALARM square, the low tone at the indicator 13 is turned on and TIMER1 is started to determine the duration of the low tone at the indicator. The label MOM3 is then stored in the re-entry pointer and a return is executed. If at the decision 313 the held mode is found, the program branches straight to turning off of the START square at 315 in MOM2, since in the held mode, scan proceeds as long as the ACTUATOR switch is held closed. Fig. 29 shows the routine following re-entry at MOM3. The routine from MOM3 and MOM5 which is also shown in Fig. 29 completes the scan dwell on the ALARM square and tests for selection of this square by, in the case of the momentary mode, the ACTUATOR switch becoming active, and in the case of the held mode, the ACTUATOR switch becoming inactive, while the white lamp 24 of the ALARM square is still on. In order to simplify the routine for dealing with the held mode, the data relating to the ACTUATOR switch is inverted if the held mode is prevailing, so that the tests for activity of the ACTUATOR can be the same at this stage for both modes. Inversion of the ACTUATOR status following detection of the held mode is indicated at block 316 and 317 in Fig. 29. It will be seen that the active time of the scan dwell monostable is added to the time of TIMER2 in the case of the ALARM square by the routines of Fig. 29. Also it will be seen that if selection of the ALARM square is detected, the selection delay monostable is triggered at block 318, which leads to an input testing routine labelled I/P TEST.

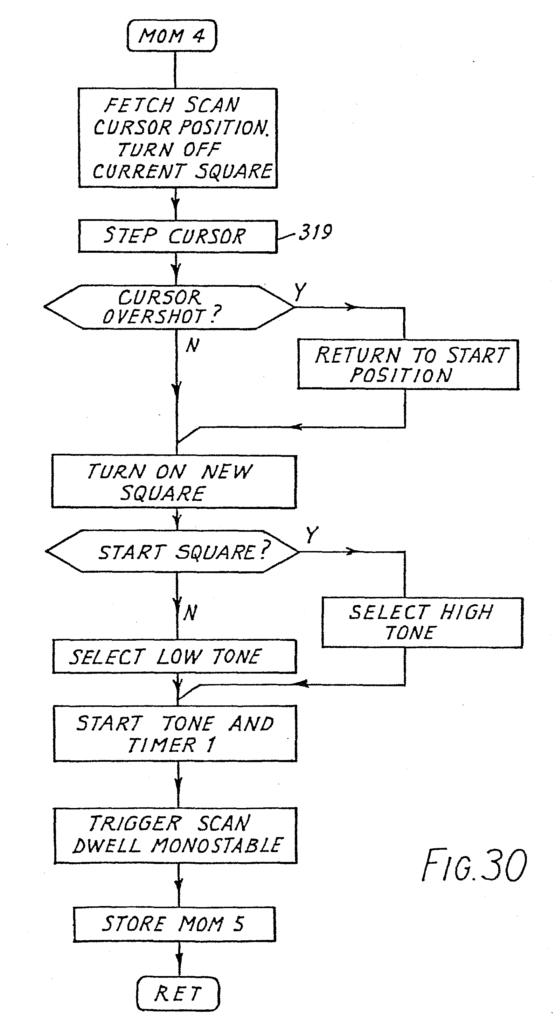

If the ALARM square is not selected, the program jumps to the re-entry point MOM4 which leads to the routine shown in Fig. 30. The first step in this routine is to fetch from the RAM 34, stored data giving the indicator address of the white lamp 24 which is currently on. This lamp 24 is then turned off by using the UART link and the address of the lamp 24 which is to be on is increased by 1 as indicated by the block inscribed STEP CURSOR at 310 in Fig. 30. The resultant indicator address is tested against the value which occurs for the unused line L14 of the indicator decoder 58 in order to discover whether the cursor has overshot. If it has, the indicator address for the cursor lamp 24 is replaced by 00H which is the address for the white lamp 24 of the START square, as indicated at block 320 of Fig. 30. If the cursor has not overshot, or if the START square address has been substituted, the UART link is used to turn on lamp 24 at the new indicator address. If this is at the START square, the high tone is selected at the indicator whereas if it is not the START square, the low tone is selected. TIMER1 is started and the scan dwell monostable is triggered before MOM5 is stored in the re-entry pointer. Thus the routine of Fig. 30 carries out the stepping of the cursor lamp 24 from one square to another on the indicator 13 and the routine beginning at the re-entry point MOM5 in Fig. 29 tests for each new square whether this square is selected.

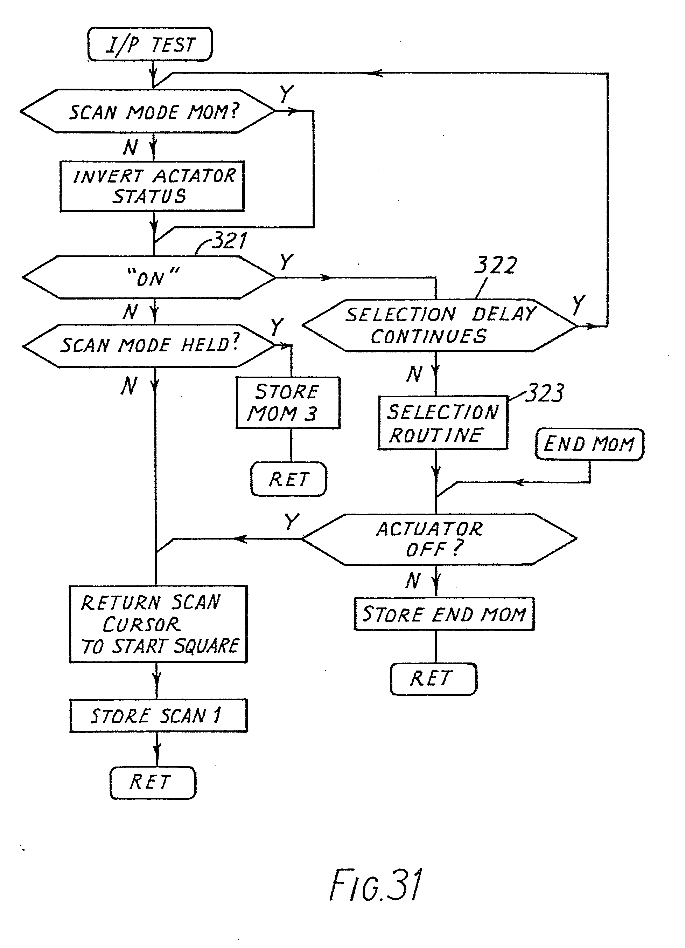

When selection for square occurs, as shown in Fig. 29 the routine jumps to a point labelled in this example, I/PTEST. Fig. 31 shows the routine following this point.

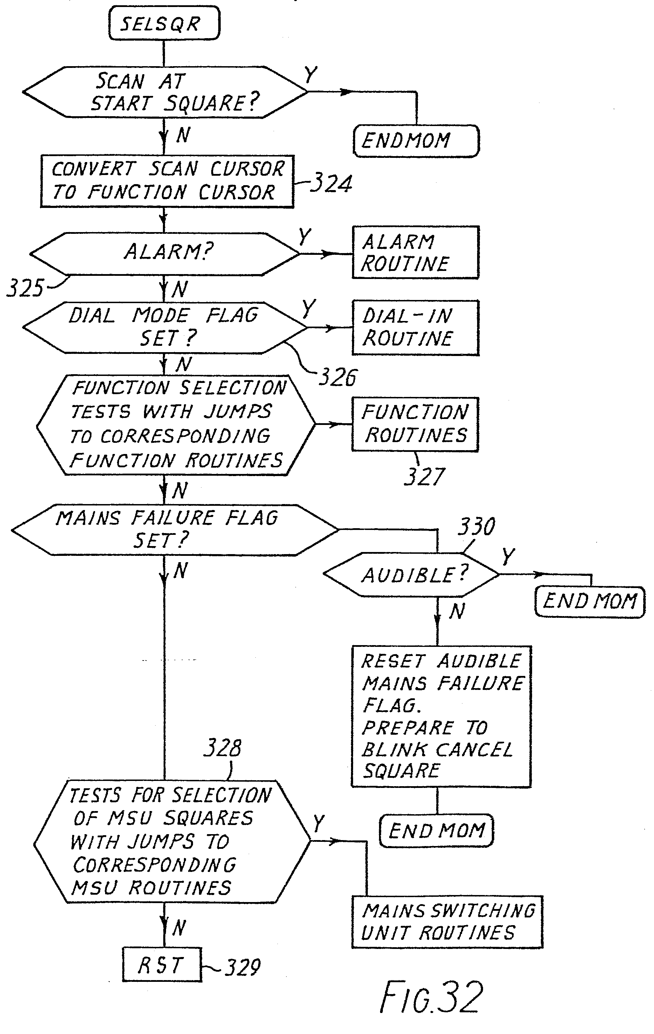

The first part of the routine following the jump to I/PTEST determines which mode is prevailing and tests at a decision 321 for maintenance of the selection. Since this test is carried out immediately after triggering of the selection delay monostable, if the ACTUATOR switch is found not to be active, i.e. in this case open, if the momentary mode is prevailing and closed, if the held mode is prevailing, the routine returns the program to a condition in which normal scanning proceeds again either from the re-entry point SCAN1 or the re-entry MOM3 depending upon whether the momentary or the held mode prevails. If however the selection is found to be maintained at the decision 321, the program loops through the initial part of this routine to the decision 321 until the selection delay expires, as indicated by the decision 322. At expiry of the selection delay, the selection routine at 323 is entered. The selection routine is shown in more detail in Fig. 32. After the selection routine has been completed, the program tests for the ACTUATOR switch being open, whether in the held or the momentary mode, and continues at each execution of the main program to carry out this test by re-entering at a point labelled ENDMOM. When the ACTUATOR is found to be off, i.e. the input device switch is open, the routine returns the indicator 13 to the state with the white lamp 24 of the START square on.

As seen from Fig. 32, if the selected square is found to be the START square, the routine returns the program to the point ENDMOM of Fig. 31. If any other square is selected, the indicator address, referred to in block 324 of Fig. 32 as the SCAN CURSOR, is converted into the indicator address for the red lamp 25 of the same square. The resultant indicator address is immediately tested at a decision 325 to discover whether the ALARM square has been selected and if it has, an alarm routine is carried out. If the selected square is not the ALARM square, a dial mode flag in the RAM 34 is tested to discover whether the system is in the dialling mode in which the indicator squares are to be treated as telephone digits, end of dial and cancel function. This decision is indicated at 326 in Fig. 32 and if it is found that the dial mode FLAG is set, the routine branches to a DIAL-IN routine. If the dial mode has not been selected, the function cursor address is tested against the addresses for the various functions indicated by the squares of the indicator 13 except for the CONTROL squares. If one of the indicator functions is found to match the selected square, the routine branches to the appropriate function routine as indicated at 327 in Fig. 32. If none of the tested addresses at this stage matches the function cursor address the program proceeds to test whether a mains failure flag is set, this being bit 6 of a byte associated with the START square in the RAM 34. Provided this flag is found not to be set, the tests for the CONTROL squares are carried out as indicated at 328 in Fig. 32 and the program branches to the appropriate routine for the selected address. If no address matches the function cursor at this stage, the system is re-started as indicated at 329. If the mains failure flag has been found to be set, instead of the tests at 328 being carried out, the routine branches to a test for the audible signal for mains failure having been cancelled by selection of CONTROL D. The decision 330 determining this leads to the point ENDMOM of Fig. 31 if the audible signal has not been cancelled and otherwise leads to re-setting of a flag for the audible signal for mains failure and the preparation of a further routine for blinking of the CANCEL square which is the CONTROL D square of the indicator 13. The routine then again jumps to ENDMOM.

If the indicator square for the external intercom 16 is selected, the corresponding function routine of block 327 of Fig. 32 turns on the red lamp 25 of the external intercom square if not already on, or turns it off if it is already on. If the selection turns the red lamp 25 on, the various relays and switches of the intercom circuit are operated to ensure that the external intercom 16 can operate as microphone and the internal intercom 17 is disconnected. If the selection turns the red lamp off, both remote intercom units 16 and 17 are disconnected. The program returns from the end of such routines to the block 306 of the main program via ENDMOM. If the internal intercom square is selected, routines similar to those for the external intercom are carried out except that the state of TIMER7 is tested and if running, the 0.25 seconds acknowledgement tone is produced at the internal intercom 17, or if not running, the 3 second call tone is produced.

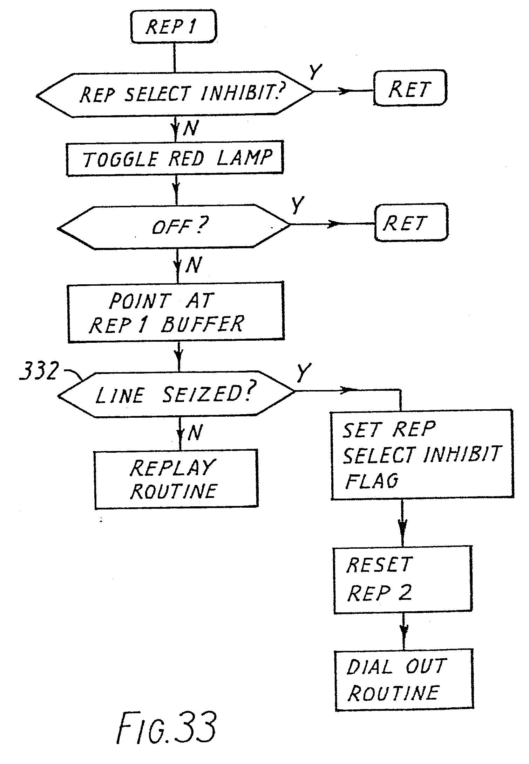

If the REP1 square is selected, the routine of Fig. 33 is carried out. If the red lamp 25 of the REP1 square is turned on by the selection, a pointer used in routing data in the routines concerned with use of the telephone 19 is arranged to point at a buffer store of the RAM 34 which is termed the REP1 buffer as indicated at 331 in Fig. 33. Stored data is then tested to discover whether the telephone line has been seized. The decision at 332 determines whether a replay routine is carried out or data is prepared for dialling out telephone digits stored in the REP1 buffer.

A similar routine is carried out in connection with a REP2 buffer if the REP2 square is selected and its red lamp 25 comes on as a result.

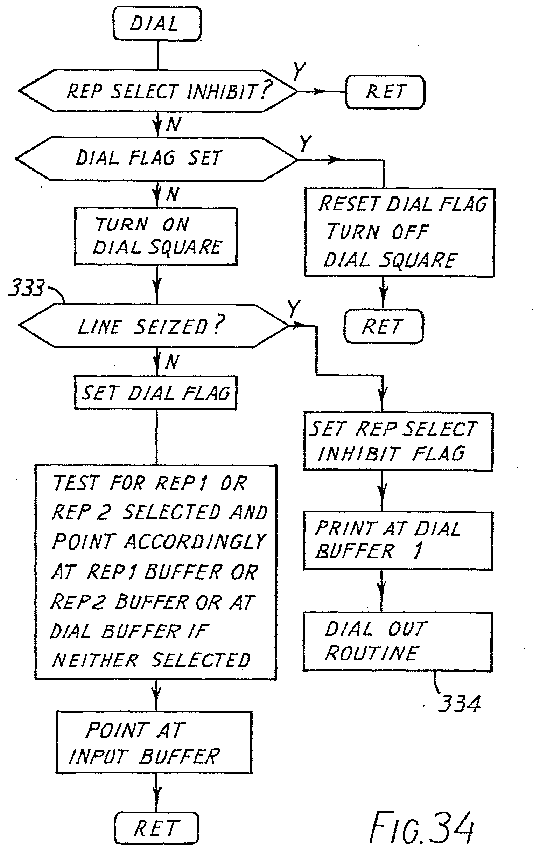

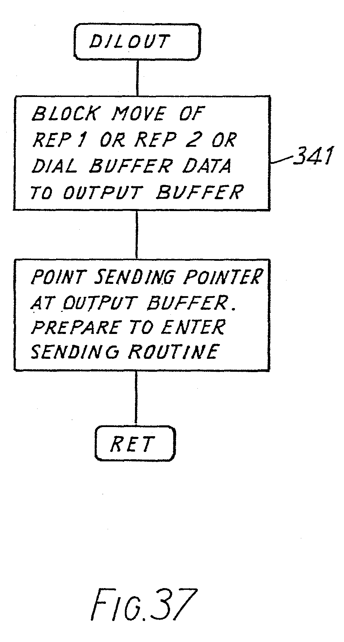

If the DIAL square is selected, the routine shown in Fig. 34 is carried out. It will be seen that the routines for selection of REP1 and DIAL and also for REP2, begin with testing for inhibition of selection of REP1 or REP2. This is a test for a flag termed the REP SELECT INHIBIT FLAG having been set. Setting of the REP SELECT INHIBIT FLAG is effected in the routines for REP1, REP2 and DIAL if selection of the corresponding square has brought the red lamp on and the telephone line is found to have been seized. As shown in the routines of Figs. 33 and 34, setting of the REP SELECT INHIBIT FLAG is carried out immediately after the test for line seizure at 332 in Fig. 33 and 333 in Fig. 34. This prevents accidental dialling out of numbers stored in REP1 buffer or REP2 buffer when REP2 or REP1 or DIAL is selected for dialling out. The DIAL routine uses a DIAL flag which is tested to effect the toggling of the red lamp 25 of the DIAL square. If the telephone line has not been seized, the DIAL flag is set, tests are carried out to discover whether the square for REP1 or REP2 has been selected immediately before, and a pointer is arranged to point at one of three buffers, namely REP1 buffer, REP2 buffer and DIAL buffer. The chosen buffer is REP1 buffer if the REP1 square was selected immediately before the DIAL square, REP2 buffer if the REP2 square was selected immediately before the DIAL square, and is the DIAL buffer if neither REP1 or REP2 was selected before the DIAL square. Another pointer is then arranged to point at an input buffer before the routine returns to block 306 of the main program. Thus data locations in the RAM are prepared for the entry of digits to be used as telephone subscriber number digits in subsequent use of the telephone 19. If at the decision 333 in Fig. 34 the telephone line is found to be seized, a block move to transfer data from the DIAL buffer is prepared before the dialling out routine, which is shown in Fig. 37, is entered as indicated at 334 of Fig. 34.

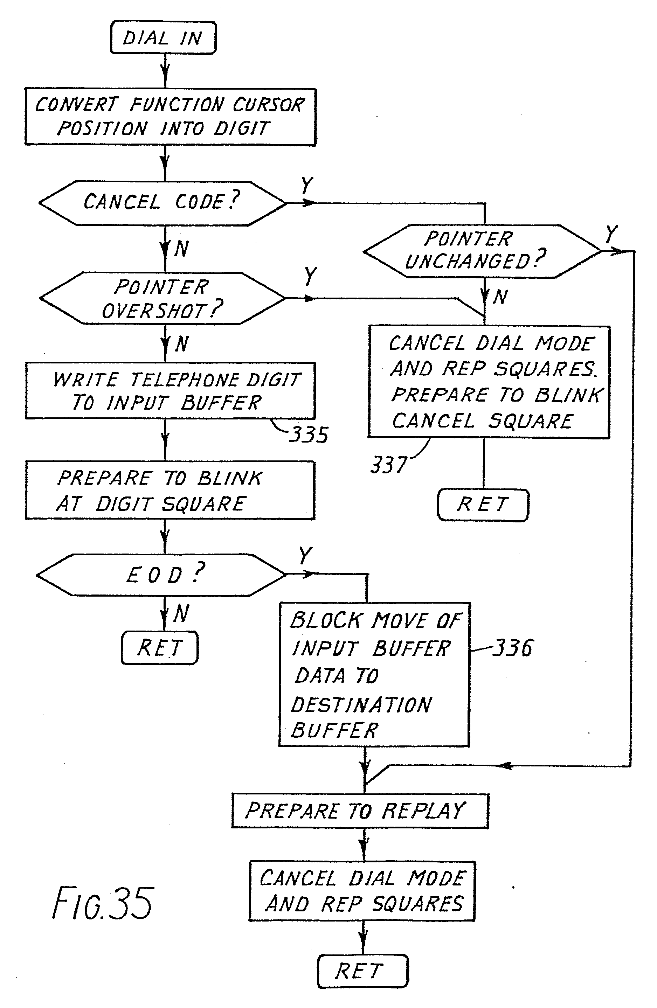

If the DIAL square has been selected so as to set the DIAL flag after the decision at 333, then on the next execution of the main program loop from the block 306 of Fig. 26 the scan and selection routines 305 lead in the selection routine of Fig. 32 at the decision 326 to the DIAL-IN routine of Fig. 35. The first stage of this routine is conversion of the function cursor indicator address into a telephone digit which is effected by subtracting 12H from the indicator address, as will be seen by consideration of Fig. 2. Then if the CANCEL code, which is the number obtained for the CONTROL D address, has not been selected, a test is carried out to determine whether the dialling in procedure is to be aborted as a result of over-shooting of the pointer pointing to the input buffer for telephone digits and, assuming that there is no over-shoot the selected digit is stored in the input buffer as indicated at 335 in Fig. 35. Preparations are then made for a routine which blinks the white lamp 24 of the selected square, the stored telephone digit is tested to discover whether it represents the end of dial code corresponding to selection of the CONTROL C square and the program returns to ENDMOM if the telephone digit is not the end of dial code. When the required number of telephone digits have been entered into the input buffer by the DIAL-IN routine, the end of dial square is selected by the user and the dialling in routine of Fig. 35 results in a block move of the input buffer data to the destination buffer which is identified by the pointer pointing at the REP1 buffer, the REP2 buffer, or the DIAL buffer as arranged in the routine of Fig. 34. For example, since the CPU 30 is a Z80, the block move can be effected by means of the instruction LDDR. This block move is represented at 336 in Fig. 35. After the block move, a replay routine for indicating the now stored telephone subscriber number in the destination buffer by means of blinking at the indicator 13 is prepared. Then the dial flag and the red lamps of the REP1, REP2 and DIAL squares are reset and turned off respectively before the program returns to ENDMOM.

If the DIAL square is selected and then the CONTROL D square is selected, the DIAL-IN routine detects the CANCEL code as indicated in Fig. 35 and if the pointer for pointing to the input buffer shows that no digits have been entered into the input buffer, the preparation of the replay of the destination buffer contents is prepared. If the dialling in routine detects that the input buffer pointer has over-shot or that the CANCEL code is present and a change has a occurred in the input buffer pointer indicating that some digits have been entered into the input buffer, then the DIAL and REP1 and REP2 squares and the DIAL mode are all cancelled and the blinking routine for the CANCEL square, i.e. the CONTROL D square, is prepared as indicated at 337 in Fig. 35.

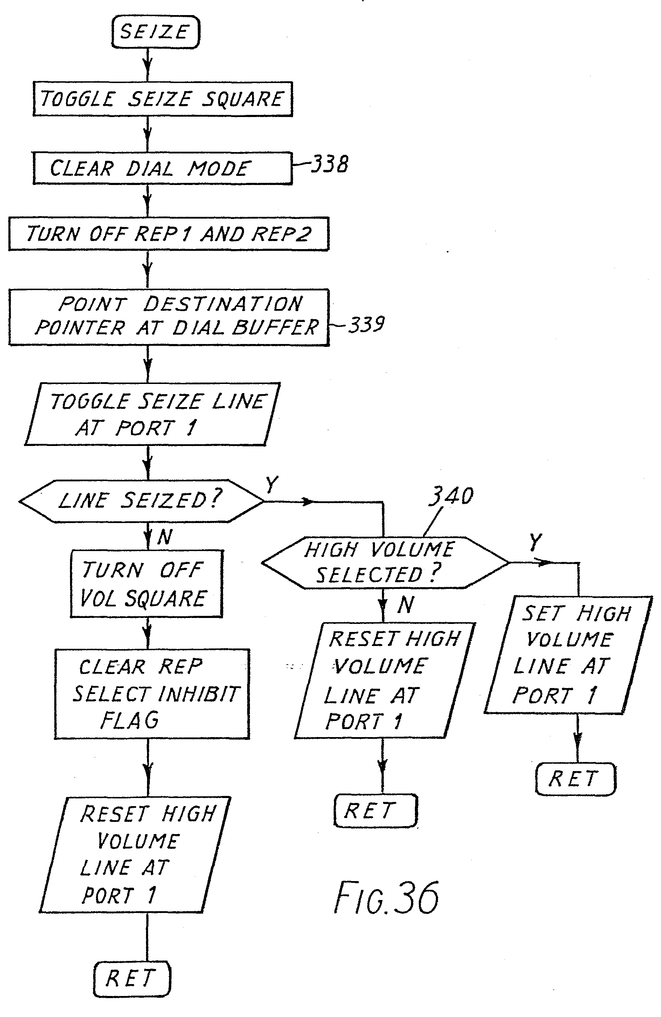

Fig. 36 shows the routine occurring on selection of the SEIZE square. The routine includes resetting the DIAL flag at 338 following toggling of the red lamp of the SEIZE square. Also, the destination pointer is pointed at the DIAL buffer since most frequently the number to be dialled out will be that held in the DIAL buffer (block 339). The data line DO at port 1 is then toggled in order to seize or release the telephone line in accordance with the circumstances of the selection of the SEIZE square. If the line has been seized, tests are then carried out to determine from information held in the RAM 34 whether the high volume has been selected for the telephone 19. This decision at 340 leads either to setting or resetting of the high volume line at port 1 via data line D2. If the line has been released, the VOL square is turned off, the REP selection inhibit flag is reset, and the high volume line at port 1 is reset before the program returns to ENDMOM.

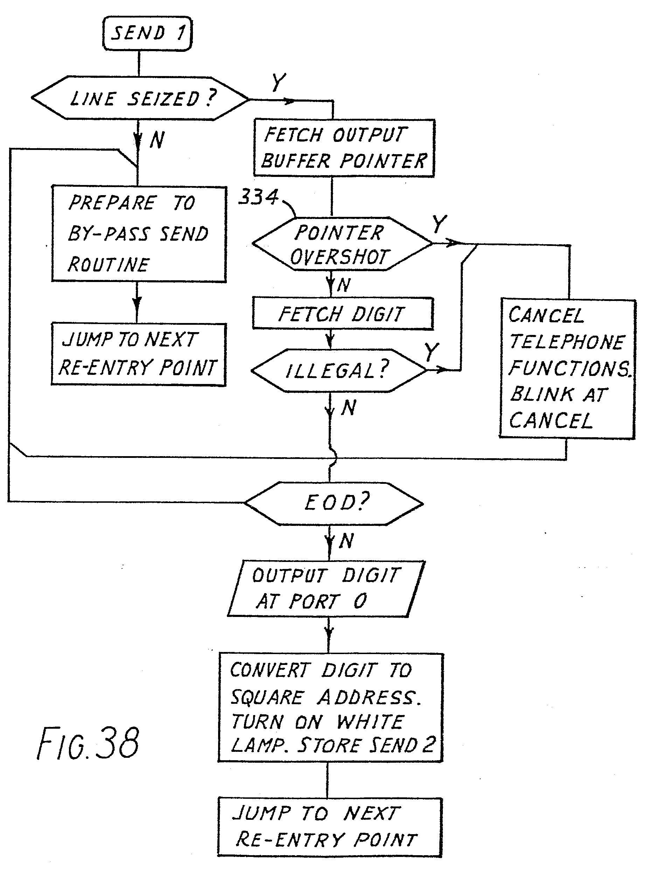

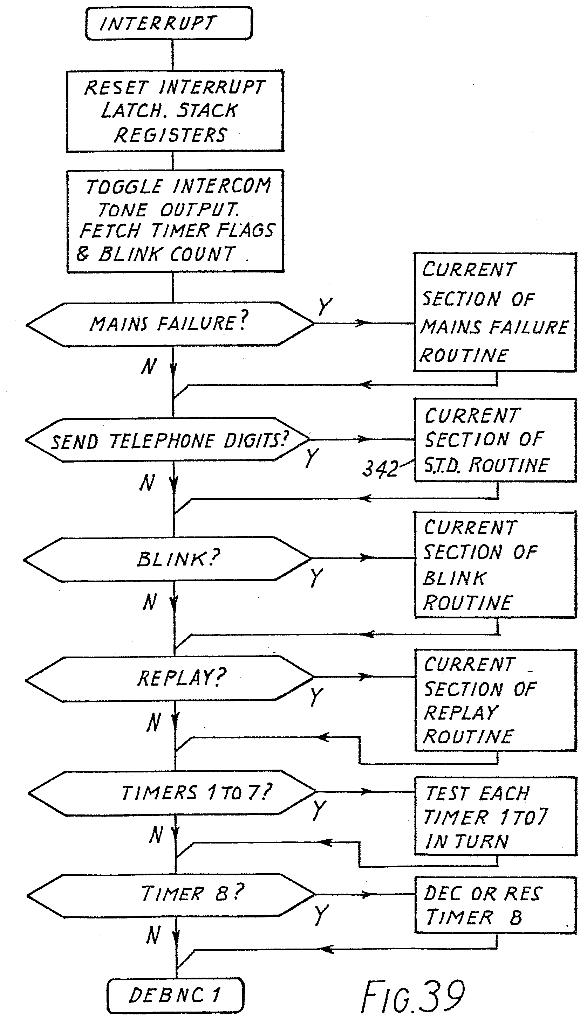

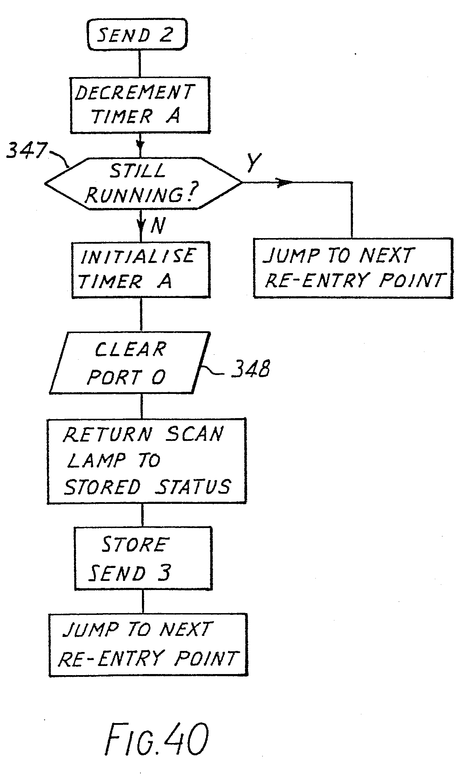

If in the REP1, REP2 or DIAL routine the telephone line is found to be seized, the routine leads to the DIAL OUT routine which is shown in Fig. 37. The first stage of the DIAL OUT routine is a block move of data held in the appropriate buffer, REP1 buffer, REP2 buffer or DIAL buffer to an output buffer, as indicated at 341 in Fig. 37. Then a re-entry pointer for a sending routine is pointed at the output buffer in order to prepare for execution of a sending routine which is effected by the interrupt. The interrupt routine is shown in Fig. 39 and includes a number of tests, in the form of retrieval of the contents of various re-entry pointers, which either cause branching to particular sections of the interrupt program or jumping to the next test in the series. One of these tests is performed by retrieving the re-entry pointer for a routine for sending telephone digits. This routine is indicated at 342 in Fig. 39 and consists of a number of sections each of which maybe performed at an execution of the interrupt routine depending upon the precise address found in the relevant re-entry pointer. There are three such re-entry points, labelled SEND1, SEND2 and SEND3, and the sections of routine into which these points lead are shown respectively in Figs. 38, 40 and 41. Normally the re-entry pointer for the sending telephone digits routine is arranged to cause the interrupt program to by-pass this routine. However, if the dialling out routine of Fig. 37 is executed, the label SEND1 is stored in the re-entry pointer and the next interrupt routine executed after this has occurred executes the section of the sending routine which begins at SEND1. The first step in this section of the routine is testing for seizure of the telephone line at 343. Unless the user has decided not to carry on with the dialling out of the selected telephone number, the line will be found to be seized and the decision 343 leads to fetching of the output buffer pointer which is checked for having over-shot the output buffer at decision 344. The pointer should not have over-shot the output buffer and therefore the next step should be the fetching of the first telephone subscriber number digit from the output buffer at step 345. The telephone digit is tested against the value OCH to discover whether it is an allowed digit value, which it should be, and if this test is passed, the telephone digit is tested against OAH which is the end of dialling code at a decision 346. If the telephone digit is not the end of dial code, the telephone digit is transmitted to port 0 where it activates one of the ten dial terminals 261 to 270. (Fig. 25). The telephone digit is then converted into the corresponding indicator square number by the addition of 02H, the corresponding white lamp 24 at the indicator is turned on, and the re-entry point SEND2 is stored in the re-entry pointer. The routine then jumps to the next test in the interrupt routine. At the next execution of the interrupt routine the result of the test for sending of telephone digits directs the program to SEND2 which, as shown in Fig. 40 begins by decrementing TIMER A, which is loaded with its initial value in the initialization routine in the beginning part of the main program, and tests for expiry of TIMER A at a decision 347. If the TIMER A is still running, the routine then jumps to the next re-entry point which is the test following that for sending telephone digits. Thus for a number of executions of the interrupt routine, the routine concerned with the sending of telephone digits only runs down TIMER A to expiry. When expiry occurs, the initial value is reloaded into TIMER A and all the telephone output terminals 261 to 270 of Fig. 25 are set high to mark the end of the first telephone digit as indicated at 348 in Fig. 40. The white lamp 24 of the telephone digit just transmitted is then turned off or allowed to stay on depending on the condition in which it was found before being turned on to indicate transmission of the telephone digit. The label of the re-entry point SEND3 is then stored in the re-entry pointer and the routine jumps to the next test in the interrupt routine. At the next execution of the interrupt routine, the telephone digits sending routine begins at SEND3 by decrementing TIMER A, and testing for its expiry as in the section from SEND2. When a sufficient number of interrupts have occurred for TIMER A to have expired, the section of routine from SEND3 leads to initialization of the value in TIMER A again, and storing of SEND1 in the pointer before the routine jumps to the next test in the interrupt routine. Thus the next telephone digit can be transmitted in the same way. This continues until the section of routine following SEND1 detects the end of dial code whereupon the re-entry pointer is loaded with the address for the next test in the interrupt routine and then jumps to that point, as indicated at blocks 349 and 350 of Fig. 38. If during the section following SEND1 an illegal code is detected or the output buffer pointer over-shoots, a cancellation stage is carried out and re-entry pointers are set to establish blinking of the CANCEL square of the indicator 13, i.e.
the CONTROL D square, as indicated at block 351 of
Fig. 38.

From Fig. 39 it will be seen that the interrupt
routine begins with the resetting of the interrupt latch,
which is the bistable circuit 66 of Fig. 6, and stacking
of the CPU registers. The intercom tone is then toggled
by changing over the signal level on data line D3 at port
2. Since the interrupt occurs three hundred times a
second, this ensures that the intercom tone is 150 Hz.
The contents of the timer flag store, labelled DESAV1, are
then fetched so that the states of TIMERs 1 to 8 can be
tested subsequently. The contents of the blink parameter
store, labelled BCSAV1, are also fetched so that an account
of the on/off and off/on transistions in a blink routine
can be tracked. The interrupt routine then proceeds to a
first test which is the test for activation of the
interrupt mains failure routine which consists in
retrieving the contents of a mains failure re-entry
pointer and jumping to the address which these contents
constitute. If this address is the beginning of the mains
failure routine, the red lamp 25 of the START square is
switched on if bit 6 of the intended status byte for the
START square is found to be set. Then bit 5 of this mains
failure byte is tested to discover whether the audible
signal for mains failure has been cancelled. If it has
not been cancelled, the relays and switches of the
intercom system are adjusted to enable the 150 Hz tone to
be produced at the internal intercom 17 concurrently with
illumination of the red lamp of the START square. The
high tone at the indicator 13 is also enabled to sound
concurrently. After this first section of the mains
failure routine, the telephone digits sending test is
carried out after a mains failure routine pointer has been
loaded to prepare for re-entry at a second section of the
mains failure routine. The second section of the mains failure routine is carried out in the next interrupt
routine executed and in subsequent interrupts, this
section serving to count down TIMER C to expiry. On
expiry of TIMER C, the second section reloads TIMER C and
switches off the red lamp of the START square and the two
concurrently operating audible signals. A third section
of the mains failure routine is executed after the second
section has reloaded the re-entry pointer. The third
section is similar to the second section but instead of
turning off the red lamp of the START square at the expiry
of TIMER C, the third section turns this lamp on again and
re-establishes the two audible signals if bit 5 of the
mains failure routine byte is not in the reset state. The
re-entry pointer is reloaded for re-entry at a fourth
section which is corresponds to the second section of the
mains failure routine and finishes with reloading of the
re-entry pointer to effect re-entry into a fifth section.
The fifth section of the mains failure routine is repeated
decrementing of TIMER B and testing for expiry and when
expiry of TIMER B occurs, the initial value is reloaded
into TIMER B and the re-entry pointer is reloaded to point
to the address of the beginning of the first section of
the mains failure routine before the program finally moves
on to the test for telephone digits to be sent.

It will be seen from Fig. 33 that when the line is
not seized and REP1 has been selected, the replay routine
is executed. The same replay routine is executed if REP2
is selected and the line is not seized. The replay
routine is set up in the main program and brought into
effect by the interrupt routine. The setting up of the
replay routine consists in storing the address of the REP1
buffer in a replay pointer labelled HLSAV1 and loading the
interrupt routine replay re-entry pointer with the address
of the beginning of the first section of the interrupt
replay routine, these two processes being carried out with
interrupts disabled. The interrupt replay routine has two replay sections. In the first section, the address stored in HLSAV1 is fetched and decremented and the resulting address tested to see whether it is a valid address for the buffer, and if it is, the telephone digit at that address is tested for validity and if valid is converted by the addition of 02H into the indicator address of the corresponding indicator square and loaded into a blink cursor store allocated in the RAM 34 and the re-entry pointer for the blink routine prepared for causing the blink routine to be executed by the interrupt routine. The replay re-entry pointer is then loaded with the address of the beginning of the second section of the ιeplay routine and a jump made to the next test in the interrupt routine which is the test for the TIMERs 1 to 7. The second section of the replay routine is accordingly entered at the next execution of the interrupt routine and consists in testing the content of the blink cursor store to discover whether the indicator square address held there is that of the end of dial square, i.e. the CONTROL C and if it is not, a jump is made immediately to the beginning of the first section of the replay routine in the interrupt to enable the next telephone digit to be obtained from the REP1 buffer and converted into an indicator address as described hereinbefore. When eventually the second section of the interrupt replay routine comes to the end of dial square address in the blink cursor store, instead of a jump being made to the beginning of the first section of the interrupt replay routine, the replay re-entry pointer is loaded with the first address in the next test, which is the test for TIMERs 1 to 7, and the interrupt routine proceeds immediately to this next test also. Consequently at the next interrupt routine execution, the replay routine is by-passed. If in the first section of the replay routine the buffer pointer is found to be pointing at an invalid address or the telephone digit obtained from the buffer is not valid, an error sub-routine is carried out in which the indicator address of the CANCEL square, i.e. the CONTROL D square is loaded into the blink cursor store, the address of the first section of the blink routine is stored in the blink routine re-entry pointer, the address of the beginning of the first section of the timer testing routine is stored in the replay routine re-entry pointer and a jump is made immediately to the beginning of the timer testing routine. Thus preparations are made for the error to be indicated by blinking of the CANCEL square and the replay routine of the interrupt routine is by-passed in subsequent interrupts.

The blink routine of the interrupt routine can be entered as a result of the execution of the replay routine or various other routines. Entry into the interrupt blink routine is effected whenever the blink routine re-entry pointer is loaded with the address of the beginning of the first section of the interrupt blink routine. Such loading is carried out in the first section of the telephone digit sending routine, which begins at SEND1. Where the blink routine is to be entered as the result of processes carried out in the main part of the program, as for example in the DIAL IN routine of Fig. 35, a main blink routine is carried out in the main program as follows. The blink transistion counter, BCSAV1, is reloaded with its maximum count of 07H, the blink re-entry pointer is loaded with the first address of the first section of the interrupt replay routine to ensure bypassing of any pre-existing blink routine, and TIMER9 is loaded with its initial value of 0.05 seconds. The address of the white lamp 24 which is currently in the blink cursor store is then obtained and that lamp 24 is returned to its intended status which is found from the contents at the corresponding address in the RAM 34 for the intended status of that lamp, and the address of the red lamp 25 of the square selected for the blink routine which is to be executed is obtained and converted into the indicator address of the corresponding white lamp 24 and that white lamp indicator address is now stored in the blink cursor store. Finally, the address of the beginning of the first section of the interrupt blink routine is stored in the blink re-entry pointer. This routine in the main part of the program for preparing for execution of the interrupt blink routine is carried out with interrupts disabled.

The interrupt blink routine has three over-lapping sections. In the first section, the blink transistions counter BCSAV1 is decremented and tested for zero, which is not normally found until the required number of executions of the blink routine have been carried out, and on finding the blink transistions counter to be not zero, port 6 is read at data line D5 to discover whether the UART is busy, and if it is busy, a jump is made to the beginning of the timer testing routine, the interrupt replay routine being by-passed. In the first section of the interrupt blink routine, the address of the second section is loaded into the re-entry pointer before the UART busy signal is tested. The beginning of the second section of the blink routine is the reading of port 6 at D5 for the UART busy signal test. Thus the end of the first section of the blink routine can be repeated as a beginning part of the second section at successive executions of the interrupt routine until the UART is not busy. As soon as the UART is found to be not busy, the second section continues by fetching the white lamp address stored in the blink cursor store, setting bit 0 of the intended status byte for this lamp as a blink flag, and toggling the on/off bit of the actual status byte and transmitting this byte to the UART to effect switching of the white lamp 24 at the indicator. TIMER9 is then decremented and tested for zero. If TIMER9 is not zero, a jump is made immediately to the beginning of the testing of timers routine. The address of the step of decrementing TIMER9 is stored in the blink re-entry pointer so that at the next execution of the interrupt routine, the blink routine jumps immediately to decrementing and testing TIMER9 for zero. When TIMER9 is found to be zero, the initial value for TIMER9 is reloaded into TIMER9 and a jump is made to the step in the first section of the blink routine after which the blink transistions counter BCSAV1 is decremented. Thus the blink routine proceeds to turn on and off the white lamp 24 at the address which is stored in the blink cursor store until the blink transistions counter BCSAV1 has reached zero whereupon a jump is made from that execution of the first section of the blink routine to a final stage which retrieves the status of the white lamp 24 stored before it began to be blinked and compares this with the actual state of the lamp. If the two states are the same, the blink transistions counter BCSAV1 is reloaded with the initial value of transistions, the blink flag of the intended status of the white lamp 24 which has just been blinked is reset, the re-entry pointer for the replay routine is restored and the blink re-entry pointer is loaded with the test for the replay routine so that the blink routine will be by-passed at the next interrupt execution. If the compared states of the white lamp 24 are found not to be the same, the blink transistions counter BCSAV1 is incremented to 01H and a jump is made to the beginning of the second section of the blink routine so that the white lamp 24 is returned to the state in which it was before the blink routine began.

The timer testing routine of the interrupt routine begins by retrieving the contents of the timer flag store DESAV1 and testing this to see whether any of the timer flags for TIMERs 1 to 7 are set. If none are set, a jump is made directly to testing of the timer flag in DESAV1 for TIMER8. Where any timer flag is found to be set, the routine decrements the current value of the timer, tests the result for zero, and reloads the timer with the initial value if the result of the decrement step is zero. Where the timer is reloaded, the corresponding timer flag in DESAV1 is reset. Also, in certain cases, other steps are carried out in connection with the operation of the timer. Thus if TIMER1 is found to be zero, the currently active indicator decoder output line L30 or L31 is rendered inactive to cause the tone being generated at the indicator 13 to be terminated. If TIMER3 is found to have expired, the tone switching transistor T29 of the intercom circuit of Fig. 24 is rendered conducting and the speech channel switching transistor T28 is rendered non-conducting so that the intercom call tone or acknowledgement tone is terminated and the intercom speech channel enabled. If TIMER4 is found to have expired, the red lamp 25 of the DOOR square and the door release device 14 are de-energised. If TIMER5 is found to have expired, the tone switching transistor T29 of the intercom circuit of Fig. 24 is made conducting to terminate the 150 Hz tone. Expiry of TIMER6 also results in turning off of the 150 Hz intercom tone.

If mains failure is detected in the main program as indicated at decision 304 of Fig. 26, the interrupt mains failure routine re-entry pointer is loaded with the address of the beginning of the interrupt mains failure routine which has five sections. The first section checks that the mains failure flag which is bit 6 of the intended status byte for the START square is still set, and if it is found to be reset, indicating that this is not the first interrupt since mains failure was detected, TIMERs B and C are reloaded with their initial values, the red lamp 25 of the START square is turned off and the mains failure re-entry pointer is loaded with the address of the beginning of the first section of the telephone digit sending routine so that the mains failure routine of the interrupt routine will be by-passed at subsquent executions of the interrupt routine. At the first execution of the interrupt routine after mains failure is detected in the main program, bit 6 of the START square intended status byte is found to be set and the first section of the interrupt mains failure routine turns on the red lamp 25 of the START square, tests bit 5 of the START square intended status byte to discover whether an audible alarm is to be given as well, and if it is, establishes the necessary connections within the intercom circuit to cause the 150 Hz tone to be produced at the internal intercom unit 17 concurrently with energisation of the red lamp 25 of the START square. Also, if audible signals are to be given, the high tone at the indicator is produced at the same time. The address of the second section of the mains failure routine is then stored in the re-entry pointer and a jump is made to the beginning of the telephone digit sending test. If bit 5 of the intended status byte of the START square is found to be in the reset state, the audible signals are omitted. In the second section of the mains failure routine, which is executed at the next interrupt, TIMER C is decremented and a jump to the telephone digit sending test executed unless TIMER C reaches zero as a result of the decrement in which case TIMER C is reloaded with its initial value, the red lamp 25 of the START square is turned off and the audible signals are turned off before the beginning address of the third section of the mains failure routine is stored in the re-entry pointer. Thus the red lamp and the tones, if not omitted, are produced for a time corresponding to the initial value of TIMER C. The third section of the mains failure routine decrements TIMER C and jumps to the telephone digit sending test unless TIMER C has expired in which case TIMER C is reloaded and the red lamp and, if allowed, the audible signals are turned on again before the beginning address for the fourth section is stored in the re-entry pointer. The fourth section is repeated by successive interrupts until TIMER C has reached zero whereupon the signals are turned off again. The fifth section decrements TIMER B at successive interrupts until TIMER B expires whereupon the initial value of TIMER B is reloaded and the mains failure pointer is loaded with the beginning address of the first section of the mains failure routine.

In the main program, when a square is selected as indicated in Fig. 32 and leads to the ALARM routine from the decision 325, the red lamp 25 of the ALARM square is turned on or off depending upon its previous state being off or on, i.e. this lamp is toggled, and the alarm device 15 of Fig. 1 is energised if the red lamp 25 is turned on or de-energised if the red lamp is turned off. This is effected by transmission of the appropriate signal on data line D4 to port 7. Also, the DIAL mode is cleared if the telephone line has been seized or, if it has not been seized, REP1 and REP2 are reset. The red lamp 25 of the DOOR square is also turned on and the door release device 14 is energised but TIMER4 is not started, the de-energisation of the door release device 14 being made dependant upon subsequent selection of the DOOR square. TIMER4 is reset to ensure that it does not prematurely de-energise the door release device 14. If selection of the ALARM square shows that the AL:SZ fquare is already active, the red lamp 25 of this square is turned off, the alarm device 15 is de-energised, and the red lamp 25 of the DOOR square is turned off and the door release device 14 is de-energised.

When the ALARM square has not been selected, but the DOOR square is selected, the function routine of the main program for the door catch release device 14 tests the intended status byte of the DOOR square to discover whether the red lamp 25 is at present on or off, and accordingly toggles the red lamp 25 of the DOOR square and energises or de-energises the door release device 14. When the door release device is de-energised, TIMER4 is reloaded with its initial value and the corresponding flag is reset in DESAV1. If the door release device 14 is energised, the intended status byte of the ALARM square is tested to discover whether this square is on, and if it is not, TIMER4 is arranged to be started by setting the flag for TIMER4 in DESAV1.

When the tests at 328 of Fig. 32 lead to a mains switching unit routine, the red lamp of the selected square is turned on at the indicator 13 and if the mains switching unit 18 is connected to the junction box 23, the corresponding controlled switch in the mains switching unit 18 is closed so that the appliance plugged in there is connected to the mains, since the controlled switch is controlled by the relay driver circuit associukpe Zith the lamp driver of the respective red lamp 25. Similarly the chosen appliance can be turned off by turning of the corresponding red lamp 25. If however the mains switching unit 18 is connected directly to the control unit 11, the same result is obtained by application of the appropriate signal levels to the chosen one of data lines D0, D1, D2 and D3 at port 7.

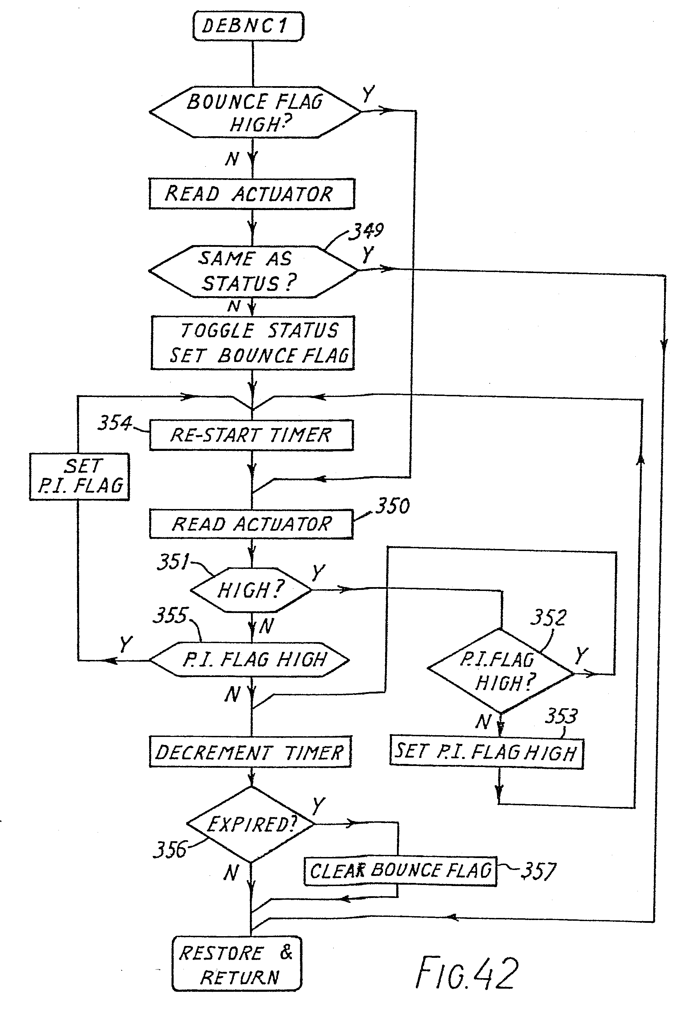

The final part of the interrupt routine is a debounce routine having a start address labelled DEBNC1 as indicated in Fig. 39. Fig. 42 shows this debounce routine which begins by retrieving the contents of the input store labelled BCSAV2 and testing bit 4 of the first byte of this two byte store to discover whether a bounce flag is set or not. If the reyint qohl su ief utfM bit 4 will be zero and reading of the actuator, i.e. the switch of the input device 12, is carried out as described hereinbefore in which the state of whatever input switch is being used is translated into a set or reset condition of data bit 2 read from port 6. This is compared with bit 2 of the first byte of BCSAV2 and if these two bits are found to be in the same state, thereby indicating that no change in the input has taken place since the last interrupt, then a jump is made from the decision, which is indicated at 349 in Fig. 2, to restoration of the contents of BCSAV2 and the internal registers of the CPU 30 and a return from the interrupt to the main program. If, however, at the decison 349 it is found that the actuator input differs from the previous state as read from bit 2 of BCSAV2 then bit 2 of BCSAV2 is toggled to bring it into the same state as the current state of the actuator input bit 2, bit 4 of the first byte of BCSAV2 is set, i.e. the bounce flag is set, the debounce timer labelled DELAY1 is loaded with its initial value and the actuator input is read again at 350 in Fig. 2 and the actuator input bit 2 is tested at a decision 351. If the actuator input bit 2 is high, this indicates that the input device 12 is not active since its switch is open. The debounce routine branches from the decision 351 if the actuator switch is open to a further decision 352 which tests bit 6 of the first byte of BCSAV2 which is used as a previous input flag and would be in the set state if the previous input state were also high. Assuming that the previous input state was not high, the bit 6 of the first byte of BCSAV2 is set at 353 and a jump is made back to the restarting of the timer DELAY1 at 354. If at the decision 351 the actuator input bit is found not to be high, a decision at 355 tests whether the previous input flag is high. Assuming that this flag is high, the routine branches through resetting of the previous input flag to restarting of DELAY1 again. The decision at 355 will then show that the previous input flag is high and the routine then proceeds to decrement the timer DELAY1 and tests whether this timer has expired at a decision 356. If the timer has not expired, the interrupt routine is left and return is made to the main program, the internal registers of the CPU 30 being restored and interrupts enabled. At the next interrupt, since the bounce flag will be found to be high, the debounce routine jumps immediately from testing the bounce flag to reading the actuator input at 350. Assuming that the actuator input bit was high when the bounce flag was first set and that no change is taking place in the actuator input bit, the debounce routine will pass from reading the actuator input bit at 350 through the decisions at 351 and 352 directly to decrementing the timer DELAY1 and the expiry test at 356. This will be repeated at each interrupt until the timer DELAY1 expires whereupon the bounce flag will be reset at 357 before restoration and return from the interrupt. If when the bounce flag was first set, the actuator input bit was low, indicating closure of the input device switch, the debounce routine again jumps immediately from testing the bounce flag to reading the actuator input bit at 350, then tests this bit at 351 which will be found to low, passes to the decision at 355 and thence to decrementing the timer and testing for expiry at 356 as in the case with the actuator input bit high. If during any interrupt after the bounce flag has been set the reading of the actuator at 350 results in detection of a change in the state of the actuator input bit either through the decision at 352 or the decision at 355, the timer DELAY1 is not decremented but is restarted at 354 by the branching either from the decision at 355 or from the passing on from the decision at 352 to the setting of the previous input flag at 353. Consequently if the actuator input bit changes after a shorter interval than the full time interval for count down of DELAY1, this timer is restarted and the bounce flag is not reset since the routine does not branch to the clearing of the bounce at 357. Consequently, since the status of the input which is stored as bit 2 of the first byte of BCSAV2 is only changed if the bounce flag is not set and a change in the actuator input bit is found at the decision 349, the stored actuator input bit status in BCSAV2 is a debounced version of the input actuator bit 2, and shows only the first change in each series of changes where the intervals between adjacent changes are shorter than the full duration of count down of the timer DELAY1.

CLAIMS

1.    An environmental control system having a control unit
and an indicator unit coupled by a channel for indicator
control signals to pass along to the indicator unit from
the control unit, the control unit including a central
processor and the indicator unit including decoding means,
the system including an input device adapted to be
operable as a switch coupled to the control unit, an
intercom circuit controlled by the control unit and
including a master acoustic transducer for use adjacent
the input device, and at least one remote acoustic
transducer, the intercom circuit being capable of
providing a speech signal channel from the master
transducer to a remote transducer and vice versa, an
audible alarm device coupled to the control unit, a door
releasing device coupled to the control unit, at least one
electrically controllable on/off switching device arranged
to be controlled by the control unit and having controlled
terminals to which an electrically powered appliance can
be manually connected by plug and socket connection, and
a telephone control circuit controlled by the control
unit and such as to enable a telephone unit incorporating
a telephone interface circuit to be so controlled by the
control unit as to make and receive telephone calls, the
control unit including a substantially permanent memory
containing a control program for the central processor
unit, a temporary memory, and a plurality of ports coupled
to the central processor and arranged to control or sense
operation of the input device, the indicator unit, the
intercom circuit, the audible alarm device, the door
releasing device, and the telephone control circuit, the
indicator unit having a plurality of visually
distinguishable indications of elements of the system
controllable by the control unit and being operable by the
control unit in such a manner that a user can select activation of indication of a particular element chosen by the user through the operation of the input device, and the control unit being such that activation of indication of a chosen element of the system is accompanied by the bringing into operation of that element.

2. A control system according to claim 1, wherein the channel for indicator control signals is a single line and the decoding means of the indicator unit decodes serial data received from the said channel into respective signals for controlling a plurality of light sources arranged in the indicator unit for effecting visually distinguishable indications of the said elements.

3. A control system according to claim 2, wherein at least one electrically controllable on/off switching device is coupled to the indicator unit and is controlled by the same indicator control signals as a corresponding light source arranged for effecting indication of the state of the said on/off switching device.

4. A control system accordingly to claim 2 or 3, wherein the indicator unit is coupled to the control unit by a plurality of lines, one of which is the said single line and another of which is a connection signal line such that connection of the indicator unit to the control unit can be sensed by the control unit at one of the said ports thereof from a signal on the said connection signal line, and the control unit is adapted by the control program to cause warning signals to be generated by the system if the connection signal line does not present the signal representative of connection of the indicator circuit.

5. A control system according to claim 4, wherein at least one other line of the said plurality is coupled to convey audio signals between the master transducer and other parts of the intercom circuit.

6. A control system according to claim 4 or 5, wherein the plurality of lines pass through a junction unit through which a further line passes connecting the input device to the control unit.

7. A control system according to any one of claims 2 to 6 wherein the light sources are visually distinguishable as a member of one group of light sources, each member of the group being accompanied by a light source visually distinguishable as member of another group, and the control program being such that the light sources belonging to the said other group can be activated in a predetermined sequence and the input device can be operated to cause activation of the light source belonging to the said one group and accompanying the currently activated one of the said other group of light sources.

8. A control system according to claim 7, wherein the indicator unit includes means controllable by the control unit to indicate audibly steps in the sequence of activation of the light sources of the said other group.

9. A control system according to any preceding claim, wherein the control unit includes a port having an output terminal which is coupled to the intercom circuit so as to allow an audio frequency signal produced at the said terminal by the control program to be controllably supplied to the master transducer or the at least one remote transducer.

10. A control system according to claim 9, wherein the central processor unit is adapted to so respond to an interrupt signal produced at a constant rate within the control unit as to provide at the said output terminal a square wave at the said audio frequency.

11. A control system according to claim 7, wherein the said sequence begins with activation of a light source of the said other group which accompanies the light source of the said one group which gives indications of the said audible alarm device.

12. A control system according to claim 7 or 11, wherein the duration of activation of the light source of the said other group which accompanies the light source of the said one group which gives indications of the said audible alarm device is greater than the duration of activation of the other light sources of the said other group.

13. A control system according to any preceding claim, wherein the control program is such that the state of the input device is sampled sufficiently frequently for a change therein to be sensed within a predetermined short time interval and that an input device status store in the temporary memory is loaded with the result of a sampling only if the interval since the previous change in the state of the input device is greater than a predetermined longer time interval than the said start time interval.

14. A control system according to claim 13, wherein the state of the input device is sampled in an interrupt routine executed in response to an interrupt signal generated at a high rate substantially continually throughout operation of the system.

15. An environmental control system substantially as described hereinbefore with reference to the accompanying drawings.

FIG.1

0123430

| 24 START 25 | 24 ALARM 25 | 24 0 DOOR 25 | 24 1 EXT. IC 25 | 24 2 INT. IC 25 | 24 3 SEIZE LINE 25 | 24 4 REP.1 24 |
|---|---|---|---|---|---|---|
| 24 5 REP.2 25 | 24 6 DIAL 25 | 24 7 VOL 25 | 24 8 CONTROL A 25 | 24 9 CONTROL B 25 | 24 EOD CONTROL C 25 | CANCEL CONTROL D 25 |

FIG.2

0123430

FIG.3

FIG 4

0123430

5/42

74HC04

FIG.5

FIG.6

FIG.7

0123430

FIG.8

0123430

FIG 9

10/42

FIG.10

FIG.11

70

+5V

16

35 →    IC.15

74C42

15  A0
14  A1
13  A2
12  A3

ADDRESS BUS

32

0 | 1 | $\overline{IO0}$
1 | 2 | $\overline{IO1}$
2 | 3 | $\overline{IO2}$
3 | 4 | $\overline{IO3}$
4 | 5 | $\overline{IO4}$
5 | 6 | $\overline{IO5}$
6 | 7 | $\overline{IO6}$
7 | 9 | $\overline{IO7}$
8 | 10 | $\overline{IO8}$
9 | 11 |

8

0V

FIG.12

D0  3 2  99

D1  5 4

D2  7 6  R11  100  70  +5V

D3  9 10  R12  R9  R10

D4  11 12  101

D5  13 14  SENSE A  102
           104

97

1,15

IRD  12
13 8  11  98
IO6

C5  C6  103

FIG.13

FIG.14

0123430

FIG.15

0123430

15/42

FIG.16

FIG.17

FIG.18

18/42

0123430

FIG.19

FIG.20

FIG.21

21/42

0123430

FIG.22

FIG.23

FIG.24

FIG. 25

0123430

FIG.26

```
        ┌─────────┐
        │   ORG   │
        └─────────┘
             │
             ▼
    ┌─────────────────────┐
    │ DISABLE INTERRUPTS  │
    │  SELECT INTERRUPT   │
    │      MODE 1         │
    └─────────────────────┘
             │
             ▼
    ┌─────────────────────┐
    │  JUMP OVER INTERRUPT │
    │      ROUTINE         │
    └─────────────────────┘
             │
             ▼
    ┌─────────────────────────┐
    │ RESET INTERRUPT LATCH   │
    │ INITIALISE STACK POINTER &│
    │ RAM AND OUTPUT PORTS    │
    └─────────────────────────┘
             │
             ▼
    ┌─────────────────────┐
    │ INITIALISE INDICATOR │
    └─────────────────────┘
```

306

```
    ┌─────────────────────┐
    │  ENABLE INTERRUPTS  │
    │    READ PORT 2      │
    └─────────────────────┘
```

302

```
    ┌─────────────────────┐
    │     INDICATOR       │
    │   DISCONNECTED      │
    │     ROUTINE         │
    └─────────────────────┘
```

DISCONNECTION ?    Y

301    N

MAINS FAILED ?    Y

303    N    304

FIRST OCCURRENCE    Y

N

```
    ┌─────────────────┐
    │  CLEAR MAINS    │
    │  FAILURE FLAGS  │
    └─────────────────┘
```

```
    ┌─────────────────────┐
    │ SET MAINS FAILURE   │
    │ FLAGS AND PREPARE   │
    │ ROUTINE.            │
    │ TURN OFF MAINS      │
    │ SWITCHES AND        │
    │ SQUARES             │
    └─────────────────────┘
```

```
    ┌─────────────────────┐
    │ TEST EXTERNAL INTERCOM │
    └─────────────────────┘
             │
             ▼
    ┌─────────────────────┐
    │ TEST INTERNAL INTERCOM │
    └─────────────────────┘
             │
             ▼
    ┌─────────────────────┐
    │ SCAN AND SELECTION  │
    │     ROUTINES        │
    └─────────────────────┘
```

305

FIG.27

0123430

```
┌─────────────┐
│   INSCAN    │
└─────────────┘
       │
       ▼
┌──────────────────────┐  312
│ FETCH ACTATOR FLAGS, │
│ TIMER FLAGS 1-8.     │
│ JUMP TO RENTRY       │
│      POINT           │
└──────────────────────┘

┌─────────────┐
│   SCAN 1    │
└─────────────┘
       │
       ▼
   ACTUATOR STATUS ──Y──┐
      ACTIVE ?          │
       │N               ▼
       ▼          ┌──────────────┐
   ┌───────┐      │ STORE SCAN 2 │
   │  RET  │      └──────────────┘
   └───────┘             │
                         ▼
┌─────────────┐      ┌───────┐
│   MOM 2     │      │  RET  │
└─────────────┘      └───────┘
       │
  314  ▼
   ACTUATOR STATUS ──Y──┐
      ACTIVE ?          │
       │N               ▼
       │            ┌───────┐
       │            │  RET  │
       │            └───────┘
       ▼
┌────────────────────────┐
│ TURN OFF START SQUARE  │── 315
│ TURN ON ALARM SQUARE   │
└────────────────────────┘
       │
       ▼
┌────────────────────────┐
│ START TIMER 2, LOW     │
│ TONE, AND TIMER 1      │
└────────────────────────┘
       │
       ▼
┌───────────────┐
│ STORE MOM 3   │
└───────────────┘
       │
       ▼
   ┌───────┐
   │  RET  │
   └───────┘
```

```
┌─────────────┐
│   SCAN 2    │
└─────────────┘
       │
       ▼
┌────────────────────┐
│ LOAD POINTERS      │
│ TO BY-PASS         │
│ REPLAY ROUTINE     │
└────────────────────┘
       │
       ▼
┌────────────────────┐
│   READ PORT 6      │
└────────────────────┘
       │
  313  ▼
   HELD MODE ? ──Y──┐
       │N           │
       ▼            │
┌──────────────┐    │
│ STORE MOM2   │    │
└──────────────┘    │
       │            │
       ▼            │
   ┌───────┐        │
   │  RET  │        │
   └───────┘        │
```

FIG. 28

FIG.29

```
        ┌──────────┐
        │  MOM 4   │
        └──────────┘
             │
    ┌────────────────────┐
    │   FETCH SCAN       │
    │ CURSOR POSITION.   │
    │    TURN OFF        │
    │  CURRENT SQUARE    │
    └────────────────────┘
             │
    ┌────────────────────┐
    │   STEP CURSOR      │───319
    └────────────────────┘
             │
        ╱─────────────╲         Y
    ╱      CURSOR       ╲──────────────┐
    ╲     OVERSHOT?     ╱              │
        ╲─────────────╱               │
             │ N                ┌──────────────────┐
             │                  │ RETURN TO START  │
             │                  │    POSITION      │
             │                  └──────────────────┘
             │                         │
             └───────────◄─────────────┘
             │
    ┌────────────────────┐
    │  TURN ON NEW       │
    │    SQUARE          │
    └────────────────────┘
             │
        ╱─────────────╲         Y
    ╱   START SQUARE?   ╲──────────────┐
        ╲─────────────╱               │
             │ N                ┌──────────────────┐
             │                  │  SELECT HIGH     │
             │                  │    TONE          │
    ┌────────────────────┐      └──────────────────┘
    │  SELECT LOW TONE   │             │
    └────────────────────┘             │
             │◄────────────────────────┘
             │
    ┌────────────────────┐
    │  START TONE AND    │
    │    TIMER 1         │
    └────────────────────┘
             │
    ┌────────────────────┐
    │  TRIGGER SCAN      │
    │ DWELL MONOSTABLE   │
    └────────────────────┘
             │
    ┌────────────────────┐
    │  STORE MOM 5       │
    └────────────────────┘
             │
        ┌──────────┐
        │   RET    │
        └──────────┘
```

FIG.30

0123430

```
                    ┌──────────────┐
                    │   I/P  TEST  │
                    └──────┬───────┘
                           │         ┌────────────────────────────────────┐
                           ▼         ▼                                     │
              ╱─────────────────────╲  Y                                   │
             ╱   SCAN MODE MOM?       ╲────────┐                           │
             ╲                        ╱        │                           │
              ╲─────────┬────────────╱         │                           │
                    N   │                      │                           │
                        ▼                      │                           │
                ┌──────────────┐               │                           │
                │ INVERT ACTATOR│              │                           │
                │    STATUS     │              │                           │
                └──────┬───────┘               │                           │
                       │                       │                           │
                       ▼         321           │                           │
              ╱─────────────────────╲  Y                                   │
             ╱        "ON"            ╲──────────────────┐                 │
             ╲                        ╱                  │   322           │
              ╲─────────┬────────────╱                   ▼                 │
                    N   │               ╱─────────────────────────╲  Y     │
                        ▼              ╱  SELECTION  DELAY          ╲───────┘
              ╱─────────────────────╲ ╲      CONTINUES             ╱
             ╱   SCAN MODE HELD?      ╲ ╲─────────────┬───────────╱
             ╲                        ╱ Y         N   │
              ╲─────────┬────────────╱───┐            ▼
                    N   │                │      ┌──────────────┐  323
                        │                ▼      │  SELECTION   │
                        │         ┌──────────┐  │   ROUTINE    │   ┌─────────────┐
                        │         │  STORE   │  └──────┬───────┘   │  END MOM    │
                        │         │  MOM 3   │         │           └──────┬──────┘
                        │         └────┬─────┘         │                  │
                        │              │               ▼                  │
                        │         ┌────────┐    ┌──────────────────────────┐
                        │         │  RET   │    ◄──┘                       │
                        │         └────────┘                               ▼
                        │              ╱─────────────────────────╲  Y
                        │         ◄───╱      ACTUATOR             ╲
                        │             ╲        OFF ?              ╱
                        ▼              ╲────────────┬────────────╱
                ┌──────────────┐               N   │
                │ RETURN SCAN  │                   ▼
                │   CURSOR     │            ┌────────────────┐
                │TO START SQUARE│           │ STORE END MOM  │
                └──────┬───────┘            └───────┬────────┘
                       ▼                            ▼
                ┌──────────────┐            ┌──────────┐
                │ STORE SCAN 1 │            │   RET    │
                └──────┬───────┘            └──────────┘
                       ▼
                ┌──────────┐
                │   RET    │
                └──────────┘
```

FIG.31

0123430

32/42

FIG.32

FIG.33

0123430

```
┌─────────┐
│  DIAL   │
└─────────┘
     │
     ▼
  REP SELECT INHIBIT? ──Y──► RET
     │ N
     ▼
  DIAL FLAG SET ──Y──► RESET DIAL FLAG
     │ N              TURN OFF
     ▼                DIAL SQUARE
  TURN ON                │
  DIAL SQUARE            ▼
     │                  RET
     ▼
  LINE SEIZED? ──Y──►
     │ N
     ▼
  SET DIAL FLAG
     │
     ▼
  TEST FOR REP1 OR
  REP 2 SELECTED AND
  POINT ACCORDINGLY
  AT REP1 BUFFER OR
  REP2 BUFFER OR AT
  DIAL BUFFER IF
  NEITHER SELECTED
     │
     ▼
  POINT AT
  INPUT BUFFER
     │
     ▼
   RET
```

333

SET REP SELECT
INHIBIT FLAG

PRINT AT DIAL
BUFFER 1

DIAL OUT
ROUTINE

334

FIG.34

```
        ┌─────────────┐
        │   DIAL IN   │
        └─────────────┘
               │
               ▼
   ┌──────────────────────────┐
   │ CONVERT FUNCTION CURSOR  │
   │   POSITION INTO DIGIT    │
   └──────────────────────────┘
               │
               ▼
      ⬡ CANCEL CODE? ⬡──────Y──────┐
               │                     │
               │N                    ▼
               ▼           ⬡ POINTER         ⬡──────Y──────┐
      ⬡  POINTER  ⬡──Y──┐    UNCHANGED?                   │
        OVERSHOT?        │        │                        │
               │         │        │N                       │
               │N        │        ▼                        │
               ▼         │  ┌──────────────────┐           │
   ┌──────────────────┐  │  │ CANCEL DIAL MODE │           │
   │ WRITE TELEPHONE  │  │  │ AND REP SQUARES. │           │
   │  DIGIT TO INPUT  │  │  │ PREPARE TO BLINK │           │
   │     BUFFER       │  │  │  CANCEL SQUARE   │           │
   └──────────────────┘  │  └──────────────────┘           │
          335            │     337    │                    │
               │         │            ▼                    │
               ▼         │      ┌──────────┐               │
   ┌──────────────────┐  │      │   RET    │               │
   │ PREPARE TO BLINK │  │      └──────────┘               │
   │  AT DIGIT SQUARE │  │                                 │
   └──────────────────┘  │                                 │
               │         │                                 │
               ▼         │                                 │
        ⬡  EOD?  ⬡───Y───┤                                 │
               │         │                                 │
               │N        ▼                                 │
               ▼  ┌──────────────┐                         │
        ┌────────┐│ BLOCK MOVE OF│                         │
        │  RET   ││ INPUT BUFFER │──336                    │
        └────────┘│   DATA TO    │                         │
                  │ DESTINATION  │                         │
                  │   BUFFER     │                         │
                  └──────────────┘                         │
                         │                                 │
                         ▼◄────────────────────────────────┘
              ┌────────────────────┐
              │ PREPARE TO REPLAY  │
              └────────────────────┘
                         │
                         ▼
              ┌────────────────────┐
              │ CANCEL DIAL MODE   │
              │  AND REP SQUARES   │
              └────────────────────┘
                         │
                         ▼
                   ┌──────────┐
                   │   RET    │
                   └──────────┘
```

FIG.35

FIG.36

FIG.37

FIG. 38

39/42

```
┌─────────────────┐
│   INTERRUPT     │
└─────────────────┘
         │
┌─────────────────┐
│ RESET INTERRUPT │
│ LATCH. STACK    │
│   REGISTERS     │
└─────────────────┘
         │
┌─────────────────┐
│ TOGGLE INTERCOM │
│ TONE OUTPUT.    │
│ FETCH TIMER     │
│ FLAGS & BLINK   │
│     COUNT       │
└─────────────────┘
         │
```

MAINS FAILURE? —Y→ CURRENT SECTION OF MAINS FAILURE ROUTINE

N

SEND TELEPHONE DIGITS? —Y→ CURRENT SECTION OF S.T.D. ROUTINE    342

N

BLINK? —Y→ CURRENT SECTION OF BLINK ROUTINE

N

REPLAY? —Y→ CURRENT SECTION OF REPLAY ROUTINE

N

TIMERS 1 TO 7? —Y→ TEST EACH TIMER 1 TO 7 IN TURN

N

TIMER 8? —Y→ DEC OR RES TIMER 8

N

```
┌─────────────────┐
│   DEBNC 1       │
└─────────────────┘
```

FIG.39

```
           ┌──────────┐
           │  SEND 2  │
           └────┬─────┘
                │
          ┌─────┴─────┐
          │ DECREMENT │
          │  TIMER A  │
          └─────┬─────┘
                │
347             ▼
         ╱─────────────╲        Y
        ⟨    STILL      ⟩──────────────┐
         ╲  RUNNING?   ╱               │
          ╲───────────╱                ▼
                │ N            ┌─────────────────┐
                ▼              │  JUMP TO NEXT   │
          ┌───────────┐        │ RE-ENTRY POINT  │
          │ INITIALISE │       └─────────────────┘
          │  TIMER A   │
          └─────┬─────┘
                │
          ╱─────┴──────╱
         ╱   CLEAR    ╱ ── 348
        ╱  PORT O    ╱
       ╱────────────╱
                │
        ┌───────┴────────┐
        │  RETURN SCAN   │
        │   LAMP TO      │
        │ STORED STATUS  │
        └───────┬────────┘
                │
          ┌─────┴─────┐
          │   STORE   │
          │  SEND 3   │
          └─────┬─────┘
                │
        ┌───────┴────────┐
        │  JUMP TO NEXT  │
        │ RE-ENTRY POINT │
        └────────────────┘
```

FIG.40

FIG.41

FIG.42

European Patent Office

**EUROPEAN SEARCH REPORT**

| | DOCUMENTS CONSIDERED TO BE RELEVANT | | Page 2 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | FR-A-2 395 559 (INSTITUT NATIONALE DE LA SANTE ET DE LA RECHERCHE MEDICALE) <br> * Page 7, line 38 - page 8, line 8; figure 1 * | 1 | |

-----

TECHNICAL FIELDS SEARCHED (Int. Cl. ³)

The present search report has been drawn up for all claims

| Place of search | Date of completion of the search | Examiner |
|---|---|---|
| THE HAGUE | 27-06-1984 | GULDNER H.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent
Office

**EUROPEAN SEARCH REPORT**

0123430
Application number

EP ·84 30 1951

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| Y | US-A-3 911 316 (FEICK et al.) <br> * Column 1, line 46 - column 12, line 8 * | 1 | H 04 Q 9/00 |
| A | | 2-9 | |
| | --- | | |
| Y | US-A-4 086 434 (BOCCHI) <br> * Column 3, line 54 - column 7, line 48; figure 1 * | 1 | |
| | --- | | |
| A | DE-A-3 036 709 (SIEMENS) <br> * Page 3, line 11 - page 5, line 1; figure * | 1 | |
| | --- | | |
| A,D | GB-A- 956 302 (W.H. SHORT) <br><br> * Page 1, line 10 - page 3, line 9 * | 1-7,13 ,14 | TECHNICAL FIELDS SEARCHED (Int. Cl. ³) |
| | --- | | H 04 Q |
| A | US-A-4 338 493 (STENHUIS et al.) <br> * Whole document * | 1,9,10 | G 08 C <br> H 04 M |
| | --- | | |
| A | WO-A-8 102 657 (MORGAN) <br> * Page 2, line 24 - page 11, line 18 * | 1 | |
| | --- -/- | | |

The present search report has been drawn up for all claims

| Place of search | Date of completion of the search | Examiner |
|---|---|---|
| THE HAGUE | 27-06-1984 | GULDNER H.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82